(19) Europäisches Patentamt
European Patent Office
Office européen des brevets

(11) EP 3 486 321 A1

# (12) EUROPEAN PATENT APPLICATION

published in accordance with Art. 153(4) EPC

(43) Date of publication:
**22.05.2019 Bulletin 2019/21**

(21) Application number: **17815521.4**

(22) Date of filing: **23.06.2017**

(51) Int Cl.:
***C12N 15/09*** *(2006.01)* ***C12Q 1/68*** *(2018.01)*

(86) International application number:
**PCT/JP2017/023253**

(87) International publication number:
**WO 2017/222056 (28.12.2017 Gazette 2017/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.06.2016 JP 2016125007**

(71) Applicants:
- **Riken**
  **Wako-shi, Saitama 351-0198 (JP)**
- **Repertoire Genesis Incorporation**
  **Ibaraki-shi, Osaka 567-0085 (JP)**

(72) Inventors:
- **SHIROGUCHI, Katsuyuki**
  **Wako-shi**
  **Saitama 351-0198 (JP)**
- **SUZUKI, Ryuji**
  **Ibaraki-shi**
  **Osaka 567-0085 (JP)**
- **MATSUTANI, Takaji**
  **Ibaraki-shi**
  **Osaka 567-0085 (JP)**
- **KITAURA, Kazutaka**
  **Ibaraki-shi**
  **Osaka 567-0085 (JP)**

(74) Representative: **Uexküll & Stolberg**
**Partnerschaft von**
**Patent- und Rechtsanwälten mbB**
**Beselerstraße 4**
**22607 Hamburg (DE)**

## (54) T-CELL RECEPTOR AND B-CELL RECEPTOR REPERTOIRE ANALYSIS SYSTEM USING ONE-STEP REVERSE TRANSCRIPTION TEMPLATE-SWITCHING PCR

(57) The present invention provides a method for analyzing the variable region repertoire of the T-cell receptors (TCR) or the B-cell receptors (BCR) of a subject, said method including: (1) a step for providing a nucleic acid sample that is amplified from RNA obtained from the subject using one-step reverse transcription template-switching PCR and that includes the nucleic acid sequences of a plurality of types of T-cell receptors (TCR) or B-cell receptors (BCR); (2) a step for determining the nucleic acid sequences included in the nucleic acid sample; and (3) a step for calculating the frequencies of occurrence of individual genes or combinations thereof on the basis of the determined nucleic acid sequences and deriving the TCR or BCR repertoire of the subject.

EP 3 486 321 A1

## Description

[Technical Field]

The present invention relates to a technology for performing reverse transcription template switching PCR in one step and a T cell receptor (TCR) or B cell receptor (BCR) repertoire analysis system utilizing this technology.

[Background Art]

A reverse transcription polymerase chain reaction (RT-PCR) is universally used in the field of genetic engineering as a method of amplifying a specific gene using RNA as a template. In RT-PCR, a reverse transcriptase (RNA dependent DNA polymerase) is used for reverse transcription of RNA into cDNA, and then the cDNA is amplified to a detectable level by a heat resistant DNA polymerase. While this reaction combination is conventionally performed in two steps (uses a separate tube for each reaction and continuously reacted), technologies are in development for performing this reaction combination in one step (successively reacted in a single tube) by improvement in reverse transcriptase or reaction solution composition.

Template switching is a technique that enables RT-PCR amplification using an RNA as a template, even if the sequence of the 5' terminus of the template RNA is unknown or lacks a common sequence. Template switching utilizes a phenomenon, where a short specific sequence (e.g., a short cytosine rich sequence for Moloney Murine Leukemia Virus derived reverse transcriptase (MMLV RT)) is automatically added to the 3' terminus of a newly synthesized cDNA by the terminal transferase activity of a reverse transcriptase when the reverse transcriptase reaches the 5' terminus of a template RNA. If a sequence that is complementary to this short added sequence is added into the system upon reverse transcription of an oligonucleotide (template switching oligonucleotide) added to the 3' terminus of an anchor sequence, the template switching oligonucleotide hybridizes to the 3' terminus of the synthesized cDNA to extend the template for a reverse transcriptase. Since a reverse transcriptase switches templates and continues cDNA synthesis to the 5' terminus of the anchor sequence, a sequence that is complementary to the anchor sequence is added to the 3' terminus of the cDNA. By using an oligo DNA comprising a sequence that is complementary to a specific sequence in a template RNA or an oligo DNA with a specific known sequence added to the 5' terminus as a reverse transcription primer, a newly synthesized cDNA would also have a known sequence at the 5' terminus. As a result, the newly synthesized cDNA (antisense strand) would comprise a known sequence on both the 5' terminus and the 3' terminus. Therefore, use of a primer set that is designed based on these known sequences enable PCR amplification.

Synthesis of a cDNA corresponding to an mRNA with a poly-(A) tail is achieved by reverse transcription using a random primer or an oligo (dT) containing primer that is complementary to the poly-(A) tail. In this reaction, a cDNA is synthesized from all mRNAs with a poly-(A) tail, so that a cDNA library is constructed. Meanwhile, only a cDNA of a specific gene can be specifically synthesized by using a primer that is specific to a specific gene in reverse transcription.

There is a need for a technology for performing a faster and simpler reverse transcription template switching PCR with higher specificity so that the PCR can be applied for a high throughput operation.

Meanwhile, various hot start technologies have been developed as a technology for avoiding side reactions in PCR. In other words, a PCR reaction mixture is exposed to a temperature from room temperature to 50°C from the preparation of a reaction solution until the temperature of a thermal cycler increases in PCR. Since Tm of a primer is generally set at 50° C or higher, the specificity of the primer is not sufficiently exhibited in this temperature range. Meanwhile, a polymerase exhibits activity (albeit weak activity) in this temperature range, resulting in extension from a mis-annealed primer to cause various side reactions (primer dimers, extra band, or the like). An oligonucleotide primer bound to a thermolabile modifying group is in development as a technology for avoiding such a side reaction (Patent Literatures 1 and 2, and Non Patent Literatures 1 and 2). In this oligonucleotide primer, a 3' terminus hydroxyl group or one or more internucleotide bonds is replaced with a modifying group. Protection by this modifying group suppresses DNA polymerase mediated oligonucleotide primer extension before the first high temperature incubation period in PCR amplification. Due to the presence of a modifying group, a primer is inactive until reaching the first denaturation temperature (in most cases 95°C). After reaching the first denaturation temperature, the modifying group leaves, resulting in a corresponding unmodified oligonucleotide primer that is capable of extending by a polymerase.

In recent years, a next generation sequence analysis technology, which has advanced rapidly, has enabled large-scale sequencing of the base sequences of genes. A next generation TCR repertoire analysis method for obtaining and analyzing more detailed gene information at a clone level can be materialized from conventional small scale TCR repertoire analysis obtaining limited information such as V chain usage frequency by PCR amplification of a TCR gene from a human sample and use of the next generation sequence analysis technology. Patent Literature 3 discloses a method of quantitatively analyzing a repertoire of variable regions of T cell receptors (TCR) or B cell receptors (BCR) using a nucleic acid sample that has been amplified in an unbiased manner by an adapter primer.

[Citation List]

[Patent Literature]

[PTL 1] US Patent No. 8133669
[PTL 2] US Patent No. 8361753
[PTL 3] International Publication No. WO 2015/075939

[Non Patent Literature]

[NPL 1] Curr Protoc Nucleic Acid Chem. 2009 Sep; Chapter 4: Unit 4.35 1-17
[NPL 2] Nucleic Acids Res. 2008 Nov; 36(20): e131

[Summary of Invention]

[Solution to Problem]

The present invention provides a technology for performing a faster and simpler reverse transcription template switching PCR with high specificity. The present invention also provides a method of quantitatively analyzing a repertoire of variable regions of T cell receptors (TCR) or B cell receptors (BCR) by applying reverse transcription template switching PCR.

The present invention is described hereinafter in more detail. The inventors used a primer that is specific to a specific gene as a reverse transcription primer and used a combination of an oligonucleotide having an anchor sequence in a template switching primer and the reverse transcription primer as a primer set for PCR in reverse transcription template switching PCR to perform a reaction combination of reverse transcription and PCR in one step (one stage in the same reaction system). However, a side reaction is induced in this method. In particular, this tendency was prominent when the number of copies of a template RNA was low and the number of PCR cycles was high. The possible causes of side reactions include: the specificity of PCR is dependent only on the reverse transcription primer; and since reverse transcription primers are in significant excess relative to the number of copies of the template RNA, reverse transcription primers hybridize non-specifically to the template RNA, resulting in non-specific reverse transcription.

In this regard, a reverse transcription primer is used as a primer in not only reverse transcription but also PCR in the reaction combination. Meanwhile, this has been revised to use a primer inactivated by protecting the 3' terminus OH of an oligonucleotide that is the same as the reverse transcription primer with a thermolabile modifying group (hereinafter, an inactivated primer is also referred to as a block primer) as a primer for PCR and to reduce the amount of reverse transcription primer added to successfully suppress side reactions. According to this method, a reverse transcription primer and a block primer hybridize with a template RNA in reverse transcription. Meanwhile, the block primer cannot contribute to reverse transcription due to the protection by the modifying group, so that reverse transcription starts only from the reverse transcription primer. The block primer, even if it hybridizes non-specifically to a template RNA, does not produce a reverse transcription product. When the modifying group leaves the block primer to be converted to an unmodified primer by the first high temperature incubation in PCR amplification, the primer can contribute to an extension reaction, so that PCR progresses. Surprisingly, PCR amplification with high specificity can be achieved even by performing reverse transcription template switching PCR in one step by adding only a block primer without adding an unmodified reverse transcription primer.

The present invention applies the one-step reverse transcription template switching PCR to quantitative analysis of a repertoire of variable regions of T cell receptors (TCR) or B cell receptors (BCR).

The inventors have completed the present invention after further research based on such findings.

In other words, the present invention includes the following:

[1] A method for amplifying a nucleic acid which amplifies at least a part of a region of an RNA using a modified oligonucleotide primer,
wherein an amplification reaction of the nucleic acid consists of a reverse transcription step a) using the RNA as a template, a template switching step b) for adding a template switching oligonucleotide to a cDNA synthesized in step a), and a DNA amplifying step c) by a PCR using a template switch cDNA synthesized in step b) as a template, wherein steps a) to c) are performed in one stage in the same reaction system,

wherein the modified oligonucleotide primer is characterized in that due to the modification, a primer function is partially or completely blocked in reverse transcription step a), and blocking of the primer function is cleared in DNA amplification step c).

[2] A method for amplifying a nucleic acid which amplifies at least a part of a region of an RNA using a modified oligonucleotide primer, comprising

1) providing a composition comprising all reagents (excluding oligonucleotide primers that initiate reverse transcription) required for template switching reverse transcription of a template RNA to a cDNA and for PCR amplification of at least a part of the cDNA, including i) a template switching oligonucleotide, ii) a primer set consisting of a 5' anchor oligonucleotide primer comprising at least a part of an anchor sequence comprised in the template switching oligonucleotide, and a modified oligonucleotide primer, and iii) the template RNA;
2) incubating the composition provided in 1) at a temperature where reverse transcription can progress, thereby generating a cDNA with a nucleotide sequence that is complementary to the anchor sequence added to a 3' terminus from the template RNA and obtaining a reaction mixture comprising the cDNA; and
3) subjecting the reaction mixture obtained in 2) to a plurality of rounds of a thermocycling protocol with which PCR can progress, thereby obtaining a nucleic acid with a region sandwiched by the primer set amplified using the cDNA as a template;
wherein the modified oligonucleotide primer has a primer function in reverse transcription that is partially or completely blocked by the modification, and blocking of the primer function is cleared (cleared) as a result of the reverse transcription or by initial thermal denaturation of

PCR.

[3] The method of [2], wherein the composition provided by 1) further comprises an oligonucleotide primer that initiates reverse transcription.

[4] The method of any one of [1] to [3], wherein the modified oligonucleotide primer has one or more complementary regions on a sequence of the same modified oligonucleotide primer, and has a turn structure by the complementary regions or comprises a thermolabile modifying group before initial thermal denaturation of PCR.

[5] The method of [4], wherein the modified oligonucleotide primer comprises a nucleotide sequence that is complementary to a partial sequence of the template RNA.

[6] The method of [5], wherein a part of the modified oligonucleotide primer whose primer function has not been blocked functions as an oligonucleotide primer that initiates reverse transcription by hybridizing to the template RNA.

[7] The method of any one of [1] to [6], wherein a concentration of an oligonucleotide primer that initiates reverse transcription is 40 nM or less.

[8] The method of any one of [1] to [7], wherein a number of rounds of thermal cycling of PCR is 40 or greater.

[9] A kit for performing one-step reverse transcription template switching PCR, comprising:

i) a template switching oligonucleotide; and
ii) a primer set consisting of a 5' anchor oligonucleotide primer comprising at least a part of an anchor sequence comprised in the template switching oligonucleotide, and a modified oligonucleotide primer;

wherein the modified oligonucleotide primer has a primer function in reverse transcription that is partially or completely blocked by the modification, and a primer function in PCR using a product of the reverse transcription as a template is acquired as a result of the reverse transcription or by initial thermal denaturation.

[10] The kit of [9], comprising the oligonucleotide of i) and the primer set of ii) as a composition comprising a mixture thereof.

[11] The kit of [9] or [10], further comprising an oligonucleotide primer that initiates reverse transcription.

[12] The kit of [9] or [10], which does not comprise an oligonucleotide primer that initiates reverse transcription.

[A1] A method of amplifying at least a part of a region of a target RNA, the method comprising the steps of:

a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and
b) subjecting the cDNA obtained in step a) to a condition under which a polymerase chain reaction occurs to amplify at least a part of a region of the cDNA;

wherein the reagent required for a polymerase chain reaction comprises a modified oligonucleotide primer designed to have a primer function that is partially or completely blocked in step a) and designed to have blocking of the primer

function cleared in step b).

[A2] A method of producing a nucleic acid sample that is amplified based on at least a part of a region of a target RNA, the method comprising the steps of:

a) mixing the target RNA, a reagent required for reverse transcription, a reagent required for template switching, and a reagent required for a polymerase chain reaction and subjecting the mixture to a condition under which reverse transcription occurs to provide a cDNA comprising a nucleic acid sequence corresponding to the target RNA and a template switching oligonucleotide; and

b) subjecting the cDNA obtained in step a) to a condition under which a polymerase chain reaction occurs;

wherein the reagent required for a polymerase chain reaction comprises a modified oligonucleotide primer designed to have a primer function that is partially or completely blocked in step a) and designed to have blocking of the primer function cleared in step b).

[A3] The method of [A1] or [A2], wherein the reagent required for a polymerase chain reaction optionally comprises a 5' anchor oligonucleotide primer comprising at least a part of an anchor sequence comprised in the template switching oligonucleotide.

[A4] The method of [A3], wherein the reagent required for a polymerase chain reaction does not comprise the 5' anchor oligonucleotide primer.

[A5] The method of any one of [A1] to [A4], wherein the reagent required for reverse transcription comprises an oligonucleotide primer that initiates reverse transcription, and the oligonucleotide primer that initiates reverse transcription is comprised in the mixture at a final concentration of about 40 nM or less, or at a mole ratio of about 1:10 or less relative to the modified oligonucleotide primer.

[A6] The method of any one of [A1] to [A5], wherein the modified oligonucleotide primer has one or more complementary regions on a sequence of the same modified oligonucleotide primer, and has a turn structure by the complementary regions or comprises a thermolabile modifying group before initial thermal denaturation of PCR.

[A7] The method of any one of [A1] to [A6], wherein the modified oligonucleotide primer comprises a nucleotide sequence that is complementary to a partial sequence of a template RNA.

[A8] The method of [A7], wherein a part of the modified oligonucleotide primer whose primer function has not been blocked functions as an oligonucleotide primer that initiates reverse transcription by hybridizing to the template RNA.

[A9] A kit for amplifying at least a part of a region of a target RNA, the kit comprising:

i) a reagent required for reverse transcription;
ii) a reagent required for template switching;
iii) a reagent required for a polymerase chain reaction using a modified oligonucleotide primer; and
iv) optionally a user manual;

characterized in that the reagents of i) to iii) and the modified oligonucleotide primer are all mixed in a reaction system as of the initiation of a reaction, wherein the modified oligonucleotide primer is designed to have a primer function that is partially or completely blocked under a condition where reverse transcription occurs and designed to have blocking of the primer function cleared under a condition where a polymerase chain reaction occurs.

[A10] The kit of [A9], wherein the reagent required for template switching comprises a template switching oligonucleotide, and the reagent required for a polymerase chain reaction optionally comprises a 5' anchor oligonucleotide primer comprising at least a part of an anchor sequence comprised in the template switching oligonucleotide.

[A11] The kit of [A10], wherein the reagent required for a polymerase chain reaction does not comprise the 5' anchor oligonucleotide primer.

[A12] The kit of any one of [A9] to [A11], characterized in that the reagent required for reverse transcription comprises an oligonucleotide primer that initiates reverse transcription, and the oligonucleotide primer that initiates reverse transcription is used at a final concentration of about 40 nM or less, or at a mole ratio of about 1:10 or less relative to the modified oligonucleotide primer.

[A13] The kit of any one of [A9] to [A12], wherein the modified oligonucleotide primer has one or more complementary regions on a sequence of the same modified oligonucleotide primer, and has a turn structure by the complementary regions or comprises a thermolabile modifying group before initial thermal denaturation of PCR.

[A14] The kit of any one of [A9] to [A13], wherein the modified oligonucleotide primer comprises a nucleotide sequence that is complementary to a partial sequence of a template RNA.

[A15] The kit of [A14], wherein a part of the modified oligonucleotide whose primer function has not been blocked functions as an oligonucleotide primer that initiates reverse transcription by hybridizing to the template RNA.

[A16] A composition for amplifying at least a part of a region of a target RNA, comprising a modified oligonucleotide primer, wherein the modified oligonucleotide primer is designed to have a primer function that is partially blocked

under a condition where reverse transcription occurs and designed to have the blocking of the primer function cleared under a condition where a polymerase chain reaction occurs, wherein a part of the modified oligonucleotide primer whose primer function has not been blocked functions as an oligonucleotide primer that initiates reverse transcription by hybridizing to a template RNA.

[A17] The composition of [A16], wherein the modified oligonucleotide primer has one or more complementary regions on a sequence of the same modified oligonucleotide primer, and has a turn structure by the complementary regions or comprises a thermolabile modifying group before initial thermal denaturation of PCR.

[A18] The composition of [A16] or [A17], wherein the composition is used in one-step reverse transcription template switching PCR.

[B1] A method of analyzing a repertoire of variable regions of T cell receptors (TCR) or B cell receptors (BCR) of a subject, comprising the steps of:

(1) providing a nucleic acid sample comprising nucleic acid sequences of a plurality of types of T cell receptors (TCR) or B cell receptors (BCR) amplified from an RNA obtained from the subject;
(2) determining the nucleic acid sequences contained in the nucleic acid sample; and
(3) calculating a frequency of appearance of each gene or a combination thereof based on the determined nucleic acid sequences to derive a TCR or BCR repertoire of the subject;

wherein step (1) comprises the steps of:

a) mixing an RNA obtained from the subject, a reagent required for reverse transcription, a reagent required for template switching, and a reagent required for a polymerase chain reaction and subjecting the mixture to a condition under which reverse transcription occurs to provide a cDNA comprising nucleic acid sequences of a plurality of types of T cell receptors (TCR) or B cell receptors (BCR); and
b) subjecting the cDNA obtained in step a) to a condition under which a polymerase chain reaction occurs to provide the nucleic acid sample comprising nucleic acid sequences of a plurality of types of T cell receptors (TCR) or B cell receptors (BCR);

wherein the reagent required for template switching comprises a template switching oligonucleotide, and
wherein the regent required for a polymerase chain reaction comprises a primer specific to a C region of the TCR or the BCR, wherein the primer specific to a C region is a modified oligonucleotide primer designed to have a primer function that is partially or completely blocked in step a) and designed to have blocking of the primer function cleared in step b).

[B2] A method of producing a nucleic acid sample for analyzing a repertoire of variable regions of T cell receptors (TCR) or B cell receptors (BCR) of a subject, the method comprising the step of (1) providing a nucleic acid sample comprising nucleic acid sequences of a plurality of types of T cell receptors (TCR) or B cell receptors (BCR) amplified from an RNA obtained from the subject, step (1) comprising the steps of:

a) mixing an RNA obtained from the subject, a reagent required for reverse transcription, a reagent required for template switching, and a reagent required for a polymerase chain reaction and subjecting the mixture to a condition under which reverse transcription occurs to provide a cDNA comprising nucleic acid sequences of a plurality of types of T cell receptors (TCR) or B cell receptors (BCR); and
b) subjecting the cDNA obtained in step a) to a condition under which a polymerase chain reaction occurs to provide the nucleic acid sample comprising nucleic acid sequences of a plurality of types of T cell receptors (TCR) or B cell receptors (BCR);

wherein the reagent required for template switching comprises a template switching oligonucleotide, and
wherein the regent required for a polymerase chain reaction comprises a primer specific to a C region of the TCR or the BCR, wherein the primer specific to a C region is a modified oligonucleotide primer designed to have a primer function that is partially or completely blocked in step a) and designed to have blocking of the primer function cleared in step b).

[B3] The method of [B1] or [B2], wherein the nucleic acid sample is a nucleic acid sample that has been amplified in an unbiased manner.

[B4] The method of any one of [B1] to [B3], wherein the reagent required for a polymerase chain reaction optionally further comprises a 5' anchor oligonucleotide primer comprising at least a part of an anchor sequence comprised in the template switching oligonucleotide.

[B5] The method of any one of [B1] to [B4], wherein the reagent required for a polymerase chain reaction does not comprise the 5' anchor oligonucleotide primer, and the template switching oligonucleotide functions as a 5' anchor

oligonucleotide primer.

[B6] The method of any one of [B1] to [B5], wherein the reagent required for reverse transcription comprises an oligonucleotide primer that initiates reverse transcription, and the oligonucleotide primer that initiates reverse transcription is comprised in the mixture at a final concentration of about 40 nM or less, or at a mole ratio of about 1:10 or less relative to the modified oligonucleotide primer.

[B7] The method of any one of [B1] to [B6], wherein the modified oligonucleotide primer has one or more complementary regions on a sequence of the same modified oligonucleotide primer, and has a turn structure by the complementary regions or comprises a thermolabile modifying group before initial thermal denaturation of PCR.

[B8] The method of any one of [B1] to [B7], wherein a part of the modified oligonucleotide, whose primer function has not been blocked, functions as an oligonucleotide primer that initiates reverse transcription by hybridizing to a template RNA.

[B9] A kit for amplifying a variable region of a T cell receptor (TCR) or a B cell receptor (BCR), the kit comprising:

i) a reagent required for reverse transcription;
ii) a reagent required for template switching;
iii) a reagent required for a polymerase chain reaction using a modified oligonucleotide primer; and
iv) optionally a user manual;

characterized in that the reagents of i) to iii) and the modified oligonucleotide primer are all mixed in a reaction system as of the initiation of a reaction,
wherein the reagent of ii) comprises a template switching oligonucleotide, and
wherein the modified oligonucleotide primer is a primer specific to a C region of the TCR or the BCR which is designed to have a primer function that is partially or completely blocked under a condition where reverse transcription occurs and designed to have blocking of the primer function cleared under a condition where a polymerase chain reaction occurs.

[B10] The kit of [B9], wherein the reagent required for a polymerase chain reaction comprises a 5' anchor oligonucleotide primer comprising at least a part of an anchor sequence comprised in the template switching oligonucleotide.

[B11] The kit of [B9] or [B10], wherein the reagent required for a polymerase chain reaction does not comprise the 5' anchor oligonucleotide primer.

[B12] The kit of any one of [B9] to [B11], characterized in that the reagent required for reverse transcription comprises an oligonucleotide primer that initiates reverse transcription, and the oligonucleotide primer that initiates reverse transcription is used at a final concentration of about 40 nM or less, or at a mole ratio of about 1:10 or less relative to the modified oligonucleotide primer.

[B13] The kit of any one of [B9] to [B12], wherein the modified oligonucleotide primer has one or more complementary regions on a sequence of the same modified oligonucleotide primer, and has a turn structure by the complementary regions or comprises a thermolabile modifying group before initial thermal denaturation of PCR.

[B14] The kit of any one of [B9] to [B13], wherein a part of the modified oligonucleotide whose primer function has not been blocked functions as an oligonucleotide primer that initiates reverse transcription by hybridizing to a partial sequence of a C region of a template RNA of the TCR or the BCR.

[B15] The kit of any one of [B9] to [B14] for providing a nucleic acid sample comprising nucleic acid sequences of a plurality of types of T cell receptors (TCR) or B cell receptors (BCR) amplified from an RNA obtained from the subject in an unbiased manner.

[B16] The method of [B1], or [B3] to [B8] when dependent from [B1], wherein step (1) further comprises a step of providing a nucleic acid sample to which a sequence that is suitable for sequence analysis is added.

[B17] A method of [B16], wherein the sequence that is suitable for sequence analysis is a sequence that is suitable for sequence analysis used in bridge PCR or emulsion PCR.

[B18] The method of [B1], [B3] to [B8] when dependent from [B1], [B16], or [B17], wherein step (1) further comprises the following steps:

c) subjecting a mixture comprising a PCR amplicon of step b), a second 5' anchor oligonucleotide primer to which a first tag sequence is added, and a second primer specific to a C region of TCR or BCR to which a second tag sequence is added to a condition under which a polymerase chain reaction occurs to provide a nucleic acid sample comprising nucleic acid sequences of a plurality of types of T cell receptors (TCR) or B cell receptors (BCR) to which a tag sequence is added; and
d) subjecting a mixture comprising a PCR amplicon of step c), a third 5' anchor oligonucleotide primer, and a third primer specific to a C region of TCR or BCR to a condition under which a polymerization chain reaction occurs to provide the nucleic acid sample comprising nucleic acid sequences of a plurality of types of T cell receptors (TCR) or B cell receptors (BCR) to which an index sequence is added, wherein an index sequence

and a sequences for immobilization to a substrate of sequence analysis are added to the third 5' anchor oligonucleotide primer and the third primer specific to a C region of TCR or BCR.

[B19] The method of [B1], [B3] to [B8] when dependent from [B1], [B16], [B17], or [B18], wherein step (3) comprises the following steps:

(3-1) providing a reference database for each gene region comprising at least one of a V region, a D region, a J region, and optionally a C region;
(3-2) providing an input sequence set which is optionally trimmed and optionally extracted to have a suitable length; (3-3) searching for homology of the input sequence set with the reference database for the each gene region and recording an alignment with an approximate reference allele and/or a sequence of the reference allele;
(3-4) assigning the V region and the J region for the input sequence set and extracting a nucleic acid sequence of the D region based on a result of assigning;
(3-5) translating the nucleic acid sequence of the D region into an amino acid sequence and classifying the D region by utilizing the amino acid sequence; and
(3-6) calculating a frequency of appearance for each of the V region, the D region, the J region and optionally the C region or a frequency of appearance of a combination thereof based on the classifying in step (3-5) to derive the TCR or BCR repertoire.

[B20] A system for quantitatively analyzing a repertoire of variable region of a T cell receptor (TCR) or a B cell receptor (BCR) of a subject by using a database, the system comprising:

(1) the kit of any one of [B9] to [B15];
(2) an apparatus for determining the nucleic acid sequence comprised in the nucleic acid sample; and
(3) an apparatus for calculating a frequency of appearance of each gene or a combination thereof based on the determined nucleic acid sequence to derive a TCR or BCR repertoire of the subject.

[B21] The system of [B20], wherein (1) the kit further comprises:

c) means for subjecting a mixture comprising a PCR amplicon of step b), a second 5' anchor oligonucleotide primer to which a first tag sequence is added, and a second primer specific to a C region of TCR or BCR to which a second tag sequence is added to a condition under which a polymerase chain reaction occurs to provide a nucleic acid sample comprising nucleic acid sequences of a plurality of types of T cell receptors (TCR) or B cell receptors (BCR) to which a tag sequence is added; and
d) means for subjecting a mixture comprising a PCR amplicon of step c), a third 5' anchor oligonucleotide primer, and a third primer specific to a C region of TCR or BCR to a condition under which a polymerization chain reaction occurs to provide the nucleic acid sample comprising nucleic acid sequences of a plurality of types of T cell receptors (TCR) or B cell receptors (BCR) to which an index sequence is added, wherein an index sequence and a sequences for immobilization to a substrate of sequence analysis are added to the third 5' anchor oligonucleotide primer and the third primer specific to a C region of TCR or BCR.

[B22] The system of [B20] or [B21], wherein (3) the apparatus for deriving a TCR or BCR repertoire comprises:

(3-1) means for providing a reference database for each gene region comprising at least one of a V region, a D region, a J region, and optionally a C region;
(3-2) means for providing an input sequence set which is optionally trimmed and optionally extracted to have a suitable length;
(3-3) means for searching for homology of the input sequence set with the reference database for the each gene region and recording an alignment with an approximate reference allele and/or a sequence of the reference allele; (3-4) means for assigning the V region and the J region for the input sequence set and extracting a nucleic acid sequence of the D region based on a result of assigning;
(3-5) means for translating the nucleic acid sequence of the D region into an amino acid sequence and classifying the D region by utilizing the amino acid sequence; and
(3-6) means for calculating a frequency of appearance for each of the V region, the D region, the J region, and optionally the C region or a frequency of appearance of a combination thereof based on the classifying in step (3-5) to derive the TCR or BCR repertoire.

[B23] A system for analyzing a disease, disorder, or condition of a subject, comprising the system of any one of

[B20] to [B22] and means for analyzing the disease, disorder, or condition of the subject based on a TCR or BCR repertoire derived based on the system.

[B24] A system for treating or preventing a disease, disorder, or condition of a subject, comprising: means for quantitatively associating the disease, disorder, or condition of the subject determined by the system of [B23] with the TCR or BCR repertoire; and means for selecting means for suitable treatment or prevention from the quantitative association.

It is intended that one or more of the aforementioned features can be provided as a combination of one or more of the aforementioned features in addition to as the explicitly shown combinations. Further embodiments and advantages of the present invention are recognized by those skilled in the art by reading and understanding the following Detailed Description as needed.

[Advantageous Effects of Invention]

According to the present invention, reverse transcription template switching PCR can be expected to be performed in one step with high specificity. In particular, a specific PCR product can be expected to be amplified while suppressing side reactions, even if the number of copies of template RNA is low and the number of PCR cycles is high.

[Brief Description of Drawings]

Figure 1 shows amplification of a TCRβ chain by one-step reverse transcription template switching PCR under various conditions. The arrow indicates the band of a full length TCRβ chain.

Figure 2 shows amplification of a TCRβ chain by one-step reverse transcription template switching PCR under various conditions. The arrow indicates the band of a full length TCRβ chain.

Figure 3 shows amplification of a TCRβ chain by one-step reverse transcription template switching PCR using a single cell of T cell as a template. The top arrow indicates the band of a full length TCRβ chain. The bottom arrow indicates a band of a TCRβ chain fragment.

Figure 4 shows amplification of a TCRβ chain by one-step reverse transcription template switching PCR under various conditions. The arrow indicates the band of a full length TCRβ chain.

Figure 5 shows amplification of a TCRβ chain by one-step reverse transcription template switching PCR under various conditions. The arrow indicates the band of a full length of a target sequence of a TCRβ chain.

Figure 6 shows a picture of electrophoresis of an index PCR reaction solution. The left lane indicates a marker DNA, and the right lane indicates a band of DNA amplified from an RNA extracted from Pmel-1 derived lymphocytes.

Figure 7 depicts V gene and J gene usage frequency graphs obtained from Pmel-1 mouse derived lymphoid cells.

Figure 8 depicts a V-J usage frequency graph obtained from Pmel-1 mouse derived lymphoid cells.

Figure 9 shows a picture of electrophoresis of an index PCR reaction solution. Each lane indicates, from the left in order, 1: marker DNA, 2 to 6: mouse spleen tissue (1000 ng, 200 ng, 40 ng, 8 ng, and 1.6 ng), 7 to 8: Pmel-1 derived lymphocytes (8 ng and 1.6 ng), 9: blank.

Figure 10 depicts usage frequency graphs for each of the V gene and J gene obtained from C57BL/6 mouse spleen tissue.

Figure 11 depicts a V-J usage frequency graph obtained from C57BL/6 mouse spleen tissue.

Figure 12 depicts a block diagram of the repertoire analysis system of the present invention.

[Description of Embodiments]

The present invention is explained hereinafter. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence. As used herein, "about" proceeding a numerical value means ± 10% of the subsequent numerical value.

The present invention relates to a method of amplifying at least a part of a region of a template RNA by one-step reverse transcription template switching PCR.

Reverse transcription template switching PCR is a technique that enables RT-PCR amplification using an RNA as a template, even if the sequence of the 5' terminus of the template RNA is unknown or lacks a common sequence. Reverse transcription template switching PCR utilizes a phenomenon, where a short specific sequence is automatically added to the 3' terminus of a newly synthesized cDNA by the terminal transferase activity of a reverse transcriptase when the reverse transcriptase reaches the 5' terminus of a template RNA. For example, a Moloney Murine Leukemia Virus derived reverse transcriptase (MMLV RT) adds a short cytosine rich sequence (e.g., CC, CCC, or CCCC) to the 3' terminus of the synthesized cDNA. If an oligonucleotide (template switching oligonucleotide) comprising a nucleotide sequence with a sequence that is complementary to the short sequence added to the 3' terminus of a specific anchor sequence (first anchor sequence) is added to a system upon reverse transcription, the template switching oligonucleotide hybridizes to the 3' terminus of the synthesized cDNA, via the interaction between the sequence added to the 3' terminus of the cDNA and the complementary sequence of the sequence added to the 3' terminus of the template switching oligonucleotide, to extend the template for a reverse transcriptase. Since a reverse transcriptase, after reaching the 5' terminus of the template RNA, switches a template to a template switching oligonucleotide and continues cDNA synthesis to the 5' terminus thereof, a sequence that is complementary to the anchor sequence (first anchor sequence) of the template switching oligonucleotide is added to the 3' terminus of the cDNA. By using an oligonucleotide primer comprising a sequence that is complementary to a specific sequence in a template RNA or an oligonucleotide primer with a specific anchor sequence (second anchor sequence) added to the 5' terminus (random primer, oligo (dT) primer, or the like) as a revers transcription primer, the newly synthesized cDNA also has a known sequence at the 5' terminus. As a result, the PCR amplification using a newly synthesized cDNA as a template is possible by using a primer set comprising an oligonucleotide primer comprising the known sequence and an oligonucleotide primer comprising at least a part of the first anchor sequence.

In some embodiments, template switching does not need to be performed known the sequence on the 5' terminus side of a template RNA is known.

In the method of the present invention, reverse transcription template switching PCR is performed in "one step (one stage)". "One-step reverse transcription template switching PCR (RT-TS-PCR)" refers to a method for amplifying a nucleic acid from a reverse transcription reaction, characterized by having all reagents required for template switching and PCR mixed as of the initiation of a reaction and advancing a reaction in the same reaction system without adding additional reagents required for reverse transcription, reagents required for template switching, or reagents required for PCR amplification, and preferably without opening the reaction system (e.g., without adding a reagent or opening/closing a tube).

In other words, in the method of the present invention, an amplification reaction of a nucleic acid consists of a reverse transcription step a) using the RNA as a template, a template switching step b) for adding a template switching oligonucleotide to a cDNA synthesized in step a), and a DNA amplifying step c) by a PCR using a template switch cDNA synthesized in step b) as a template, wherein steps a) to c) are performed in one stage in the same reaction system.

In another embodiment, the present invention is a method of amplifying at least a part of a region of a target RNA, the method comprising the steps of: a) mixing the target RNA, a reagent required for reverse transcription, and a reagent required for a polymerase chain reaction and subjecting the mixture to a condition under which reverse transcription occurs, the mixing optionally comprising mixing a reagent required for template switching; and b) subjecting the mixture to a condition under which a polymerase chain reaction occurs to amplify the at least a part of a region of the target RNA; wherein the reagent required for a polymerase chain reaction comprises a modified oligonucleotide primer designed to have a primer function that is partially or completely blocked in step a) and designed to have blocking of the primer function cleared in step b).

Furthermore, the present invention provides a method of producing a nucleic acid sample that is amplified based on at least a part of a region of a target RNA, the method comprising the steps of: a) mixing the target RNA, a reagent required for reverse transcription, and a reagent required for a polymerase chain reaction and subjecting the mixture to a condition under which reverse transcription occurs, the mixing optionally comprising mixing a regent required for template switching; and b) subjecting the mixture to a condition under which a polymerase chain reaction occurs; wherein the reagent required for a polymerase chain reaction comprises a modified oligonucleotide primer designed to have a primer function that is partially or completely blocked in step a) and designed to have blocking of the primer function cleared in step b).

If the sequence of the 5' terminus of the template RNA is unknown or lacks a common sequence, it is advantageous to perform template switching because a specific anchor sequence can be added to the 5' terminus of the template RNA. On the other hand, if the sequence on the 5' terminus side of the template RNA is known, template switching does not need to be performed.

In one embodiment, the reagent required for template switching can comprise a template switching oligonucleotide. In still another embodiment, a reagent required for a polymerase chain reaction can, but does not need to comprise a 5' anchor oligonucleotide primer comprising at least a part of an anchor sequence comprised in the template switching oligonucleotide. As demonstrated in the Examples herein, a template switching oligonucleotide (TS-Oligo) can

also unexpectedly function as a forward primer in PCR amplification. Therefore, a reagent required for a polymerase chain reaction can be free of the 5' anchor oligonucleotide primer or comprise a smaller amount that an amount that is commonly used.

Surprisingly, PCR amplification with high specificity was able to be achieved even by adding only the modified oligonucleotide primer without adding a reverse transcription primer to perform reverse transcription PCR. Although not wishing to be bound by any theory, a part of a modified oligonucleotide primer does not have the function blocked at the time of a reverse transcription reaction, so that a part of the modified oligonucleotide primer whose function is not blocked can function as a reserve transcription primer, or a function of a modified oligonucleotide primer is partially blocked at the time of a reverse transcription reaction, so that the modified oligonucleotide primer whose function is partially blocked can function as a reverse transcription primer in a limited capacity. Therefore in some embodiment, a reagent required for reverse transcription does not need to comprise an oligonucleotide primer that initiates reverse transcription. Even if it is comprised, the oligonucleotide primer that initiates reverse transcription used in the present invention can be contained at a smaller amount than an amount that is commonly used. In some embodiments, the concentration of the oligonucleotide primer that initiates reverse transcription in the composition is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less. In another embodiment, the oligonucleotide primer that initiates reverse transcription in the composition is comprised at a mole ratio of about 1:10 or less relative to a modified oligonucleotide primer, preferably about 1:20 or less, about 1:40 or less, about 1:160 or less, about 1:200 or less, about 635:1 or less, about 2000:1 or less, about 2500:1 or less, about 20,000:1 or less, about 200,000:1 or less, about 2,000,000:1 or less, or about 20,000,000:1 or less.

In the method of the present invention, at least one of the oligonucleotide primers in PCR has a primer function in reverse transcription that is partially or completely blocked by a modification, and the blocking of the primer function is cleared in the nucleic acid amplification step of PCR. This can accomplish functional separation of primers that are used in each of the nucleic acid amplification stage of PCR and reverse transcription reaction stage while being in the same reaction system, and is characterized by significant differentiation in primer concentrations at each reaction stage.

Examples of means for blocking/clearing a primer function include the following approaches. 1) a primer function is blocked at the time of reverse transcription by a primer designed to retain a turn structure in the reverse transcription reaction stage or to comprise a thermolabile modifying group.

After a reverse transcription reaction, blocking of a primer function is cleared by detachment of a thermolabile modifying group or dissolution of a turn structure by heat treatment. 2) A primer comprising an artificial base blocks the function as a primer at the reverse transcription reaction stage.

A reverse transcription reaction results in synthesis of a cDNA in which a nucleic acid forming a pair with an artificial base contained in a primer is incorporated from a template RNA by the reverse transcription reaction, and allowing the primer to be annealed to the artificial nucleic acid, thus clearing the blocking of the primer function.

In one-step RT-PCR, all reagents required for reverse transcription of a template RNA into cDNA and all reagents required for PCR using the resulting cDNA as a template are generally included within the reaction system as of the initiation of reverse transcription. Since Tm of a PCR primer is generally set at 50° C or higher, specificity of the primer may not be sufficiently exhibited in a temperature zone where reverse transcription can progress (e.g., 42° C). Further, since the number of copies of a template increases exponentially in PCR, the required PCR primer concentration is dramatically higher than the reverse transcription primer concentration. Therefore, there is a risk of a PCR primer mis-annelaing to a template RNA to cause non-specific reverse transcription due to the mis-annealing as the initiation point and ultimately producing non-specific PCR products in reverse transcription. In the present invention, non-specific reverse transcription can be suppressed by using, as a reverse primer in PCR, a modified oligonucleotide primer, which has a primer function in reverse transcription partially or completely blocked by a modification and has acquired a primer function in PCR using the reverse transcription product as a template as a result of the reverse transcription or by thermal denaturation.

As used herein, "oligonucleotide", "primer", or "oligonucleotide primer" generally refers to a single stranded polynucleotide. This may be naturally-occurring or synthetic. This is generally comprised of a sequence of about 5 to about 50 nucleotides, more preferably about 10 to about 30 nucleotides, or more preferably about 15 to about 25 nucleotides. Oligonucleotides encompass DNA, RNA, and DNA/RNA chimeras.

As used herein, the term "forward primer" refers to an oligonucleotide primer that anneals to an antisense strand when the template RNA in RT-PCR is a sense strand. "Reverse primer" refers to an oligonucleotide primer that anneals to a sense strand.

In one embodiment, the modified oligonucleotide primer used in the present invention comprises a sequence that is complementary to a partial sequence of a template RNA. Although the length of the partial sequence is not particularly limited, the length is generally 10 to 40 bases, preferably 15 to 30 bases, and more preferably 18 to 25 bases. The partially sequence can be a partial sequence of the 3' terminus of a region intended to be amplified in a template RNA. The modified oligonucleotide primer preferably comprises a sequence that is complementary to a partial sequence

of a template RNA at the 3' terminus thereof. The modified oligonucleotide primer can comprise a sequence added to the 5' terminus of a sequence that is complementary to a partial sequence of a template RNA. Although the added sequence is not particularly limited, the sequence optimally does not comprise a sequence that is complementary to a partial sequence of a template RNA from the viewpoint of avoiding non-specific hybridization. Examples of the added sequence include specific restriction enzyme recognizing sequences. Although the length of the added sequence is not particularly limited, but shorter sequences are preferred to avoid non-specific hybridization. The length of the added sequence is generally 1 to 50 bases, preferably 1 to 30 bases, and more preferably 1 to 10 bases. In one embodiment, the modified oligonucleotide primer consists of a sequence that is complementary to a partial sequence of a template RNA without an added sequence.

Exemplary embodiments of modifications in the modified oligonucleotide primer used in the present invention include the following:

(1) oligonucleotide primers comprising a thermolabile modifying group;
(2) oligonucleotide primers having one or more complementary regions on a sequence of the same modified oligonucleotide primer and having a turn structure by the complementary regions prior to initial thermal denaturation of PCR to form an intermolecular hairpin loop to exhibit a structure masking a sequence that is complementary to a partial sequence of a template RNA;
(3) oligonucleotide primers comprising an artificial base.

Each of the embodiments is discussed in detail below.

(1) Oligonucleotide primers comprising thermolabile modifying group

In this embodiment, an oligonucleotide primer comprises a thermolabile modifying group so that a modifying nucleotide primer cannot extend the chain along a polynucleotide to which it has hybridized, i.e., cannot extend due to enzyme blocking or a decrease in hybridization to a target nucleic acid. In a preferred embodiment, the 3' terminus hydroxyl group or one or more internucleotide bonds of an oligonucleotide primer is substituted with a thermolabile modifying group. Therefore, a chain does not extend to a substantial degree unless and until a modifying or modified nucleotide is removed. While the modifying group is thermolabile, the group hardly dissociates until reaching the first denaturation temperature in PCR amplification (e.g., about 80 to 105°C, preferably about 85 to 100°C, and more preferably about 90 to 96° C (e.g., 95" C)), so that the primer function is partially or completely blocked in reverse transcription. Once the first denaturation temperature is reached, partial or complete dissociation of a modifying group from a modified oligonucleotide primer is thermally induced. The modified oligonucleotide primer is converted to a corresponding unmodified oligonucleotide primer. An unmodified oligonucleotide primer has an active phosphodiester bond and can extend by polymerase.

Examples of oligonucleotide primers comprising a thermolabile modifying group include the modified oligonucleotide primers with a hydroxyl group at the 3' terminus substituted with a thermolabile modifying group disclosed in US Patent No. 8133669 (the disclosed content is incorporated herein by reference to the same extent as the entirety thereof is explicitly described herein), modified oligonucleotide primers comprising a thermolabile modifying group in one or more internucleotide bonds disclosed in US Patent No. 8361753 (the disclosed content is incorporated herein by reference to the same extent as the entirety thereof is explicitly described herein), and the like.

(1-1) Modified oligonucleotide primers with a hydroxyl group at the 3' terminus substituted with a thermolabile modifying group (US Patent No. 8133669)

In one embodiment, the modifying group contained at the 3' terminus of the modified oligonucleotide primer is one of the groups selected from the group consisting of

[Chemical formula 1]

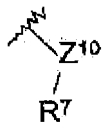 , 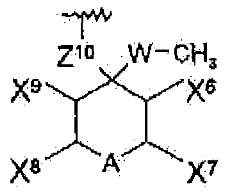 , 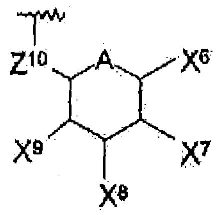 , 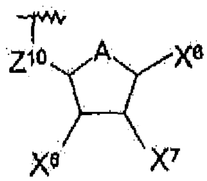 ,

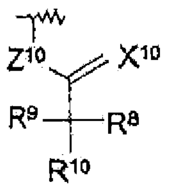 ,and 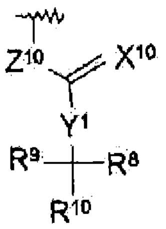 ,

wherein

$Z^{10}$ is selected from the group consisting of O, S, and Se;

each $R^7$, each $R^8$, each $R^9$, and each $R^{10}$ is independently selected from the group consisting of hydrogen, and a straight or branched optionally substituted hydrocarbyl group having from 1 to 20 carbon atoms, preferably 1 to 10 carbon atoms, and preferably 1 to 6 carbon atoms, wherein

the hydrocarbyl is alkyl, alkenyl, or alkynyl which may include at least one substituent selected from the group consisting of halo, oxo, hydroxyl, alkoxy, amino, amido, cycloalkyl, heterocycloalkyl, aryl, aryloxy, and heteroaryl;

each $X^6$, each $X^7$, each $X^8$, and each $X^9$ is independently selected from any substituted or unsubstituted group consisting of acyl, acyloxy, alkenyl, alkenylaryl, alkenylene, alkyl, lower alkyl, alkylene, alkynyl, alkynylaryl, alkoxy, lower alkoxy, alkylaryl, alkylcarbonylamino, alkylsulfinyl, alkylsulfonyl, alkylsulfonylamino, alkylthio, alkynylene, amido, amidino, amino, arylalkynyl, aralkyl, aroyl, arylalkyl, aryl, arylcarbonylamino, arylene, aryloxy, arylsulfonylamino, carbamate, dithiocarbamate, cycloalkenyl, cycloalkyl, cycloalkylene, guanidinyl, halo, halogen, heteroaryl, heteroarylcarbonylamino, heteroaryloxy, heteroarylsulfonylamino, heterocycle, heterocycle, hydrocarbyl, hydrocarbyl, hydrocarbylcarbonyl, hydrocarbyloxycarbonyl, hydrocarbylcarbonyloxy, hydrocarbylene, organosulfinyl, hydroxyl, organosulfinyl, organosulfonyl, sulfinyl, sulfonyl, sulfonylamino, and sulfuryl;

each $X^{10}$ is independently selected from the group consisting of O, S, Se, $NR^{11}$, N-$OR^{11}$, and $CR^{11}R^{12}$;

each $R^{11}$ and each $R^{12}$ is independently selected from any substituted or unsubstituted group consisting of acyl, acyloxy, alkenyl, alkenylaryl, alkenylene, alkyl, lower alkyl, alkylene, alkynyl, alkynylaryl, alkoxy, lower alkoxy, alkylaryl, alkylcarbonylamino, alkylsulfinyl, alkylsulfonyl, alkylsulfonylamino, alkylthio, alkynylene, amido, amidino, amino, arylalkynyl, aralkyl, aroyl, arylalkyl, aryl, arylcarbonylamino, arylene, aryloxy arylsulfonylamino, carbamate, dithiocarbamate, cycloalkenyl, cycloalkyl, cycloalkylene, guanidinyl, halo, halogen, heteroaryl, heteroarylcarbonylamino, heteroaryloxy, heteroarylsulfonylamino, heterocycle, heterocycle, hydrocarbyl, hydrocarbyl, hydrocarbylcarbonyl, hydrocarbyloxycarbonyl, hydrocarbylcarbonyloxy, hydrocarbylene, organosulfinyl, hydroxyl, organosulfinyl, organosulfonyl, sulfinyl, sulfonyl, sulfonylamino, and sulfuryl; and

each $Y^1$ is independently selected from the group consisting of O, S, Se, $NR^6$, N-$OR^6$, and $CR^6R^7$.

In a preferred embodiment, the modifying group is selected from the group consisting of: O-(p-toluene)sulfonate; O-phosphate; O-nitrate; O-[4-methoxy]-tetrahydropyranyl; O-[4-methoxy]-tetrahydrothiopyranyl; O-tetrahydrothiopyranyl; O-[5-methyl]-tetrahydrofuranyl; O-[2-methyl,4-methoxy]-tetrahydropyranyl; O-[5-methyl]-tetrahydropyranyl; O-tetrahydropyranyl; O-tetrahydrofuranyl; O-phenoxyacetyl; O-methoxyacetyl; O-acetyl; O-C(O)-$OCH_3$; O-C(O)-$CH_2CH_2CN$; and O-C(S)-$OCH_3$. In some particularly preferred embodiments, the modifying group is selected from the group consisting of O-methoxytetrahydropyranyl; O-tetrahydropyranyl; and O-tetrahydrofuranyl.

In another embodiment, a modified oligonucleotide primer is a compound represented by formula V

[Chemical formula 2]

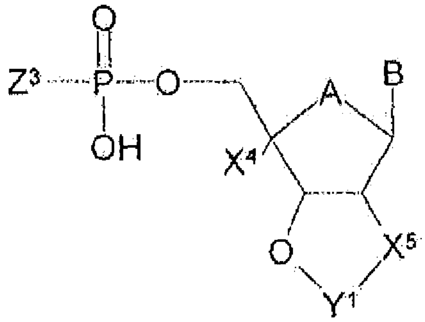

wherein

$Z^3$ is a 3'-O-oligonucleotidyl residue or an oligonucleotide primer;
B is selected from a substituted or non-substituted purine or pyrimidine, any aza or deaza derivative thereof, or any "universal base" or "degenerate base" of any NTP analog which is preferably recognizable by a nucleic acid polymerase;
A is selected from the group consisting of O, S, Se, $CR^1R^2$, and $NR^1$;
each $R^1$ and each $R^2$ is independently selected from the group consisting of H, F, Cl, Br, I, $OR^3$, $SR^3$, $NR^3R^4$, $C(Y)R^5$, substituted or unsubstituted alkyl, alkenyl, alkynyl, aryl, and aralkyl, wherein any substituent may each optionally contain one or more heteroatoms;
each Y is independently selected from the group consisting of O, S, Se, $CR^1R^2$, and $NR^1$;
each $R^3$ and each $R^4$ is independently selected from the group consisting of H, substituted or unsubstituted alkyl, substituted or unsubstituted alkenyl, substituted or unsubstituted alkynyl, substituted or unsubstituted aryl, and substituted or unsubstituted aralkyl, wherein any substituent may each optionally contain one or more heteroatoms;
each $R^5$ is independently selected from the group consisting of H, F, Cl, Br, $OR^3$, $SR^3$, $NR^3R^4$, substituted or unsubstituted alkyl, substituted or unsubstituted alkenyl, substituted or unsubstituted alkynyl, substituted or unsubstituted aryl, and substituted or unsubstituted aralkyl, wherein any substituent may each optionally contain one or more heteroatoms;
$X^4$ is independently selected from the group consisting of $R^1$, F, Cl, Br, I, $OR^3$, $SR^3$, $SeR^3$, $NR^3R^4$, $NR^3OR^3$, $NR^3$-$NR^3R^4$, CN, $N_3$, $C(Y)R^5$, $NO_2$, CN, and $SSR^3$;
$X^5$ is selected from the group consisting of O, S, Se, $NR^6$, N-$OR^6$, and $CR^6R^7$;
$Y^1$ is selected from the group consisting of O, S, Se, $NR^6$, N-$OR^6$, $CR^6R^7$, and C(Y);
each $R^6$ and each $R^7$ is independently selected from the group consisting of hydrogen, and a straight or branched optionally substituted hydrocarbyl group having from 1 to 20 carbon atoms, preferably 1 to 10 carbon atoms, and preferably 1 to 6 carbon atoms, wherein
the hydrocarbyl is alkyl, alkenyl or alkynyl which may include at least one substituent selected from the group consisting of halo, oxo, hydroxyl, alkoxy, amino, amido, cycloalkyl, heterocycloalkyl, aryl, aryloxy, and heteroaryl; and $X^5$ and $Y^1$ may each be optionally covalently attached through appropriate atoms or group of atoms to $X^4$, $X^5$, $Z^3$, A, W, or B portion of the NTP molecule depicted in Formula IB.

In a specific embodiment of formula V, B is thymine, cytosine, adenine, guanine, uracil, aminoallyl-uracil, 7-deazaguanine, 7-deaza-7-methylguanine, 7-deaza-7-iodoguanine, 7-deaza-7-aminoallyl-guanine, 7-deaza-8-azaguanine, 7-deazadenine, 2,6-diaminopurine, 5-nitro-cytosine, 5-aminoallyl-cytosine, 5-(Biotin-16)-cytosine, 5-(Fluorescein-11)-cytosine, 4-methylamino-cytosine, and 2-thio-5-methyluracil, or 4-thio-5-methyluracil.
In a preferred embodiment of formula V, B is adenine, guanine, cytosine, thymine, or uracil.
In a preferred embodiment, a modified oligonucleotide primer is one of the compounds selected from the group consisting of:

[Chemical formula 3]

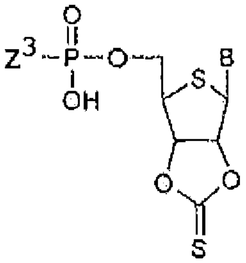
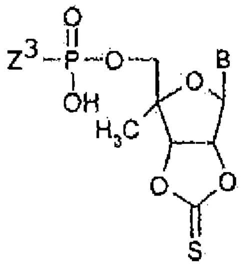
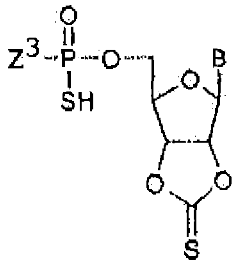

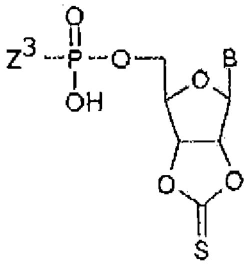
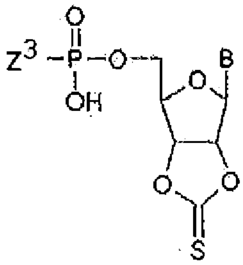

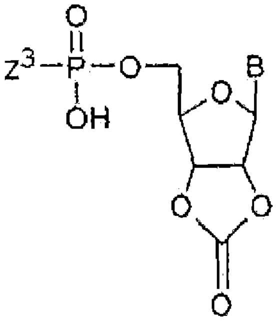
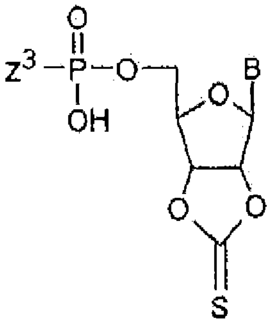
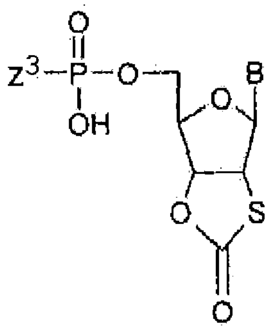

The modified oligonucleotide primer of 1-1 can be manufactured by the method described in US Patent No. 8361753.

(1-2) Modified oligonucleotide primers comprising a thermolabile modifying group in one or more internucleotide bonds (US Patent No. 8361753)

In one embodiment, a modifying group in the modified oligonucleotide primer comprises a compound of formula I:

[Chemical formula 4] $-L-X-R^1$

wherein

L is a straight or branched optionally substituted hydrocarbylene group having from 1 to 10 carbon atoms, preferably 2 to 5 carbon atoms, more preferably 3 to 4 carbon atoms, and still more preferably 4 carbon atoms;
X is O, S, S(O), $S(O)_2$, C(O), C(S), or C(O)NH; and
$R^1$ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In one embodiment, a modifying group provides a compound of formula 1a:

[Chemical formula 5]

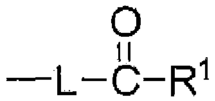

wherein

L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and $R^1$ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

Preferred embodiments of a modifying group of formula Ia are the following:

[Chemical formula 6]

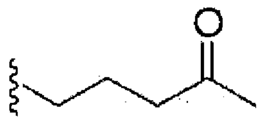

4-oxo-1-pentyl

[Chemical formula 7]

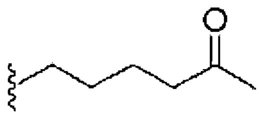

5-oxo-1-hexyl

[Chemical formula 8]

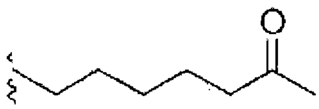

6-oxo-1-heptyl

[Chemical formula 9]

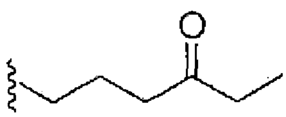

4-oxo-1-hexyl

[Chemical formula 10]

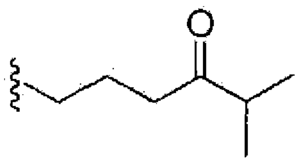

5-methyl-4-oxo-1-hexyl

[Chemical formula 11]

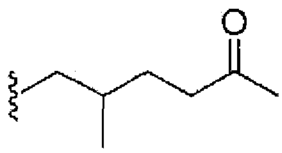

2-methyl-5-oxo-hexyl

[Chemical formula 12]

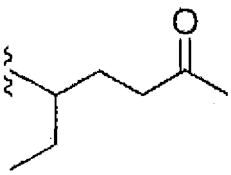

1-ethyl-4-oxo-pentyl

[Chemical formula 13]

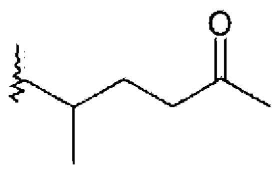

1-methyl-4-oxo-pentyl

[Chemical formula 14]

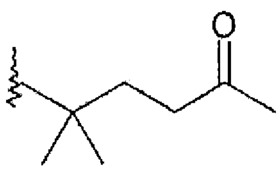

1,1-dimethyl-4-oxo-pentyl

[Chemical formula 15]

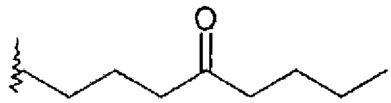

4-oxo-1-octyl

[Chemical formula 16]

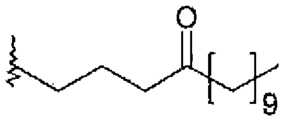

4-oxo-1-tetradecyl, and

[Chemical formula 17]

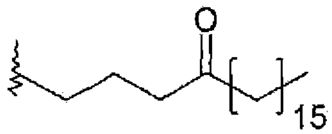

4-oxo-1-eicosanyl.

In one embodiment, a modifying group provides a compound of formula Ib:

[Chemical formula 18] $-L-S(O)_k-R^1$

wherein

k is an integer from 0 to 2;
L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
$R^1$ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In a preferred embodiment, a modifying group of formula Ib is 4-methylthio-1-butyl described below:

[Chemical formula 19]

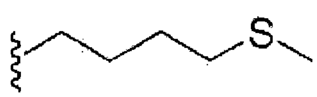

In one embodiment, a modifying group provides a compound of formula Ic:

[Chemical formula 20]

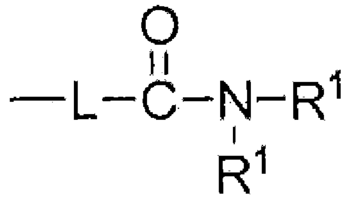

wherein

L is a straight or branched optionally substituted hydrocarbylene group having from 1 to 10 carbon atoms, preferably 2 to 5 carbon atoms, more preferably 3 to 4 carbon atoms, and still more preferably 4 carbon atoms; and
$R^1$ is hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In a preferred embodiment, a modifying group of formula Ic is 3-(N-tert-butylcarboxamide)-1-propyl described below:

[Chemical formula 21]

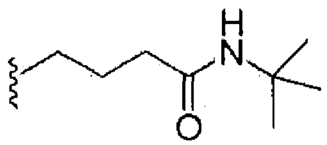

In one embodiment, a modifying group provides a compound of formula Id:

[Chemical formula 22]

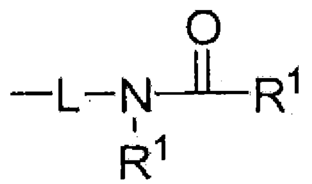

wherein

L is a straight or branched hydrocarbylene group having from 1 to 10 carbon atoms, preferably 2 to 5 carbon atoms, more preferably 3 to 4 carbon atoms, and still more preferably 4 carbon atoms; and
each $R^1$ is independently hydrogen or a straight or branched optionally substituted hydrocarbyl group having from 1 to 20 carbon atoms, preferably 1 to 10 carbon atoms, and more preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

Examples of a preferred embodiment of a modifying group formula Id includes 2-(N-formyl-N-methyl)aminoethyl and 2-(N-acetyl-N-methyl)aminoethyl (described below):

[Chemical formula 23]

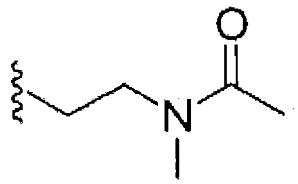

2-(N-acetyl-N-methyl)aminoethyl

In another embodiment, a modifying group provides a compound of formula II:

[Chemical formula 24] $-L-R^2$

wherein

L is a straight or branched hydrocarbylene group having from 1 to 10 carbon atoms, preferably 2 to 5 carbon atoms, more preferably 3 to 4 carbon atoms, and still more preferably 4 carbon atoms; and
$R^2$ is hydrogen, cyano, or optionally substituted carbocyclic ring, heterocycle, aryl, or heteroaryl having from 5 to 10 atoms.

In a preferred embodiment, a modifying group of formula II is N-(2-hydroxyethyl)-phthalimide described below:

[Chemical formula 25]

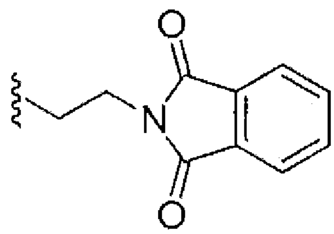

N-(2-hydroxyethyl)-phthalimide

In another embodiment, a modifying group provides a compound of formula III:

[Chemical formula 26] $-L^a-A-L^b-B$

wherein

$L^a$ and $L^b$ is each independently selected from a single bond or a straight or branched optionally substituted hydrocarbylene group having a single bond or 1 to 8 carbon atoms, preferably 2 to 5 carbon atoms, and more preferably 3 to 4 carbon atoms;
A is O, S, S(O), $S(O)_2$, Se, $CR^3R^4$, $NR^3$, C(O), C(S), or $CNR^3$;
B is $C(O)R^3$, $C(S)R^3$, $C(O)NR^3R^4$, $OR^3$, or $SR^3$;
$R^3$ and $R^4$ is each independently hydrogen or a straight or branched optionally substituted hydrocarbyl group having from 1 to 20 carbon atoms, preferably 1 to 10 carbon atoms, and preferably 1 to 6 carbon atoms, wherein hydrocarbyl is preferably alkyl, alkenyl or alkynyl which may optionally include at least one substituent selected from the group consisting of halo, oxo, hydroxyl, alkoxy, amino, amido, cycloalkyl, heterocycloalkyl, aryl, aryloxy, and heteroaryl.

In another embodiment, a modifying group provides a compound of formula IV:

[Chemical formula 27] $-L^a-D-L^b-E-L^e-F$

wherein

$L^a$, $L^b$, and $L^c$ are each independently selected from a single bond or a straight or branched optionally substituted hydrocarbylene group having or 1 to 8 carbon atoms, preferably 2 to 5 carbon atoms, and more preferably 3 to 4 carbon atoms;
D is O, S, S(O), $S(O)_2$, $CR^5R^6$, or $NR^5$;
E is O, S, S(O), $S(O)_2$, $CR^5R^6$, or $NR^6$;
F is hydrogen, $C(O)R^7$, $C(S)R^7$, $C(O)NR^7R^8$, $OR^7$, or $SR^7$;
$R^5$ and $R^6$ are each independently hydrogen, aryl, alkyl, halo, oxo, hydroxyl, alkoxy, aryloxy, or amino, or $R^5$ and $R^6$ may together form a monocycle or bicycle comprising D, $R^5$, $R^6$, E and $L^b$, consisting of 5 to 10 atoms, wherein if $R^5$ and $R^6$ together form a ring, n is from 0 to 2; and
$R^7$ and $R^8$ are each independently selected from aryl, alkyl, halo, oxo, hydroxyl, alkoxy, aryloxy, amino, amido, optionally substituted cycloalkyl, optionally substituted heterocycloalkyl, optionally substituted aryl, optionally substituted aryloxy, or optionally substituted heteroaryl.

In one embodiment of a compound of formula IV wherein $R^5$ and $R^6$ together form a ring, a modifying group is methoxymethyl-cyclohexy-1,3-yl-ethyl described below:

[Chemical formula 28]

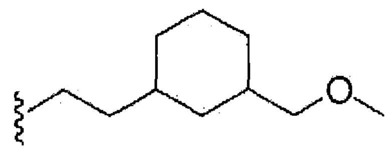

methoxymethyl-cyclohexy-1,3-yl-ethyl

In one embodiment, a modified oligonucleotide primer has a modified backbone of structure I:

[Chemical formula 29]

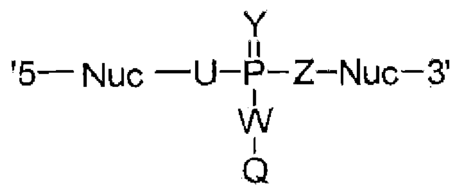

wherein

Nuc is a nucleoside in a primer sequence;
U and Z are independently O, S, Se, $NR^9$, or $CR^9R^{10}$;
$R^9$ and $R^{10}$ are each independently hydrogen or a straight or branched optionally substituted hydrocarbyl having from 1 to 10 carbon atoms; wherein the hydrocarbyl is preferably alkyl, alkenyl or alkynyl which may each include at least one substituent selected from halo, oxo, hydroxyl, alkoxy, aryloxy, amino, amido, or a detectable label;
Y is O, S, or Se;
W is any chemical component that enables Q to be thermally cleaved such as O, S, S(O), $S(O)_2$, Se, C(O), C(S), C(O)NH, C(N)H, NH, $-C(=NR^{11})-$, or $NR^9$;
$R^{11}$ is hydrogen or an optionally substituted hydrocarbyl having 1 to 10 carbon atoms, preferably 1 to 6 carbon atoms, wherein $R^{11}$ is preferably H, alkyl, or lower alkyl; and
Q is a modifying group comprising one or more thermally cleavable groups.

In one embodiment, modifying group Q comprises one or more thermally cleavable group selected from formulas I, Ia, Ib, Ic, Id, II, III, and IV.

A modified oligonucleotide primer comprises one of the aforementioned modifying groups in at least one internucleotide bonds. A modified oligonucleotide primer preferably comprises one or more of the aforementioned modifying groups at the 3' terminus thereof. A modified oligonucleotide primer preferably comprises one or more of the aforementioned modifying groups in one of the last 6 internucleotide bonds, preferably one of the last three internucleotide bonds, at the 3' terminus thereof.

In another embodiment, an oligonucleotide primer can comprise a sequence with 2, 3, 4, 5, or 6 consecutive modified internucleotide bonds ending at the 3' terminus of the oligonucleotide primer. In still another embodiment, an oligonucleotide primer may comprise a plurality of non-consecutive 3' modified internucleotide bonds. The 5' terminus of the modified oligonucleotide primer may also have a sequence of a nucleotide comprising a modified internucleotide bond. In yet another embodiment, a11 internucleotide bonds of an oligonucleotide may be modified.

In another preferred embodiment, a modified oligonucleotide primer comprises a modifying group in a 3' n internucleotide bonds of an oligonucleotide primer, wherein n is an internucleotide bond at the 3' terminus. In yet another embodiment, a modifying group is present in 3' n-1, n-2, n-3, or n-4 internucleotide bond of an oligonucleotide. In yet another embodiment, an oligonucleotide has modifying groups of 2 or more at positions n, n-1, n-2, n-3, n-4, n-5, and n-6; preferably 2 or more at positions n, n-1, n-2, n-3, n-4, n-5, and n-6; preferably 3 or more at positions n, n-1, n-2, n-3, n-4, n-5, and n-6; preferably 4 or more at positions n, n-1, n-2, n-3, n-4, n-5, and n-6; preferably 5 or more at positions n, n-1, n-2, n-3, n-4, n-5, and n-6; or preferably 6 or more at positions n, n-1, n-2, n-3, n-4, n-5, and n-6.

The modified oligonucleotide primer of 1-2 can be manufactured by the method described in US Patent No. 8361753.

(2) Oligonucleotide primers having one or more complementary regions on a sequence of the same modified oligonucleotide primer and having a turn structure by the complementary regions prior to initial thermal denaturation of PCR to form an intermolecular hairpin loop to exhibit a structure masking a sequence that is complementary to a partial sequence of a template RNA

This embodiment has one or more complementary regions on a sequence of the same modified oligonucleotide primer and has a turn structure by the complementary region prior to initial thermal denaturation processing of PCR to form an intermolecular hairpin loop. The complementary regions refer to a combination of a first sequence comprised of one or more oligonucleotides and a second sequence comprising one or more oligonucleotides that are complementary thereto.

The first and second sequences may be posited adjacent to each other or positioned with one or more oligonucleotides interposed therebetween. If the first sequence or the second sequence comprises a sequence that is complementary to a partial sequence of a template RNA, the sequence that is complementary to the partial sequence of the template RNA is masked by a complementary bond of the first and second sequences. Therefore in such a case, the number of oligonucleotides of the first and second sequences is not particularly limited.

If the first and second sequences do not comprise a sequence that is complementary to a partial sequence of a template RNA, a sequence that is complementary to a partial sequence of a template RNA is comprised between oligonucleotides of the first and second sequences, and the sequence that is complementary to the partial sequence of the template RNA is masked by an intermolecular hairpin loop formation.

Since a sequence that is complementary to a partial sequence of a template RNA is masked, it is unable to hybridize to a corresponding partial sequence of the template RNA upon reverse transcription, so that the primer function is partially or completely blocked. However, since a hairpin loop structure dissociates to expose the sequence that is complementary to the partial sequence of the template RNA at a denaturation temperature in PCR amplification (e.g., about 55 to 105°C, preferably about 85 to 100° C, and more preferably about 90 to 96° C (e.g., 95° C)), the sequence can hybridize to a corresponding partial sequence in a cDNA at a subsequent pairing temperature (i.e., acquires a primer function). The length of the loop portion of the hairpin loop is generally about 5 to 25 bases. The nucleotide sequence of the loop portion is not particularly limited, as long as an intermolecular hairpin loop can be formed.

(3) Oligonucleotide primers comprising an artificial base

The modified oligonucleotide primer of this embodiment comprises an artificial base (non-naturally occurring base), so that the complementary sequence of a nucleotide sequence of the modified oligonucleotide primer is substantially non-existent in a template RNA (template RNA free of an artificial base). For this reason, hybridization of the modified oligonucleotide primer to the template RNA is suppressed, so that the primer function in reverse transcription would be partially or completely blocked. In one embodiment, 1 or more bases, preferably 3 or more bases, 5 or more bases, 10 or more bases, 12 or more bases, or preferably all 15 bases among the 15 bases at the 3' terminus of the modified oligonucleotide primer are artificial bases. In a preferred embodiment, the base at the most 3' end of the modified oligonucleotide primer is an artificial base.

The modified oligonucleotide primer in this embodiment is used in combination with an oligonucleotide primer

for initiating reverse transcription, comprising a partial sequence comprising an artificial base of the modified oligonucleotide primer. The length of the partial sequence comprising an artificial base is 10 to 40 bases, preferably 15 to 30 bases, and more preferably 18 to 25 bases. A partial sequence comprising an artificial base, while not particularly limited, can be for example a partial sequence of the 3' terminus of the modified oligonucleotide primer. The oligonucleotide primer for initiating reverse transcription comprises a sequence that is complementary to a partial sequence of a template RNA and the partial sequence comprising the artificial base, and the partial sequence comprising the artificial base is added to the 5' side of the sequence that is complementary to the partial sequence of the template RNA. The length of the partial sequence of the template RNA is not particularly limited, but is generally 10 to 40 bases, preferably 15 to 30 bases, and more preferably 18 to 25 bases. The partial sequence can be a partial sequence of the 3' terminus of a region intended to be amplified in the template RNA. An oligonucleotide primer for initiating reverse transcription preferably comprises a sequence that is complementary to a partial sequence of a template RNA at the 3' terminus thereof.

When such a combination is used to perform one-step reverse transcription template switching PCR, a cDNA with a partial sequence comprising an artificial base of a modified oligonucleotide primer added to the 5' terminus is synthesized in reverse transcription. The modified oligonucleotide primer comprising an artificial base acquires, as a result thereof, a primer function in PCR using the cDNA as a template. In addition, a region of interest can be specifically modified by PCR amplification using said cDNA as a template and the modified oligonucleotide primer as one of the primers.

Examples of artificial bases include, but are not limited to, Z base/F base (Proc. Natl. Acad. Sci. USA 1997, 94, 105061; Nat. Struct. Biol. 1998, 5, 950; Nat. Struct. Biol. 1998, 5, 954), Q base (J. Am. Chem. Soc. 1999, 121, 2323), iso-G base/iso-C base (J. Am. Chem. Soc. 1989, 111, 8322), 2-thio T ($T^S$) base (Nucleic Acids Res. 2005, 33, 5640), P base/Z base (Nucleic Acids Res. 2007, 35, 4238), PICS base (J. Am. Chem. Soc. 1999, 121, 11585), 5SICS base/MMO2 base/NaM base (J. Am. Chem. Soc. 2009, 131, 14620), 2-amino-6-dimethylaminopurine(x)/2-oxopyridine(y)(Proc. Natl. Acad. Sci. USA 2001, 98, 4922), 2-amino-6-(2-thienyl)purine (s) (J. Am. Chem. Soc. 2005, 127, 17286; Nucleic Acids Res. 2005, 33, e129; Biotechniques 2006, 40, 711), imidazolin-2-one(z) (J. Am. Chem. Soc. 2004, 126, 13298), Ds base/Pa base (Nat. Methods 2006, 3, 729), Pn base (J. Am. Chem. Soc. 2007, 129, 15549), Px base (Nucleic Acids Res. 2009, 37, e14), xA base, xT base (J. Am. Chem. Soc. 2004, 126, 11826), Im-$N^O$ base/Na-$O^N$ base, Im-$O^N$ base/Na-$N^O$ base (J. Am. Chem. Soc. 2009, 131, 1644; and Angew. Chem. Int. Ed. 2005, 44, 596), and the like. These artificial bases can contribute to reverse transcription and/or PCR amplification by forming the following base pairs: Z-F base pair, Q-F base pair, isoG-isoC base pair, A-$T^S$ base pair, P-Z base pair, PICS-PICS base pair (self-complementary), 5SICS-MMO2 base pair, 5SICS-NaM base pair, x-y base pair, s-y base pair, s-z base pair, Ds-Pa base pair, Ds-Pn base pair, Ds-Px base pair, xA-T base pair, A-xT base pair, Im-$N^0$-Na-$O^N$ base pair, and Im-$O^N$-Na-$N^0$ base pair.

The method of the present invention is described hereinafter in further detail.

The method of the present invention first provides a composition comprising all reagents (excluding oligonucleotide primers that initiate reverse transcription) that are required for template switching reverse transcription of a template RNA into a cDNA, and for PCR amplification of at least a part of the cDNA, including

i) a template switching oligonucleotide,
ii) a primer set consisting of a 5' anchor oligonucleotide primer comprising at least a part of an anchor sequence comprised in the template switching oligonucleotide, and the modified oligonucleotide primer, and
iii) the template RNA.

The template switching oligonucleotide comprises an anchor sequence and a sequence that is complementary to a sequence added to the 3' terminus of a newly synthesized cDNA (also simply referred to as an RT addition sequence) by the terminal transferase activity of the reverse transcriptase when a reverse transcriptase has reached the 5' terminus of the template RNA, and an anchor sequence (first anchor sequence) is added to the 5' terminus of a complementary sequence of the RT addition sequence. Preferably, the complementary sequence of the RT addition sequence is positioned at the 3' terminus of the template switching oligonucleotide. The RT addition sequence is dependent on the type of reverse transcriptase. For example, a Moloney Murine Leukemia Virus derived reverse transcriptase (MMLV RT) adds a short cytosine rich sequence (e.g., CC, CCC, or CCCC) to the 3' terminus of the synthesized cDNA. Thus, a short guanine rich sequence (e.g., GG, GGG, or GGGG), which is the complementary sequence thereof, is comprised in the template switching oligonucleotide as the complement sequence of the RT addition sequence. An anchor sequence refers to an artificial sequence that is added to the 5' terminus of an oligonucleotide. An anchor sequence is preferably a sequence that does not exist in the nature. The length of an anchor sequence is not particularly limited, but is generally about 10 bases to 100 bases, and preferably about 15 bases to about 50 bases.

A template switching oligonucleotide may be a DNA or an RNA, or a DNA/RNA chimera. To efficiently function as a template in reverse transcription, a template switching oligonucleotide is optimally an RNA or a DNA/RNA chimera, and more preferably a DNA/RNA chimera. In one embodiment, a part of a complementary sequence of an RT addition sequence is an RNA, and a part of an anchor sequence is a DNA or a DNA/RNA chimera. A template switching oligonucleotide also functions as the 5' anchor oligonucleotide primer explained below. Therefore, in some embodiments, a 5' anchor oligonucleotide primer can be omitted or added at a small amount. There has been no example of performing reverse transcription template switching and PCR amplification in the same reaction system. The Examples herein are the first to demonstrate that a template switching oligonucleotide functions as a forward primer of PCR amplification.

A 5' anchor oligonucleotide primer comprises a part or all of the anchor sequence (first anchor sequence) comprised in the template switching oligonucleotide. The length of a part or all of the anchor sequence is generally 10 to 40 bases, preferably 15 to 30 bases, and more preferably 18 to 25 bases. The primer is a DNA or a DNA/RNA chimera and preferably a DNA so that it can function as a primer in PCR. A 5' anchor oligonucleotide primer can be a forward primer in PCR.

Examples of a template RNA that can be used include, but are not limited to, mRNA, rRNA, tRNA, non-coding RNA, chemically synthesized RNA, and the like. The mRNA, rRNA, and tRNA may be derived from any cell or tissue. The mRNA, rRNA, and tRNA may be collected from a small amount of cell/tissue (e.g., single cell) obtained by utilizing a cell sorter or the like. The mRNA, rRNA, and tRNA may be in a form contained as a part of a total RNA.

The composition comprises all of the reagents (excluding oligonucleotide primers that initiate reverse transcription) that are required for template switching reverse transcription of the template RNA into a cDNA and for PCR amplification of at least a part of the cDNA. In addition to the aforementioned template switching oligonucleotide, primer set, and template RNA, examples of the reagent include the following.

*Reverse transcriptase (RNA dependent DNA polymerase)
*Heat resistant DNA polymerase (DNA dependent DNA polymerase)
*dNTPs mixture

To form an RT addition sequence to the 3' terminus of a cDNA, a reverse transcriptase that is used has terminal transferase activity. Examples of reverse transcriptases with terminal transferase activity include, but are not limited to, Moloney Murine Leukemia Virus derived reverse transcriptases (MMLV RT). Terminal transcriptase activity is preferably activity of adding a short cytosine rich sequence (e.g., CC, CCC, or CCCC) to the 3' terminus of a synthesized cDNA.

Representative examples of heat resistant DNA polymerases include, but are not limited to, Taq, Tth, KOD, Pfu, Bst, and the like. Various heat resistant DNA polymerases that can be used in PCR have been developed, which can all be used in the present invention. Heat resistant DNA polymerases that can be used in PCR are well known to, and appropriately selectable by, those skilled in the art.

In one embodiment, the composition further comprises an oligonucleotide primer that initiates reverse transcription. An oligonucleotide primer that initiates reverse transcription initiates reverse transcription by hybridizing to a template RNA due to comprising a sequence that is complementary to a partial sequence of a template RNA. The length of the partial sequence is not particularly limited, but is generally 10 to 40 bases, preferably 15 to 30 bases, and more preferably 18 to 25 bases. An oligonucleotide primer that initiates reverse transcription preferably comprises a sequence that is complementary to a partial sequence of a template RNA at the 3' terminus thereof. An anchor sequence (second anchor sequence) may be added to the 5' terminus of a sequence that is complementary to a partial sequence of a template RNA. A second anchor sequence is preferably a sequence that does not exist in nature. The length of a second anchor sequence is not particularly limited, but is generally about 10 bases to 100 bases, and preferably about 15 bases to 50 bases. A second anchor sequence is preferably non-identical to the first anchor sequence. In one embodiment, a second anchor sequence comprises an artificial base. In one embodiment, an oligonucleotide primer that initiates reverse transcription does not comprise a second anchor sequence. An oligonucleotide primer that initiates reverse transcription is a primer that is specific to a specific gene, an oligo dT primer that binds to a poly-A tail of mRNA, or a random primer such as a random hexamer primer, but is preferably a primer that is specific to a specific gene. Said primer comprises a sequence that is complementary to a partial sequence of an RNA (e.g., mRNA) encoding a gene of interest. An oligonucleotide primer that initiates reverse transcription is a DNA or a DNA/RNA chimera and preferably a DNA so that the primer can function as a primer in reverse transcription.

In one embodiment, a region where an oligonucleotide primer that initiates reverse transcription hybridizes and a region where the modified oligonucleotide primer hybridizes on a template RNA at least partially overlap. The length of an overlapping hybridization region is not particularly limited, but is generally 10 bases or greater, preferably 15 bases or greater, and more preferably 18 bases or greater. The length of an overlapping hybridization region can be, for example, 40 bases or less, 30 bases or less, or 25 bases or less.

In a preferred embodiment, the 5' terminus of a region of a template RNA where a modified oligonucleotide primer hybridizes is positioned closer to the 5' side (upstream) of the template RNA than the 5' terminus of a region of the template RNA where an oligonucleotide primer that initiates reverse transcription hybridizes. In other words, both primers are designed so that the 3' terminus of the modified oligonucleotide primer hybridizes with the template RNA closer to the 5' side (upstream) of the template RNA than the 3' terminus of the oligonucleotide primer that initiates reverse transcription. Improvement in the specificity of amplification can be expected by designing the two primers in

such a semi-nested positional relationship. In such a case, the region of the template RNA where the oligonucleotide primer that initiates reverse transcription hybridizes and the region of the template RNA where the modified oligonucleotide primer hybridizes may be positioned to partially overlap in a semi-nested form, or positioned in a full-nested form without overlap.

When the region of the template RNA where the oligonucleotide primer that initiates reverse transcription hybridizes partially overlaps the region of the template RNA where the modified oligonucleotide primer hybridizes, both primers are preferably designed so that the 5' terminus of the region of the template RNA where the modified oligonucleotide primer hybridizes is closer to the 5' side (upstream) of the template RNA than the 5' terminus of the region of the template RNA where the oligonucleotide primer that initiates reverse transcription hybridizes by, for example, 1 to 12 bases, preferably 1, 2, 3, 4, or 5 bases (i.e., so that the 3' terminus of the modified oligonucleotide primer hybridizes closer to the 5' side of the template RNA than the 3' terminus of the oligonucleotide primer that initiates reverse transcription by, for example, 1 to 10 bases, and preferably 1, 2, 3, 4 or 5 bases), but the design is not limited thereto.

In another embodiment, a region of a template RNA where an oligonucleotide primer that initiates reverse transcription hybridizes and a region of the template RNA where the modified oligonucleotide primer hybridizes at least partially overlap, and the 5' terminus of the region of the template RNA where the modified oligonucleotide primer hybridizes matches the 5' terminus of region of the template RNA where the oligonucleotide primer that initiates reverse transcription hybridizes. In other words, the 3' terminus of the modified oligonucleotide primer hybridizes with the template RNA at the same position as the 3' terminus of the oligonucleotide primer that initiates reverse transcription.

In one embodiment, a region of a template RNA where an oligonucleotide primer that initiates reverse transcription hybridizes and a region of the template RNA where the modified oligonucleotide primer hybridizes are identical. In this embodiment, the oligonucleotide primer that initiates reverse transcription can be an unmodified oligonucleotide primer corresponding to the modified oligonucleotide primer.

In another embodiment, the modified oligonucleotide primer comprises a partial sequence of an oligonucleotide primer that initiates reverse transcription at the 3' terminus thereof. The length of said partial sequence (hereinafter, also called a common sequence) is not particularly limited, but is generally 10 bases or greater, preferably 15 bases or greater, and more preferably 18 bases or greater. The length of said 3' terminus partial sequence can be, for example, 40 bases or less, 30 bases or less, or 25 bases or less. In one embodiment, said common sequence can be a partial sequence of the 3' terminus of an oligonucleotide primer that initiates reverse transcription. In another embodiment, the 3' terminus of said common sequence is positioned closer to the 5' side than the 3' terminus of the oligonucleotide primer that initiates reverse transcription by at least 1 base (e.g., 1 to 20 bases, 1 to 10 bases, or 1 to 8 bases). In one embodiment, said common sequence is a sequence that is complementary to a partial sequence of a template RNA, or a partial sequence thereof, comprised in an oligonucleotide primer that initiates reverse transcription. In one embodiment, said common sequence is a second anchor sequence or a partial sequence thereof. In one embodiment, said common sequence is a partial sequence of an oligonucleotide primer that initiates reverse transcription, which straddles a sequence that is complementary to a partial sequence of a template RNA and a second anchor sequence. In one embodiment, the modified oligonucleotide primer is an oligonucleotide primer comprising an artificial base, and an oligonucleotide primer that initiates reverse transcription comprises a second anchor sequence comprising an artificial base at the 5' terminus, and a common sequence is a second anchor sequence or a partial sequence thereof.

If the compound comprises an oligonucleotide primer that initiates reverse transcription, the concentration of the oligonucleotide primer may be an amount that is sufficient for initiating reverse transcription. If one copy of a cDNA comprising a region intended to be amplified can be synthesized, this can be amplified to a detectable level by the subsequent PCR. Therefore, the composition (reaction system) only needs to comprise at least one copy, preferably 10 copies or more, and more preferably 100 copies or more of oligonucleotide primer that initiates reverse transcription. If the concentration of the oligonucleotide primer that initiates reverse transcription is too high, side reactions due to non-specific hybridization can be induced. The concentration of the oligonucleotide primer that initiates reverse transcription in the composition is for example about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less.

In another embodiment, the composition does not comprise an oligonucleotide primer that initiates reverse transcription. In this embodiment, an oligonucleotide primer comprising a thermolabile modifying group and a sequence that is complementary to a partial sequence of a template RNA is used as the modified oligonucleotide primer. The modified oligonucleotide primer preferably comprises a thermolabile modifying group at the 3' terminus or one or more internucleotide bonds. A thermolabile modifying group comprised in the modified oligonucleotide primer hardly dissociates until reaching the first denaturation temperature (e.g., about 80 to 105°C, preferably about 85 to 100° C, and more preferably about 90 to 96° C (e.g., 95° C)) in PCR amplification. Meanwhile, the inventors have found that such a thermolabile modifying group slightly dissociates at a temperature where reverse transcription progresses (e.g., 45° C), and a corresponding unmodified oligonucleotide generated as a result thereof can function as an oligonucleotide primer that initiates reverse transcription.

The composition may comprise a buffer, salt (magnesium ion or the like), or RNAase inhibitor as needed.

The concentration of a template switching oligonucleotide comprised in the composition is not particularly limited as long as the method of the present invention can be practiced, but is, for example, about 0.05 to 5.0 μM and preferably 0.1 to 1.0 μM.

The concentration of the modified oligonucleotide primer and 5' anchor oligonucleotide primer comprised in the composition is equivalent to the primer concentration for conventional PCR, such as about 0.1 to 1.0 μM.

The concentration of other constituents (template RNA, reverse transcriptase, heat resistant DNA polymerase, dNTPs mixture, buffer, salt, and RNAase inhibitor) that can be contained in the composition is well known in prior art one-step RT-PCR. The concentration used in the context of the present invention can also be optimized from routine experimentation.

Next, the composition provided above is incubated at a temperature where reverse transcription can progress. A temperature at which reverse transcription can progress can be appropriately adjusted depending on the type of reverse transcriptase, but is generally 37°C to 62°C and preferably 37°C to 55°C. Incubation time can be appropriately adjusted while considering the size of a template RNA or the like, but is generally 30 seconds to 120 minutes and preferably 5 minutes to 60 minutes. With the incubation, an oligonucleotide primer that initiates reverse transcription comprised in the composition or an unmodified oligonucleotide generated by dissociation of a thermolabile modifying group from a modified oligonucleotide primer primes reverse transcription to synthesize a cDNA (antisense strand) that is complementary to a template RNA. A reverse transcriptase, after reaching the 5' terminus of the template RNA, switches the template to a template switching oligonucleotide and continues cDNA synthesis to the 5' end thereof, thus producing a single stranded cDNA (antisense strand) to which a sequence that is complementary to an anchor sequence of the template switching oligonucleotide is added on the 3' end.

Next, a reaction mixture comprising the resulting cDNA is subjected to a plurality of rounds of a thermal cycling protocol with which PCR can progress. A cycle of the thermal cycling protocol is comprised of a three temperature steps, i.e., denaturation (also called thermal denaturation), annealing, and extension. Denaturation is not particularly limited as long as the temperature is sufficient for dissociating a double stranded DNA. The preferred lower limit and upper limit of the thermal denaturation temperature are 90° C and 100°C, respectively. Annealing is a step of annealing a primer to a dissociated DNA. The temperature in this step (annealing temperature) is not particularly limited, but the lower limit of the annealing temperature is preferably 45° C and more preferably 50°C. Meanwhile, the upper limit is preferably 75° C and more preferably 70° C. Extension is a step of synthesizing a complementary strand with a DNA polymerase. The temperature at this time (extension temperature) is not particularly limited, but the lower limit and the upper limit of a preferred extension temperature is 50°C and 80°C, respectively. In this cycle, the annealing temperature does not exceed the extension reaction temperature. The annealing and extension can be performed at one temperature to configure a thermal cycling protocol as a cycle of substantially two temperature steps. In such a case, the lower limit of a temperature for annealing and extension is preferably 50° C and more preferably 55° C. Meanwhile, the upper limit is preferably 70° C and more preferably 65° C. Examples of incubation time in each step include 1 second to 5 minutes, but those skilled in the art can readily determine a suitable incubation time while considering the size of amplification product or the like.

A denaturation step (pre-incubation step) can be performed to inactive a reverse transcriptase before subjecting a reaction mixture to a thermal cycling protocol. The denaturation temperature is not particularly limited as long as a reverse transcriptase can be inactivated, but the lower limit and the upper limit of a preferred thermal denaturation temperature are 90° C and 100° C, respectively. The denaturation time is not particularly limited as long as a reverse transcriptase can be inactivated, but is generally 1 minute to 15 minutes.

If an oligonucleotide primer comprising a thermolabile modifying group is used as a modified oligonucleotide primer, a modifying group is dissociated from a modified oligonucleotide primer and the primer is converted to a corresponding unmodified oligonucleotide primer in the first denaturation step or pre-incubation step of a thermal cycling protocol. An unmodified oligonucleotide primer has an active phosphodiester bond and can prime the extension by a polymerase.

In the first annealing and extension steps of a thermal cycling, a 5' anchor oligonucleotide primer is annealed to a sequence that is complementary to an anchor sequence at the 3' end of a single stranded cDNA (antisense strand) obtained in the reverse transcription step, leading to extension by a polymerase and synthesis of a cDNA (sense strand) in which an anchor sequence (first anchor sequence) is added to the 5' terminus. As a result, a double stranded cDNA in which an anchor sequence is added to the 5' terminus of a sense strand is produced.

In addition, a reaction mixture comprising the double stranded cDNA is subsequently subjected to a plurality of rounds of thermal cycling protocol to amplify a region sandwiched by a 5' anchor oligonucleotide primer and a modified oligonucleotide primer (i.e., from the 5' terminus anchor sequence to the region where the modified oligonucleotide primer hybridizes).

The number of rounds of thermal cycling can be appropriately determined while considering the amount of template RNA or the like, but is for example 20 rounds or more, and preferably 30 rounds or more, 40 rounds or more, 45 rounds or more, 50 rounds or more, or 55 rounds or more. In a common RT-PCR, even with a low number of copies

of template RNA (e.g., single copy), an amplification reaction reaches saturation after about 40 rounds of thermal cycling. Meanwhile in the method of the present invention (especially when using a modified oligonucleotide primer comprising a thermolabile modifying group), an amplification reaction does not reach saturation even after 45 rounds or more, 50 rounds or more, or 55 rounds or more of thermal cycling, so that further amplification can be possible. Although not wishing to be bound by any theory, the amplification efficiency per a round of thermal cycle can be more suppressed than common RT-PCR in the method of the present invention (especially when using a modified oligonucleotide primer comprising a thermolabile modifying group). Thus, when the number of copies of a template RNA intended to be amplified is low (e.g., 100 copies or less, 10 copies or less, or a single copy), or when the method of the present invention is performed using an RNA (especially total RNA) isolated from a single cell as a template RNA, the number of rounds of thermal cycling is preferably 40 rounds or more, 45 rounds or more, 50 rounds or more, or 55 rounds or more.

The method of the present invention can be expected to perform reverse transcription template switching PCR with high specificity in one step. The specificity of reverse transcription template switching PCR can be substantially determined only by a reverse primer, but the present Invention can amplify a gene of interest with high specificity by employing the aforementioned modified oligonucleotide primer as a reverse primer. Especially when the number of copies of a template RNA is low (e.g., when RNA from a single cell is used as a template), a specific PCR product can be expected to be amplified while minimizing side reactions even when the number of PCR cycles is high. Therefore, a reverse primer that is specific to a constant region of an antigen receptor (e.g., antibody (heavy chain or light chain) or T cell receptor ($\alpha$ chain, $\beta$ chain, $\gamma$ chain, or $\delta$ chain) can be used as the aforementioned modified oligonucleotide primer to perform sequence analysis of an antigen recognition site of the antigen receptor at a single cell level.

The present invention can also provide a kit for performing one-step reverse transcription template switching PCR, comprising:

i) a template switching oligonucleotide; and

ii) a primer set consisting of a 5' anchor oligonucleotide primer comprising at least a part of an anchor sequence comprised in the template switching oligonucleotide, and a modified oligonucleotide primer, and a modified oligonucleotide primer;

wherein the modified oligonucleotide primer has a primer function in reverse transcription that is partially or completely blocked by the modification, and a primer function in PCR using a product of the reverse transcription as a template is acquired as a result of the reverse transcription or by initial thermal denaturation.

In one embodiment, the kit of the present invention further comprises an oligonucleotide primer that initiates reverse transcription.

In one embodiment, the kit of the present invention does not further comprise an oligonucleotide primer that initiates reverse transcription.

The kit of the present invention may also comprise other reagents required for performing one-step reverse transcription template switching PCR (e.g., reverse transcriptase (RNA dependent DNA polymerase), heat resistant DNA polymerase (DNA dependent DNA polymerase), dNTPs mixture, buffer, salt (magnesium ion or the like), or RNAase inhibitor).

The reagents may be contained in a single package after being sealed in their respective separate container or provided as a composition comprising a mixture of some or all of the reagents.

In one embodiment, the kit of the present invention comprises the oligonucleotide of i) and the primer set of ii) as a composition comprising a mixture thereof.

In one embodiment, the composition further comprises an oligonucleotide primer that initiates reverse transcription.

In one embodiment, the composition does not further comprise an oligonucleotide primer that initiates reverse transcription.

The composition may comprise 1, 2, 3, 4, 5, or 6 reagents selected from the group consisting of a reverse transcriptase (RNA dependent DNA polymerase), heat resistant DNA polymerase (DNA dependent DNA polymerase), dNTPs mixture, buffer, salt (magnesium ion or the like), and RNAase inhibitor.

If the kit of the present invention is used, one-step reverse transcription template switching PCR can be readily performed with the method of the present invention by using any template RNA.

The definitions of the terms of each constituent comprised in the kit of the present invention are described above in the method of the present invention.

The present invention also provides a kit for amplifying at least a part of a region of a target RNA, the kit comprising: i) a reagent required for reverse transcription; ii) optionally a reagent required for template switching; iii) a reagent required for a polymerase chain reaction using a modified oligonucleotide primer; and iv) optionally a user manual; characterized in that the reagents of i), the reagent of ii) if present, the reagent of iii) and the modified oligonucleotide primer are all mixed in a reaction system as of the initiation of a reaction, wherein the modified oligonucleotide

primer is designed to have a primer function that is partially or completely blocked under a condition where reverse transcription occurs and designed to have blocking of the primer function cleared under a condition where a polymerase chain reaction occurs.

In another embodiment, the present invention provides a method of analyzing a repertoire of variable regions of T cell receptors (TCR) or B cell receptors (BCR) of a subject, comprising the steps of: (1) providing a nucleic acid sample comprising nucleic acid sequences of a plurality of types of T cell receptors (TCR) or B cell receptors (BCR) amplified from an RNA obtained from the subject; (2) determining the nucleic acid sequences contained in the nucleic acid sample; and (3) calculating a frequency of appearance of each gene or a combination thereof based on the determined nucleic acid sequences to derive a TCR or BCR repertoire of the subject; wherein step (1) comprises the steps of: a) mixing an RNA obtained from the subject, a reagent required for reverse transcription, a reagent required for template switching, and a reagent required for a polymerase chain reaction and subjecting the mixture to a condition under which reverse transcription occurs to provide a cDNA comprising nucleic acid sequences of a plurality of types of T cell receptors (TCR) or B cell receptors (BCR); and b) subjecting the cDNA obtained in step a) to a condition under which a polymerase chain reaction occurs to provide the nucleic acid sample comprising nucleic acid sequences of a plurality of types of T cell receptors (TCR) or B cell receptors (BCR); wherein the reagent required for template switching comprises a template switching oligonucleotide, and wherein the regent required for a polymerase chain reaction comprises a primer specific to a C region of the TCR or the BCR, wherein the primer specific to a C region is a modified oligonucleotide primer designed to have a primer function that is partially or completely blocked in step a) and designed to have blocking of the primer function cleared in step b). The nucleic acid sample is preferably amplified in an unbiased manner.

The method of analyzing a repertoire of the present invention utilizes reverse transcription template switching PCR (steps a) and b)) for a nucleic acid sample for sequence analysis. Use of reverse template switching PCR (RT-TS-PCR) in repertoire analysis is convenient in that time, labor, and cost can be reduced. RT-TS-PCR, due to having a low number of steps, can process samples in a short period of time, can reduce labor, and is advantageous in multi-specimen processing. Since the number of reagents used is low, cost can also be minimized. The method of the present invention is advantageous not only in terms of reduced time, labor, and cost, but also for the following reasons: 1. Reactions from RNA to PCR amplification is completed within a single tube, contamination of a PCR product due to operations such as opening/closing of the tube, addition of a reagent, or purification can be prevented. 2. Since restriction enzyme is not used, accidental cleavage of the main body of the amplicon can be prevented. 3. Analysis with high sensitivity is possible without loss in the purification operation at each step until a sample subjected to sequencing is obtained.

Furthermore, the present invention provides a method of producing a nucleic acid sample for analyzing a repertoire of variable regions of T cell receptors (TCR) or B cell receptors (BCR) of a subject, the method comprising the step of (1) providing a nucleic acid sample comprising nucleic acid sequences of a plurality of types of T cell receptors (TCR) or B cell receptors (BCR) amplified from an RNA obtained from the subject, step (1) comprising the steps of: a) mixing an RNA obtained from the subject, a reagent required for reverse transcription, a reagent required for template switching, and a reagent required for a polymerase chain reaction and subjecting the mixture to a condition under which reverse transcription occurs to provide a cDNA comprising nucleic acid sequences of a plurality of types of T cell receptors (TCR) or B cell receptors (BCR); and b) subjecting the cDNA obtained in step a) to a condition under which a polymerase chain reaction occurs to provide the nucleic acid sample comprising nucleic acid sequences of a plurality of types of T cell receptors (TCR) or B cell receptors (BCR); wherein the reagent required for template switching comprises a template switching oligonucleotide, and wherein the regent required for a polymerase chain reaction comprises a primer specific to a C region of the TCR or the BCR, wherein the primer specific to a C region is a modified oligonucleotide primer designed to have a primer function that is partially or completely blocked in step a) and designed to have blocking of the primer function cleared in step b). The nucleic acid sample is preferably amplified in an unbiased manner.

In one embodiment, the reagents required for template switching can comprise a template switching oligonucleotide. In another embodiment, a reagent required for a polymerase chain reaction can, but does not need to comprise a 5' anchor oligonucleotide primer comprising at least a part of an anchor sequence comprised in a template switching oligonucleotide. As demonstrated in the Examples herein, a template switching oligonucleotide (TS-Oligo) can also unexpectedly function as a forward primer in PCR amplification. Therefore, a reagent required for a polymerase chain reaction can be free of the 5' anchor oligonucleotide primer or comprise a smaller amount that an amount that is commonly used.

Surprisingly, PCR amplification with high specificity was able to be achieved even by adding only the modified oligonucleotide primer without adding a reverse transcription primer to perform reverse transcription PCR. Although not wishing to be bound by any theory, a part of a modified oligonucleotide primer does not have the function blocked at the time of a reverse transcription reaction, so that a part of the modified oligonucleotide primer whose function is not blocked can function as a reserve transcription primer, or a function of a modified oligonucleotide primer is partially blocked at the time of a reverse transcription reaction, so that the modified oligonucleotide primer whose function is partially blocked can function as a reverse transcription primer in a limited capacity. Therefore in some embodiment, a reagent required

for reverse transcription does not need to comprise an oligonucleotide primer that initiates reverse transcription. Even if it is comprised, the oligonucleotide primer that initiates reverse transcription used in the present invention can be contained at a smaller amount than an amount that is commonly used. In some embodiments, the concentration of the oligonucleotide primer that initiates reverse transcription in the composition is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less. In another embodiment, the oligonucleotide primer that initiates reverse transcription in the composition is comprised at a mole ratio of about 1:10 or less relative to a modified oligonucleotide primer, preferably about 1:20 or less, about 1:40 or less, about 1:160 or less, about 1:200 or less, about 635:1 or less, about 2000:1 or less, about 2500:1 or less, about 20,000:1 or less, about 200,000:1 or less, about 2,000,000:1 or less, or about 20,000,000:1 or less.

The repertoire analysis method of the present invention may further comprise a step of providing a nucleic acid sample to which a suitable sequence for sequence analysis is added after performing reverse transcription template switching PCR. Those skilled in the art can select a suitable sequence for sequence analysis depending on the type of sequence analysis. Examples of a suitable sequence for sequence analysis include, but are not limited to, sequences that are suitable for sequence analysis using bride PCR or emulsion PCR. Preferably, the sequence analysis used in the present invention is sequence analysis using bridge PCR. Suitable sequences added for sequence analysis using bridge PCR include index sequences, tag sequences, sequences for immobilization to a substrate (e.g., flow cell) of sequence analysis, and the like.

In a preferred embodiment, the repertoire analysis method of the present invention can perform the reverse transcription template switching PCR (first PCR amplification reaction), tag PCR (second PCR amplification reaction) and index PCR (third PCR amplification reaction) to provide a nucleic acid sample for sequence analysis.

A 5' anchor oligonucleotide primer used in reverse transcription template switching PCR (first PCR amplification reaction) is also called a first 5' anchor oligonucleotide primer. A first 5' anchor oligonucleotide primer is an oligonucleotide primer comprising at least a part of an anchor sequence comprised in a template switching oligonucleotide. In a first PCR amplification reaction, the first 5' anchor oligonucleotide primer does not need to be used because a template switching oligonucleotide also functions as the 5' anchor oligonucleotide primer.

In one embodiment, step (1) may further comprise the following steps: c) subjecting a mixture comprising a PCR amplicon of step b), a second 5' anchor oligonucleotide primer to which a first tag sequence is added, and a second primer specific to a C region of TCR or BCR to which a second tag sequence is added to a condition under which a polymerase chain reaction occurs to provide a nucleic acid sample comprising nucleic acid sequences of a plurality of types of T cell receptors (TCR) or B cell receptors (BCR) to which a tag sequence is added; and d) subjecting a mixture comprising a PCR amplicon of step c), a third 5' anchor oligonucleotide primer, and a third primer specific to a C region of TCR or BCR to a condition under which a polymerization chain reaction occurs to provide the nucleic acid sample comprising nucleic acid sequences of a plurality of types of T cell receptors (TCR) or B cell receptors (BCR) to which an index sequence is added, wherein an index sequence and a sequences for immobilization to a substrate of sequence analysis are added to the third 5' anchor oligonucleotide primer and the third primer specific to a C region of TCR or BCR.

A tag sequence is a sequence used for sequencing (e.g., sequencing by Miseq). Sequencing is initiated from a tag sequence. A tag sequence also may function as a priming site of index PCR (third PCR amplification reaction) for adding an index sequence. A first tag sequence can be a sequence to which a second 5' anchor oligonucleotide primer is added, and a second tag sequence can be a sequence to which a second primer specific to a C region of TCR or BCR is added.

As used herein, "first TCR or BCR C region specific primer" is a primer used in a PCR amplification reaction by reverse transcription template switching PCR of the present invention, comprising a sequence that is specific to a C region of TCR or BCR.

As used herein, "second primer specific to a C region of TCR or BCR" is a primer used in a PCR amplification reaction (second PCR amplification reaction) for providing a nucleic acid sample comprising a nucleic acid sequence of TCR or BCR to which a tag is added, comprising a sequence that is specific to a C region of TCR or BCR. A tag sequence that is a priming site for a third PCR amplification reaction is added to a second primer specific to a C region of TCR or BCR.

As used herein, "third primer specific to a C region of TCR or BCR" is a primer used in a PCR amplification reaction (third PCR amplification reaction) for providing a nucleic acid sample comprising a nucleic acid sequence of TCR or BCR to which an index sequence is added, comprising a sequence that is specific to a C region of TCR or BCR. An index sequence and a sequence for immobilization to a substrate of sequence analysis are optionally added to a third primer specific to a C region of TCR or BCR.

As used herein, "first 5' anchor oligonucleotide primer" is a primer used in a PCR amplification reaction using reverse transcription template switching PCR of the present invention. Since a template switching oligonucleotide can also function as a 5' anchor oligonucleotide primer, a first 5' anchor oligonucleotide primer does not need to be used, or used in a small quantity.

As used herein, "second 5' anchor oligonucleotide primer" is a primer used in a PCR amplification reaction

(second PCR amplification reaction) for providing a nucleic acid sample comprising a nucleic acid sequence of TCR or BCR to which a tag sequence is added.

As used herein, "third 5' anchor oligonucleotide primer" is a primer used in a PCR amplification reaction (third PCR amplification reaction) for providing a nucleic acid sample comprising a nucleic acid sequence of TCR or BCR to which an index sequence is added. An index sequence and a sequence for immobilization to a substrate of sequence analysis are optionally added to a third 5' anchor oligonucleotide primer.

As used herein, "first PCR amplification reaction" is a PCR amplification reaction using RNA obtained from a subject by the one-step reverse transcription template switching PCR of the present invention as a template.

As used herein, "second PCR amplification reaction" is a PCR amplification reaction for providing a nucleic acid sample comprising a nucleic acid sequence of TCR or BCR to which a tag sequence is added, the reaction using a product of a first PCR amplification reaction as a template in producing a sample for the repertoire analysis of the present invention. In a second PCR amplification reaction, a nucleic acid sample to which a tag sequence for providing a priming site for a third PCR amplification reaction is added is provided. Such a tag sequence is also an initiation point in sequence analysis.

As used herein, "third PCR amplification reaction" is a PCR amplification reaction using an amplicon of a second PCR amplification reaction as a template in producing a sample for the analysis of the present invention, a product thereof comprising a sequence that is suitable for use in the sequence analysis of the present invention. An index sequence and a sequence for immobilization to a substrate of sequence analysis are added in a third PCR amplification reaction.

In one embodiment, step (3) may comprise the following steps: (3-1) providing a reference database for each gene region comprising at least one of a V region, a D region, a J region, and optionally a C region; (3-2) providing an input sequence set which is optionally trimmed and optionally extracted to have a suitable length; (3-3) searching for homology of the input sequence set with the reference database for the each gene region and recording an alignment with an approximate reference allele and/or a sequence of the reference allele; (3-4) assigning the V region and the J region for the input sequence set and extracting a nucleic acid sequence of the D region based on a result of assigning; (3-5) translating the nucleic acid sequence of the D region into an amino acid sequence and classifying the D region by utilizing the amino acid sequence; and (3-6) calculating a frequency of appearance for each of the V region, the D region, the J region and optionally the C region or a frequency of appearance of a combination thereof based on the classifying in step (3-5) to derive the TCR or BCR repertoire.

The present invention also provides a kit for amplifying a variable region of a T cell receptor (TCR) or a B cell receptor (BCR), the kit comprising: i) a reagent required for reverse transcription; ii) a reagent required for template switching; iii) a reagent required for a polymerase chain reaction using a modified oligonucleotide primer; and iv) optionally a user manual; characterized in that the reagents of i) to iii) and the modified oligonucleotide primer are all mixed in a reaction system as of the initiation of a reaction, wherein the reagent of ii) comprises a template switching oligonucleotide, and wherein the modified oligonucleotide primer is a primer specific to a C region of the TCR or the BCR which is designed to have a primer function that is partially or completely blocked under a condition where reverse transcription occurs and designed to have blocking of the primer function cleared under a condition where a polymerase chain reaction occurs. The kit of the present invention can provide a nucleic acid sample comprising nucleic acid sequences of a plurality of types of T cell receptors (TCR) or B cell receptors (BCR), which is amplified in an unbiased manner from an RNA obtained from a subject.

As used herein, "kit" refers to a unit providing portions to be provided (e.g., reagent, agent, label, manual and the like) generally in two or more sections. This form of a kit is preferred when a composition that should not be provided in a mixed state and is preferably mixed immediately before use for safety or the like is intended to be provided. Such a kit advantageously comprises an instruction or manual describing how the provided portions (e.g., agents) are used or how a reagent should be handled. When the kit is used herein as a reagent kit, the kit generally comprises an instruction describing how an agent, antibody and the like is used.

As used herein, "instruction" is a document that explains to a user the method of using the present invention. The instruction has an instruction for the reverse transcription template switching PCR and the method of using a reagent of the present invention. The instruction may also have instructions for a method of use (screening method). The instruction is prepared in accordance with a format defined by a regulatory authority of the country in which the present invention is practiced, with an explicit description showing approval by the regulatory authority. The instruction is a so-called package insert, which can be provided in paper media or in a form such as electronic media (e.g., web sites provided on the Internet or emails).

A reagent required for a polymerase chain reaction comprised in the kit of the present invention does not need to comprise a primer. A primer may be included in the kit of the present invention or provided separately. Those skilled in the art can design and manufacture a suitable primer based on the target RNA or outsource the manufacture to a primer manufacturer. The modified oligonucleotide primer is used as a reverse primer used in the kit of the present invention. A template switching oligonucleotide or a 5' anchor oligonucleotide primer comprising at least a part of an

anchor sequence comprised in a template switching oligonucleotide can be used as a forward primer.

In one embodiment, a reagent required for template switching in the kit of the present invention can comprise a template switching oligonucleotide. In another embodiment, a reagent required for a polymerase chain reaction in the kit of the present invention can, but does not need to comprise a 5' anchor oligonucleotide primer comprising at least a part of an anchor sequence comprised in a template switching oligonucleotide. As demonstrated in the Examples herein, a template switching oligonucleotide (TS-Oligo) can also unexpectedly function as a forward primer in PCR amplification. Therefore, a reagent required for a polymerase chain reaction can be free of the 5' anchor oligonucleotide primer or comprise a smaller amount than an amount that is commonly used.

Surprisingly, PCR amplification with high specificity was able to be achieved even by adding only the modified oligonucleotide primer without adding a reverse transcription primer to perform reverse transcription PCR. Although not wishing to be bound by any theory, a part of a modified oligonucleotide primer does not have the function blocked at the time of a reverse transcription reaction, so that a part of the modified oligonucleotide primer whose function is not blocked can function as a reserve transcription primer, or a function of a modified oligonucleotide primer is partially blocked at the time of a reverse transcription reaction, so that the modified oligonucleotide primer whose function is partially blocked can function as a reverse transcription primer in a limited capacity. Therefore in some embodiment, a reagent required for reverse transcription does not need to comprise an oligonucleotide primer that initiates reverse transcription. Even if it is comprised, the oligonucleotide primer that initiates reverse transcription used can be contained at a smaller amount than an amount that is commonly used. In some embodiments, the final concentration of the oligonucleotide primer that initiates reverse transcription to be used is, for example, about 40 nM or less, preferably about 20 nM or less, about 10 nM or less, about 2.5 nM or less, about 2.0 nM or less, about 0.63 nM or less, about 0.2 nM or less, about 0.16 nM or less, about 0.02 nM or less, about 2.0 pM or less, about 0.2 pM or less, or about 0.02 pM or less. In another embodiment, the oligonucleotide primer that initiates reverse transcription to be used is used at a mole ratio of about 1:10 or less relative to a modified oligonucleotide primer, preferably about 1:20 or less, about 1:40 or less, about 1:160 or less, about 1:200 or less, about 635:1 or less, about 2000:1 or less, about 2500:1 or less, about 20,000:1 or less, about 200,000:1 or less, about 2,000,000:1 or less, or about 20,000,000:1 or less.

The modified oligonucleotide primer used in the kit of the present invention has been described above in detail.

The present invention also provides a system for quantitatively analyzing a repertoire of variable regions of T cell receptors (TCR) or B cell receptors (BCR) of a subject by using a database, the system comprising: (1) a kit for providing a nucleic acid sample comprising nucleic acid sequences of a plurality of types of T cell receptors (TCR) or B cell receptors (BCR), which is amplified in an unbiased manner from an RNA obtained from a subject; (2) an apparatus for determining the nucleic acid sequences comprised in the nucleic acid sample; and (3) an apparatus for calculating a frequency of appearance of each gene or a combination thereof based on the determined nucleic acid sequences to derive a TCR or BCR repertoire of the subject.

The kit used in the system of the present invention can further comprise: c) means for subjecting a mixture comprising a PCR amplicon of step b), a second 5' anchor oligonucleotide primer to which a first tag sequence is added, and a second primer specific to a C region of TCR or BCR to which a second tag sequence is added to a condition under which a polymerase chain reaction occurs to provide a nucleic acid sample comprising nucleic acid sequences of a plurality of types of T cell receptors (TCR) or B cell receptors (BCR) to which a tag sequence is added; and d) means for subjecting a mixture comprising a PCR amplicon of step c), a third 5' anchor oligonucleotide primer, and a third primer specific to a C region of TCR or BCR to a condition under which a polymerization chain reaction occurs to provide the nucleic acid sample comprising nucleic acid sequences of a plurality of types of T cell receptors (TCR) or B cell receptors (BCR) to which an index sequence is added, wherein an index sequence and a sequences for immobilization to a substrate of sequence analysis are added to the third 5' anchor oligonucleotide primer and the third primer specific to a C region of TCR or BCR.

In one embodiment, the (3) apparatus for deriving a TCR or BCR repertoire can comprise: (3-1) means for providing a reference database for each gene region comprising at least one of a V region, a D region, a J region, and optionally a C region; (3-2) means for providing an input sequence set which is optionally trimmed and optionally extracted to have a suitable length; (3-3) means for searching for homology of the input sequence set with the reference database for the each gene region and recording an alignment with an approximate reference allele and/or a sequence of the reference allele; (3-4) means for assigning the V region and the J region for the input sequence set and extracting a nucleic acid sequence of the D region based on a result of assigning; (3-5) means for translating the nucleic acid sequence of the D region into an amino acid sequence and classifying the D region by utilizing the amino acid sequence; and (3-6) means for calculating a frequency of appearance for each of the V region, the D region, the J region, and optionally the C region or a frequency of appearance of a combination thereof based on the classifying in step (3-5) to derive the TCR or BCR repertoire.

In another embodiment, the present invention provides a system for analyzing a disease, disorder, or condition of a subject, comprising the system for quantitatively analyzing a repertoire of variable regions of TCRs or BCRs and means for analyzing the disease, disorder, or condition of the subject based on a TCR or BCR repertoire derived based

the system.

In another embodiment, the present invention provides a system for treating or preventing a disease, disorder, or condition of a subject, comprising: means for quantitatively associating the disease, disorder, or condition of the subject determined by the system for analyzing a disease, disorder, or condition of a subject with the TCR or BCR repertoire; and means for selecting means for suitable treatment or prevention from the quantitative association.

As used herein, "database" refers to any database related to genes and especially to a database comprising T cell receptor and B cell receptor repertoires in the present invention. Examples of such a database include, but are not limited to, IMGT (the international ImMunoGeneTics information system, www.imgt.org) database, DNA Data Bank of Japan (DDBJ, DNA Data Bank of Japan, www.ddbj.nig.ac.jp) database, GenBank (National Center for Biotechnology Information, www.ncbi.nlm.nih.gov/genbank/) database, ENA (EMBL (European Molecular Biology Laboratory), www.ebi.ac.uk/ena) database, and the like.

As used herein, "repertoire of variable regions" refers to a collection of V(D)J regions created in any manner by gene rearrangement in a TCR or BCR.

As used herein, "index sequence" is a sequence for imparting uniqueness so that an amplicon can be identified. Thus, an index sequence is preferably different from a sequence of interest. It is also preferable that an index sequence is sequence that does not affect amplification. Baselines for the determination of an index sequence and a representative example thereof are the following. As an explanation of the baseline for determining an index sequence, an index sequence, in other words, is a base sequence that is added for identifying each sample when a plurality of samples are mixed and sequenced simultaneously. Reads from a single sample are matched to a single index sequence, so that it is possible to identify the sample from which the acquired read sequence is derived. An index sequence is any sequence of 4 types of bases, i.e., A, C, G, and T. Theoretically, about 1 million types of sequences can be created with 10 bases, and about one trillion types of sequences can be created with 20 bases. The length of a base sequence is preferably 2 to 40 bases and more preferably 6 to 10 bases. It is also desirable to use a sequence that does not comprise a consecutive sequence (AA, CC, GG, or TT).

As used herein, "isotypes" refer to IgM, IgA, IgG, IgE, IgD or the like that belong to the same type but have a difference sequence from one another. Isotypes can be denoted by using various abbreviations or symbols of genes.

As used herein, "subtype" is a type within a type that is present in IgA and IgG for BCRs. There are IgG1, IgG2, IgG3, and IgG4 for IgG, and IgA1 and IgA2 for IgA. Subtypes are also known to be present in $\beta$ and $\gamma$ chains for TCRs, which are TRBC1 and TRBC2 and TRGC1 and TRGC2, respectively.

As used herein, "homology" of genes refers to the degree of identity of two or more genetic sequences to one another. In general, having "homology" refers to having a high degree of identity or similarity. Thus, a higher degree of homology of two genes results in a higher degree of identity or similarity of the sequences thereof. It is possible to examine whether two types of genes are homologous by direct comparison of sequences or by hybridization under stringent conditions for nucleic acids. When directly comparing two genetic sequences, the genes are homologous typically when DNA sequences between the genetic sequences are at least 50% identical, preferably at least 70% identical, and more preferably at least 80%, 90%, 95%, 96%, 97%. 98% or 99% identical. Thus, as used herein, "homolog" or "homologous gene product" refers to a protein in another species, preferably a mammal, which exerts the same biological function as a protein constituent of a complex further described herein.

As used herein, "subject" refers to a source of a sample for repertoire analysis of the present invention or a target subjected to diagnosis of the present invention. Examples of subjects include mammals (e.g., humans, mice, rats, hamsters, rabbits, cats, dogs, cows, horses, sheep, monkeys, and the like), but primates are preferred and humans are particularly preferred.

As used herein, "sample" refers to any substance obtained from a subject or the like. For example, nucleic acids (e.g., RNA), cells, tissue, organs, and the like are encompassed. Those skilled in the art can appropriately select a preferred sample based on the descriptions herein.

As used herein, "means" refers to anything that can be a tool for accomplishing an objective.

As used herein, "diagnosis" refers to identifying various parameters associated with a disease, disorder, condition or the like in a subject to determine the current or future state of such a disease, disorder, or condition. The condition in the body can be examined by using the method, apparatus, or system of the present invention. Such information can be used to select and determine various parameters of a formulation or method for treatment or prevention to be administered, disease, disorder, or condition in a subject or the like. As used herein, "diagnosis" when narrowly defined, refers to diagnosis of the current state, but when broadly defined includes "early diagnosis", "predictive diagnosis", "prediagnosis" and the like. Since the diagnostic method of the present invention in principle can utilize what comes out from a body and can be conducted away from a medical practitioner such as a physician, the method is industrially useful. In order to clarify that the method can be conducted away from a medical practitioner such as a physician, the term as used herein may be particularly called "assisting" "predictive diagnosis, prediagnosis or diagnosis".

The formulation procedure for a diagnostic agent or the like of the present invention as a medicament or the like is known in the art. The procedure is described, for example, in the Japanese Pharmacopoeia, the United States

Pharmacopeia, pharmacopeia of other countries, or the like. Thus, those skilled in the art can determine the amount to be used without undue experimentation with the descriptions herein.

As used herein, "trimming" refers to removal of an unsuitable portion in gene analysis. Trimming is performed by removing low quality regions from both ends of a read, partial sequence of an artificial nucleic acid sequence imparted in an experimental procedure, or both. Trimming can be performed with a software known in the art or by referring to references (for example, cutadapt http://journal.embnet.org/index.php/embnetjournal/article/v iew/200/ (EMBnet.journal, 2011); fastq-mcf Aronesty E., The Open BioinformaticsJournal (2013) 7, 1-8 (DOI: 10.2174/1875036201307010001); and fastx-toolkit http://hannonlab.cshl.edu/fastx_toolkit/ (2009)).

As used herein, "suitable length" refers to a length that is compatible with analysis when analyzing an alignment or the like in the gene analysis of the present invention. For example, such a length can be determined to be a length including 100 bases toward a D region on a V region from a sequencing initiation position on a C region. In the present invention, examples of a suitable length include, but are not limited to, 200 nucleotides or longer and preferably 250 nucleotides or longer for TCRs and 300 nucleotides or longer and preferably 350 nucleotides or longer for BCRs.

As used herein, "input sequence set" refers to a set of target sequences of TCR or BCR repertoire analysis in the gene analysis of the present invention.

As used herein, "gene region" refers to each of V region, D region, J region, C region and the like. Such gene regions are known in the art and can be appropriately determined by referring to a database or the like. As used herein, "homology" of genes refers to the degree of identity of 2 or more genetic sequences to one another. In general, having "homology" refers to having a high degree of identity or similarity. Thus, a higher degree of homology of two genes results in a higher degree of identity or similarity of the sequences thereof. It is possible to examine whether two types of genes are homologous by direct comparison of sequences or by hybridization under stringent conditions for nucleic acids. As used herein, "homology search" refers to a search for homology. Preferably, homology can be searched in silico by using a computer.

As used herein, "approximate" refers to having a high degree of homology when homology search is performed. A software for homology search (BLAST, FASTA or the like), when executed, generally lists results in order of high degree of homology. Thus, approximation can be performed by appropriately selecting a result that is highly ranked.

As used herein, "closest" refers to the highest degree of homology when homology search is performed. When homology is searched with software, the result displayed as ranking number one is selected.

As used herein, "reference allele" refers to a reference allele that results in a match in a reference database when homology search is performed.

As used herein, "alignment" (or align) in bioinformatics refers to similar regions of a primary structure of a biomolecule such as DNA, RNA, or protein arranged in alignment to be identifiable or the act of such arranging. Alignment can provide a clue for understanding the functional, structural, or evolutionary relationship of sequences.

As used herein, "assign" refers to allocating specific information such as a gene name, function, or characteristic region (e.g., V region, J region or the like) to a certain sequence (e.g., nucleic acid sequence, protein sequence or the like). Specifically, this can be accomplished by inputting or linking specific information to a certain sequence or the like.

As used herein, "CDR3" refers to the third complementarity-determining region (CDR). In this regard, CDR is a region that directly contacts an antigen and undergoes a particularly large change among variable regions, and refers to such a hypervariable region. Each variable region of a light chain and a heavy chain has three CDRs (CDR1 to CDR3) and 4 FRs (FR1 to FR4) surrounding the three CDRs. Since a CDR3 region is considered to be present across the V region, D region and J region, it is considered as an important key for a variable region, and is thus used as a subject of analysis.

As used herein, "front of CDR3 on a reference V region" refers to a sequence corresponding to the front of CDR3 in a V region targeted by the present invention.

As used herein, "end of CDR3 on a reference J" refers to a sequence corresponding to the end of CDR3 in a J region targeted by the present invention.

As used herein, "condition tolerating random mutations to be scattered throughout" refers to any condition which results in random mutations being scattered around. For example, such a condition is often expressed by the following condition for BLAST/FASTA optimal parameters: tolerates a maximum mismatch of 33% across the full length of an alignment; and tolerates a maximum nonconsecutive mismatch of 60% for any 30 bp therein. A functional equivalent such as an isotype of a molecule, e.g. IgG, used in the present invention can be found by searching a database or the like. As used herein, "search" refers to utilizing a certain nucleic acid base sequence electronically, biologically, or by another method, preferably electronically, to find another nucleic acid base sequence having a specific function and/or property. Examples of electronic search include, but are not limited to, BLAST (Altschul et al., J.Mol.Biol.215: 403-410 (1990)). FASTA (Pearson & Lipman, Proc.Natl.Acad.Sci., USA 85: 2444-2448 (1988)), Smith and Waterman method (Smith and Waterman, J.Mol.Biol.147: 195-197 (1981)), Needleman and Wunsch method (Needleman and Wunsch, J.Mol.Biol.48: 443-453 (1970)) and the like. BLAST is typically used. Examples of biological search include, but are not limited to, stringent hybridization, a macroarray with a genomic DNA applied to a nylon membrane or the like or a

microarray with a genomic DNA applied to a glass plate (microarray assay), PCR, in situ hybridization, and the like. Herein, a gene used in the present invention is intended to include corresponding genes identified by such electronic search or biological search.

In one embodiment where quantitative analysis is performed on a repertoire of variable regions of a BCR, the primer specific to a C region comprises a sequence that is a complete match with an isotype C region of interest selected from the group consisting of IgM, IgA, IgG, IgE and IgD and has a sequence that is not homologous with other C regions. Preferably, the primer specific to a C region is a sequence that is a complete match with one of the subtypes IgG1, IgG2, IgG3 and IgG4 or one of IgA1 and IgA2 for IgA or IgG. In another embodiment where quantitative analysis is performed on a repertoire of variable regions of a TCR, the primer specific to a C region is a sequence that is a complete match with a C region of a chain of interest selected from the group consisting of $\alpha$ chain, $\beta$ chain, $\gamma$ chain and $\delta$ chain and is not homologous with other C regions.

In another embodiment, it is preferable that a portion of a sequence that is a complete match with all C region allelic sequences of the same isotype in the database is selected for the primer specific to a C region. Such selection of a complete match enables highly precise analysis.

(Large-scale analysis)

In another aspect, the present invention provides a method of performing gene analysis using a sample manufactured by the method of the present invention.

Gene analysis can be performed using any analytic approach. For example, it is possible to use an approach of assigning V, D, J, and C sequences of each read sequence by using V, D, J, and C sequences obtained from a known IMGT (the international ImMunoGeneTics information system, http://www.imgt.org) database as a reference sequence and utilizing HighV-Quest of the IMGT, or a new software (Repertoire Genesis) developed by the Applicant, which is described herein as a preferred example of an analysis system.

In a preferred embodiment, the gene analysis is the quantitative analysis of a repertoire of variable regions of T cell receptors (TCR) or B cell receptors (BCR).

Different sequences can be distinguished by sequencing individual amplification molecules. Thus, sequencing has sensitivity to detect a quantitative change in clone proliferation. In summary, one provided embodiment of the present invention provides a method of determining a profile of a recombinant DNA sequence in a T cell and/or B cell. The present method can comprise the steps of: isolating a sample from a subject; performing one or more rounds of nucleic acid amplification and spatially isolation of individual nucleic acids; and sequencing the nucleic acids.

One aspect provides a method of determining a correlation of one or more repertoires in a subject or an individual. Another aspect provides a method of developing an algorithm capable of predicting a correlation of one or more repertoires in any sample derived from a subject having a disease. Another aspect provides a method of using an algorithm capable of predicting a correlation of one or more repertoires in any sample derived from a subject to find a correlation of one repertoire of an individual or correlation of a plurality of repertoires. Another aspect provides a method of creating an algorithm that calculates a disease activity score. Another aspect provides a method of monitoring a condition of a disease of an individual.

(Analysis system)

The present invention provides bioinformatics for performing quantitative analysis of a repertoire of variable regions of T cell receptors (TCR) or B cell receptors (BCR) by using a next generation sequencing technology.

In one aspect, the present invention is a method of analyzing a TCR or BCR repertoire, comprising the following steps: (1) providing a reference database for each gene region comprising at least one of a V region, a D region, a J region, and optionally a C region; (2) providing an input sequence set which is optionally trimmed and optionally extracted to have a suitable length; (3) searching for homology of the input sequence set with the reference database for the each gene region and recording an alignment with an approximate reference allele and/or a sequence of the reference allele; (4) assigning the V region and the J region for the input sequence set and extracting a nucleic acid sequence of the D region based on a result of assigning (preferably, assigning the V region and the J region for the input sequence set and extracting a CDR3 sequence, with the front of CDR3 on a reference V region and end of CDR3 on reference J as guides); (5) translating the nucleic acid sequence of the D region into an amino acid sequence and classifying the D region by utilizing the amino acid sequence (preferably translating the nucleic acid sequence of the CDR3 into an amino acid sequence and classifying the D region by utilizing the amino acid sequence); and (6) calculating a frequency of appearance for each of the V region, the D region, and the J region and optionally the C region or a frequency of appearance of a combination thereof based on the classifying in (5) to derive the TCR or BCR repertoire.

In the method of the present invention, (1) providing a reference database for each gene region comprising at least one of a V region, a D region, a J region, and optionally a C region can be accomplished, for example for the V

region, by appropriately selecting and providing a database comprising information on the V region.

In the method of the present invention, (2) providing an input sequence set which is optionally trimmed and optionally extracted to have a suitable length is accomplished by optional trimming using a function of an appropriate software or the like and optional extraction after appropriate selection of a length to provide an an input sequence set. An input sequence can be, for example, a set of amplicons amplified by a known method or a set of amplicons amplified by PCR with the reverse transcription template switching PCR of the present invention.

In the method of the present invention, (3) searching for homology of the input sequence set with the reference database for the each gene region and recording an alignment with an approximate reference allele and/or a sequence of the reference allele is performed by appropriately using a software for performing homology search to perform homology search with a reference database on the input sequence set for each gene region (for example, the V region and the like), and to record alignment with an approximate reference allele and/or a sequence of the reference allele obtained as a result.

In the method of the present invention, (4) assigning the V region and the J region for the input sequence set and extracting a nucleic acid sequence of the D region based on a result of assigning can be accomplished by determining a V region and/or J region based on known information or the like from a sequence alignment. Such extraction can be preferably accomplished by assigning the V region and the J region for the input sequence set and extracting a CDR3 sequence, with the front of CDR3 on a reference V region and end of CDR3 on reference J as guides.

In the method of the present invention, (5) translating the nucleic acid sequence of the D region into an amino acid sequence and classifying the D region by utilizing the amino acid sequence can be accomplished by translating into an amino acid using a known method in the art and picking out a sequence corresponding to the D region by homology search or the like on the amino acid sequence. Preferably, the nucleic acid sequence of the CDR3 can be translated into an amino acid sequence and the D region can be classified by utilizing the amino acid sequence.

In the method of the present invention, (6) calculating a frequency of appearance for each of the V region, the D region, and the J region, and optionally the C region or a frequency of appearance of a combination thereof based on the classifying in (5) to derive the TCR or BCR repertoire can be accomplished by calculating a frequency of appearance of the V region, D region, J region and/or the C region calculated in the above steps, for example, by organizing the frequencies into a list. A TCR or BCR repertoire can be derived thereby.

The following steps are further explained while referring to Figure **12.**

In **S1** (step (1)), a reference database is provided. This may be stored in an external storage apparatus **1405,** but can generally be obtained as a publically disclosed database through a communication device **1411.** Alternatively, an input apparatus **1409** may be used to input and optionally record a database in a RAM **1403** or the external storage apparatus **1405.** In this regard, a database comprising a region of interest such as a V region is provided.

In **S2** (step (2)), an input sequence set is provided. For example, a set of sequence information obtained from a set of amplicons amplified in a PCR amplification reaction is inputted by using the input apparatus **1409** or through the communication device **1411.** In this regard, an apparatus that receives an amplicon of a PCR amplification reaction and performs genetic sequence analysis thereon may be connected. Such a connection is made through a system bus **1420** or through the communication device **1411.** Trimming and/or extraction of a suitable length can be optionally performed at this stage. Such processing is performed with a CPU **1401.** A program for trimming and/or extraction can be each provided via the external storage apparatus, communication device, or input apparatus.

In **S3** (step (3)), alignment is performed. At this stage, homology search is performed on the input sequence set with the reference database for each of the gene regions. For the homology search, the reference database obtained via the communication device **1411** or the like is processed with a homology search program. The CPU **1401** performs the processing. Further, results obtained as a result thereof are analyzed for alignment with an approximate reference allele and/or a sequence the reference allele. This is also processed by the CPU **1401.** A program for the execution thereof can be provided via each of the external storage apparatus, communication device, or input apparatus.

In **S4** (step (4)), nucleic acid sequence information on D is detected. This is also processed by the CPU **1401.** A program for the execution thereof can be provided via each of the external storage apparatus, communication device, or input apparatus. This assigns a V region and a J region for the input sequence set. Assignment is also processed by the CPU **1401.** The CPU **1401** also extracts a nucleic acid sequence of the D region based on a result of assigning. A program for the assigning and extracting process can also be provided via each of the external storage apparatus, communication device, or input apparatus. Preferably, this can be accomplished by determining a V region and/or J region based on known information or the like from sequence alignment. Results can be stored in the RAM **1403** or external storage apparatus **1405.** Preferably, such extraction can be accomplished by assigning the V region and the J region for the input sequence set and extracting a CDR3 sequence, with the front of CDR3 on a reference V region and end of CDR3 on reference J as guides. Such processing can also be performed by the CPU **1401.** A program therefor can also be provided via each of the external storage apparatus, communication device, or input apparatus.

In **S5** (step (5)), a D region is classified. A nucleic acid sequence of the D region is translated into an amino acid sequence and the D region is classified by utilizing the amino acid sequence. This is also processed by the CPU

**1401.** A program for this processing can also be provided via each of the external storage apparatus, communication device, or input apparatus. A sequence corresponding to the D region may be picked out by homology search or the like on the resulting amino acid sequence. This is also processed by the CPU **1401.** A program for this processing can also be provided via each of the external storage apparatus, communication device, or input apparatus. Preferably, a nucleic acid sequence of the CDR3 can be translated into an amino acid sequence to classify the D region by utilizing the amino acid sequence. This is also processed by the CPU **1401.** A program for this processing can also be provided via each of the external storage apparatus, communication device, or input apparatus.

In S6 (step (6)), a frequency of appearance for each of the V region, the D region, the J region, and optionally the C region or a frequency of appearance of a combination thereof is calculated based on the classifying to derive a TCR or BCR repertoire. The calculating and deriving are also processed by the CPU **1401.** A program for this processing can also be provided via each of the external storage apparatus, communication device, or input apparatus.

In one preferred embodiment, the gene region used in the present invention comprises all of the V region, the D region, the J region, and optionally the C region.

In one embodiment, the reference database is a database with a unique ID assigned to each sequence. A sequence of a gene can be analyzed based on a simple indicator, i.e., ID, by uniquely assigning an ID.

In one embodiment, the input sequence set is an unbiased sequence set. An unbiased sequence set can be implemented by PCR amplification with reverse transcription template switching PCR as described herein.

In another embodiment, the sequence set is trimmed. An unnecessary or unsuitable nucleic acid sequence can be removed by trimming, such that efficiently of analysis can be enhanced.

In a preferred embodiment, trimming is accomplished by the steps of: deleting low quality regions from both ends of a read; deleting a region matching 10 bp or more with a sequence of a template switching oligonucleotide from both ends of the read; and using the read as a high quality read in analysis if the remaining length is 200 bp or more (TCR) or 300 bp or more (BCR). Preferably, the low quality refers to a 7 bp moving average of QV value of less than 30.

In a preferred embodiment, the approximate sequence is the closest sequence. In a specific embodiment, the approximate sequence is determined by a ranking of 1. number of matching bases, 2. kernel length, 3. score, and 4. alignment length.

In another embodiment, the homology search is conducted under a condition tolerating random mutations to be scattered throughout. Such a condition is often expressed by for example the following condition for BLAST/FASTA optimal parameters: tolerates a maximum mismatch of 33% across the full length of an alignment; and tolerates a maximum nonconsecutive mismatch of 60% for any 30 bp therein. In one embodiment, the homology search comprises at least one condition from (1) shortening of a window size, (2) reduction in a mismatch penalty, (3) reduction in a gap penalty, and (4) a top priority ranking of an indicator is a number of matching bases, compared to a default condition.

In another embodiment, the homology search is carried out under the following conditions in BLAST or FASTA:

V mismatch penalty = -1, shortest alignment length = 30, and shortest kernel length = 15;
D word length = 7 (for BLAST) or K-tup = 3 (for FASTA), mismatch penalty = -1, gap penalty = 0, shortest alignment length = 11, and shortest kernel length = 8;
J mismatch penalty = -1, shortest hit length = 18, and shortest kernel length = 10; and
C shortest hit length = 30 and shortest kernel length = 15.

This condition can be used, for example, as long as it is a case where a shorter (up to 200 bp) sequence is used to classify only some of the region (a case that does not fall under the "preferred example"). This condition can also be used in a case where an Illumina sequencer is used. In such a case, the possibility of using bwa or bowtie for homology search is considered.

In a specific embodiment, the D region is classified by a frequency of appearance of the amino acid sequence.

In a further embodiment, a combination of a result of search for homology with the nucleic acid sequence of CDR3 and a result of amino acid sequence translation is used as a classification result when there is a reference database for the D region in the step (5).

In another embodiment, only the frequency of appearance of the amino acid sequence is used for classification when there is no reference database for the D region in the step (5).

In a specific embodiment, the frequency of appearance is counted in a unit of a gene name and/or a unit of an allele.

In another embodiment, step (4) comprises the step of assigning the V region and the J region for the input sequence set and extracting a CDR3 sequence, with the front of CDR3 on a reference V region and end of CDR3 on reference J as guides.

In a further embodiment, step (5) comprises translating the nucleic acid sequence of the CDR3 into an amino acid sequence and classifying a D region by using the amino acid sequence.

In one aspect, the present invention provides a system for analyzing a TCR or BCR repertoire, wherein the

system comprises: (1) means for providing a reference database for each gene region comprising at least one of a V region, a D region, a J region, and optionally a C region; (2) means for providing an input sequence set which is optionally trimmed and optionally extracted to have a suitable length; (3) means for searching for homology of the input sequence set with the reference database for the each gene region and recording an alignment with an approximate reference allele and/or a sequence of the reference allele; (4) means for assigning the V region and the J region for the input sequence set and extracting a nucleic acid sequence of the D region based on a result of assigning; (5) means for translating the nucleic acid sequence of the D region into an amino acid sequence and classifying the D region by utilizing the amino acid sequence; and (6) means for calculating a frequency of appearance for each of the V region, the D region, the J region, and optionally the C region or a frequency of appearance of a combination thereof based on the classifying in (5) to derive the TCR or BCR repertoire.

In another aspect, the present invention provides a computer program for having a computer execute processing of a method of analyzing a TCR or BCR repertoire, the method comprising the following steps: (1) providing a reference database for each gene region comprising at least one of a V region, a D region, a J region, and optionally a C region; (2) providing an input sequence set which is optionally trimmed and optionally extracted to have a suitable length; (3) searching for homology of the input sequence set with the reference database for the each gene region and recording an alignment with an approximate reference allele and/or a sequence of the reference allele; (4) assigning the V region and the J region for the input sequence set and extracting a nucleic acid sequence of the D region based on a result of assigning; (5) translating the nucleic acid sequence of the D region into an amino acid sequence and classifying the D region by utilizing the amino acid sequence; and (6) calculating a frequency of appearance for each of the V region, the D region, the J region, and optionally the C region or a frequency of appearance of a combination thereof based on the classifying in (5) to derive the TCR or BCR repertoire.

In still another aspect, the present invention provides a recording medium for storing a computer program for having a computer execute processing of a method of analyzing a TCR or BCR repertoire, the method comprising the following steps: (1) providing a reference database for each gene region comprising at least one of a V region, a D region, a J region, and optionally a C region; (2) providing an input sequence set which is optionally trimmed and optionally extracted to have a suitable length; (3) searching for homology of the input sequence set with the reference database for the each gene region and recording an alignment with an approximate reference allele and/or a sequence of the reference allele; (4) assigning the V region and the J region for the input sequence set and extracting a nucleic acid sequence of the D region based on a result of assigning; (5) translating the nucleic acid sequence of the D region into an amino acid sequence and classifying the D region by utilizing the amino acid sequence; and (6) calculating a frequency of appearance for each of the V region, the D region, the J region, and optionally the C region or a frequency of appearance of a combination thereof based on the classifying in (5) to derive the TCR or BCR repertoire.

(System configuration)

The configuration of a system **1** of the present invention is explained while referring to the functional block diagram in Figure **12.** The Figure shows a case that is materialized with a single system.

The gene analysis system **1** of the present invention is configured by connecting a RAM **1403,** external storage apparatus **1405** such as ROM, HDD, magnetic disk, or flash memory such as USB memory and an input output interface (I/F) **1425** via a system bus **1420** to a CPU **1401** installed in a computer system. An input apparatus **1409** such as a keyboard or a mouse, an output apparatus **1407** such as a display, and a communication device **1411** such as a modem are each connected to the input output I/F **1425.** The external storage apparatus **1405** comprises an information database storage section **1430** and a program storage section **1440,** which are both constant storage regions reserved in the external storage apparatus **1405.**

Such a hardware configuration is designed to achieve a function of the present invention in cooperation with an OS (operating system) by the CPU **1401** calling out, deploying, and executing a software program installed on the storage apparatus **1405** on the RAM **1403** from having various instructions (commands) being input via the input apparatus **1409** or from receiving a command via the communication I/F, communication device **1411,** or the like.

A reference database, input sequence set, created classification data, data of a TCR or BCR repertoire or the like, or information obtained via the communication device **1411** or the like is constantly written and updated into the database storage section **1430.** Information such as information ID of each gene in a reference database and each sequence in each input sequence set is managed with each master table to allow information from a sample that is subjected to accumulation to be managed by IDs defined in each master table.

As input sequence set entry information, a sample provider ID, sample information, result of nucleic acid analysis, known individual/physiological information and result of TCR or BCR repertoire analysis are associated with a sample ID and stored in the database storage section **1430.** In this regard, the result of TCR or BCR repertoire analysis is the information obtained via the processing of the nucleic acid analysis result by the processing of the present invention.

Further, a computer program stored in the program storage section **1440** configures a computer as the processing system, e.g., a system for implementing processing such as trimming, extraction, alignment, assignment, classification, or translation. Each of the features is an independent computer program, module or routine thereof or the like, which is executed by the CPU **1401** to configure a computer as each system or apparatus. Hereinafter, each system is constituted by cooperation of each function in each system.

(Repertoire analysis system/analysis method)

In one aspect, the present invention provides a method of quantitatively analyzing a repertoire of variable regions of T cell receptors (TCR) or B cell receptors (BCR) of a subject by using a database. The method comprises: (1) providing a nucleic acid sample comprising a nucleic acid sequence of the T cell receptor (TCR) or the B cell receptor (BCR) which is amplified from the subject in an unbiased manner; (2) determining the nucleic acid sequence comprised in the nucleic acid sample; and (3) calculating a frequency of appearance of each gene or a combination thereof based on the determined nucleic acid sequence to derive a TCR or BCR repertoire of the subject. This method and methods comprising one or more additional features explained herein are also called "repertoire analysis method of the present invention" herein. A system materializing the repertoire analysis method of the present invention is also referred to as the "repertoire analysis system of the present invention".

The step of (1) providing a nucleic acid sample comprising a nucleic acid sequence of the T cell receptor (TCR) or the B cell receptor (BCR) which is amplified from the subject in an unbiased manner in the method of the present invention may provide any sample, as long as the sample is suitable for determining a nucleic acid sequence. As such an approach, it is possible to use the aforementioned preferred amplification methods of the present invention as well as Reverse transcriptase-PCR, real-time PCR, digital PCR, emulsion PCR, amplified fragment length polymorphism (AFLP) PCR, allele specific PCR, assembly PCR, asymmetrical PCR, colony PCR, helicase-dependent amplification, hot start PCR, inverse PCR, in situ PCR, nested PCR, Touchdown PCR, loop-mediated isothermal PCR (LAMP), Nucleic acid sequence based amplification (NASBA), Ligase Chain Reaction, Branch DNA Amplification, Rolling Circle Amplification, Circle to circle Amplification, SPIA amplification, Target Amplification by Capture and Ligation (TACL), 5'-Rapid amplification of cDNA end (5'-RACE), 3'-Rapid amplification of cDNA end (3'-RACE), Switching Mechanism at 5'-end of the RNA Transcript (SMART).

The step of (2) determining the nucleic acid sequence comprised in the nucleic acid sample in the method of the present invention may use any method, as long as a nucleic acid sequence can be determined. Generally, a large quantity of sequencing is required. Thus, it is preferable to use an automated large-scale sequencing method. Examples of such a sequencing method include sequencing using a Roche 454 sequencer (GS FLX+, GS Junior), sequencing using the approach of an Ion Torrent sequencer (Ion PGM™ Sequencer), and sequencing using the approach of Illumina (GenomeAnalyzer IIx, Hiseq, Miseq). Other sequencing methods include Heliscope™ Sequencer, Helicos True Single Molecule Sequencing (tSMA) (Harris. T.D. et. al Science 2008, 320-160-109), SoliD™ Sequencing (Life Technologies, Inc.), Single Molecule Real Time (SMRT™) PacBio system (Pacific Biosciences, CA), Nanopore Sequencing (Oxford Nanopore Technologies, UK), LaserGen™ (LaserGen, Inc. CA) (reference: Litosh VA et al., Nucleic Acids Res. 2011 Mar; 39(6): e39), Lightspeed Genomics™ (Lightspeed Genomics, CA), GnuBIO (GnuBIO Inc., MA), Polonator sequencing (M.Danaher/Dover, Azco Biotec. Inc., CA), Mebious Biosystem's single molecule sequencing (Mebious Biosystems Limited), Millikan sequencing (Caerus Molecular Diagnostics, Inc), Intelligent Bio-Systems, Inc. (reference: Hutter D, et al Nucleosides Nucleotides Nucleic Acid 2010; 29(11): 879-95.), Hybridization-Assisted Nanopore Sequencing (Nabsys Inc., RI), Nanopore sequencing (Noblegen Biosciences, Inc.), Nanopore sequencing (Electronic Biosciences, CA), Thermosequencing (GENIUS™ technology) (Genapsys, Inc., CA), CAERUS MOLECULAR DIAGNOTICS, INC, CA, Individual Molecule Placement Rapid Nanotransfer (IMPRNT) (Halcyon Molecular, Inc), Monochromatic aberration-corrected dual-beam low energy electron microscopy (Electron Optica, Inc., CA), ZS Genesis DNA Sequencing (ZS Genetyics, Inc), and the like. A Roche 454 sequencer creates a single stranded DNA bound to two types of adaptors, which specifically bind to the 3' terminus and the 5' terminus. The single stranded DNA is bound to a bead via an adaptor and wrapped in a water-in-oil emulsion to form a microreactor having a bead and a DNA fragment. A gene of interest is then amplified by emulsion PCR in the water-in-oil emulsion. The bead is applied to a picotiter plate and sequenced. ATP is generated by sulfrylase, with pyrophosphoric acid generated when dNTP is incorporated into a DNA by DNA polymerase as a substrate (Pyrosequencing). With the ATP and Luciferin as the substrate, luciferase emits fluorescence, which is detected with a CCD camera to determine a base sequence. For the approach of Ion Torrent, emulsion PCR is performed by the same method as Roche, and then a bead is transferred to a microchip, where a sequencing reaction is performed. For detection, the concentration of hydrogen ions released when a DNA is extended by polymerase is detected on a semiconductor chip and converted to a base sequence. The sequencing of Illumia is a method of sequencing while amplifying and synthesizing a DNA of interest on a flow cell by the approach of bridge PCR and sequencing-by-synthesis. Bridge PCR creates a single stranded DNA, to which different adaptor sequences are added to both ends. An adaptor sequence is immobilized on the 5' terminus side in advance on a flow cell, where it is immobilized to the flow cell by an extension reaction. Similarly, an adaptor is immobilized on the 3' terminus side at an adjacent position and binds to the

3' terminus of a synthesized DNA to synthesize a double stranded DNA while forming a so-called bridge. Bridge binding → extension → denaturation are then repeated, such that numerous single stranded DNA fragments are locally amplified to form an accumulated cluster. With such a single stranded DNA as a template, sequencing is performed. For sequencing-by-synthesis, after a sequencing primer is added, a single base synthesis reaction is performed with 3' terminus block fluorescent dNTP using DNA polymerase. A fluorescent substance bound to a base is excited by a laser beam, and light emission is recorded as a photograph with a fluorescence microscope. The base sequence is then determined by proceeding with a step of separating the fluorescent substance and the block to perform the next extension reaction and detecting fluorescence. Preferably, it is advantageous to sequence a plurality of sequence by a single sequencing. It is also advantageous in that a longer sequence length can be sequenced at once.

For the step of (3) calculating a frequency of appearance of each gene or a combination thereof based on the determined nucleic acid sequence to derive a TCR or BCR repertoire of the subject in the present invention, any technique can be used as long as a frequency of appearance of genes and a combination thereof can be calculated and a TCR repertoire and/or BCR repertoire can be derived. For example, the analysis tool HighV-Quest provided by IMGT can be used, in addition to the aforementioned preferred examples of analysis methods. It is also possible to use other approaches by using a software implemented with an alignment feature or a mapping feature, i.e., AbMapper, ALLPATHS, Arachne, BACCardl, Bfast, BLAT, Bowtie, BWA-MEM, BWA-SW, BWA, CCRa VAT & QuTie, CLC workstation, CNV-seq, Elvira, ERNE-map (rNA), GSMapper, Glimmer, gnumap, Goseq, ICAtools, LOCAS, MapSplice, Maq, MEME, Mosaik, NGSView, Novoalign, OSLay, Partek, Perm, Projector, Qpalma, RazerS, SHARCGS, SHRiMP2, SNP-o-matic, Splicemap, SSAHA2, Stampy, Tablet, TMAP, Tophat, or Velve.

In one embodiment, the nucleic acid sample comprises nucleic acid sequences of a plurality of types of T cell receptors (TCR) or B cell receptors (BCR) and step (2) for determining a sequence determines the nucleic acid sequences by a single sequencing. The method of the present invention can reduce or eliminate bias that can occur by determining a plurality of types of sequences by a single sequencing. Thus, the present invention is useful especially in accurately detecting a TCR or BCR read that occurs at a low frequency.

In another embodiment, the single sequencing is characterized in that at least one of the sequences used as a primer in amplification from the nucleic acid sample into a sample for sequencing has the same sequence as a nucleic acid sequence encoding a C region or a complementary strand thereof. Any TCR or BCR can be amplified in the same manner to achieve unbiasedness by using a primer having the same sequence as a nucleic acid sequence encoding a C region or a complementary strand thereof.

In one embodiment, the unbiased amplification includes being non-V region specific amplification. Bias can be further reduced or eliminated compared to a case of performing unbiased amplification by devising a multiplex or the like using a V specific primer.

In one embodiment, the repertoire targeted by the present invention is a repertoire of variable regions of a BCR, and the nucleic acid sequence is a BCR nucleic acid sequence. BCRs are considered to be prone to having a mutation, especially in a V region. Thus, accurate analysis of a BCR repertoire is difficult with an approach using V region specific amplification.

In one aspect, the present invention provides a method of analyzing a disease, disorder, or condition of the subject based on a TCR or BCR repertoire derived based on the repertoire analysis method of the present invention.

In the method of analyzing a disease, disorder, or condition of the present invention, the approach of analyzing a disease, disorder, or condition of the subject based on a TCR or BCR repertoire derived based on the repertoire analysis method of the present invention starts from linking derived read data consisting of read types, number of reads, read frequency, V region, J region, C region, CDR3 sequence or the like with clinical information such as disease, disorder, or condition to form a database by using a spreadsheet such as EXCEL. First, for a derived individual read sequence: 1. a TCR having a known function such as NKT or MAIT is search; 2. existing public database is searched for collation with a TCR or BCR with a known function such as antigen specificity; and 3. the constructed database or an existing public database is searched to associate a common sample origin, property or function with a disease, disorder, or condition. Next, for a read sequence in a sample: 1. it is clarified whether the frequency of a specific read increases (clonality increases); 2. examination is carried out to find out whether a specific V chain or J chain usage frequency increases or decreases depending on the condition of a disorder or onset of a disease; 3. examination is carried out to find out whether the length of a CDR3 sequence in a specific V chain increases or decreases depending on the condition of a disorder or onset of a disease; 4. the composition or sequence of a CDR3 region that changes depending on the condition of a disorder or onset of a disease is examined. 5. a read that appears or disappears depending on the condition of a disorder or onset of a disease is searched; 6. a read that increases or decreases depending on the condition of a disorder or onset of a disease is searched; 7. a read that appears or increases/decreases depending on the condition of a disorder or onset of a disease is searched in another sample and associated with a disease, disorder, or condition; 8. a diversity index or similarity index is calculated with statistical analysis software such as ESTIMATES or R (vegan) by using data such as number of samples, read type, or the number of reads; and 9. a change in the diversity index or similarity index can be associated with the condition of a disorder or onset of a disease.

In one embodiment, the disease, disorder, or condition of the subject in the analysis method of the present invention includes, but is not limited to, hematological tumor, colon cancer, immunological condition, rheumatoid arthritis, adult T-cell leukemia, T-cell large granular lymphocyte leukemia, idiopathic thrombocytopenic purpura, and the like.

In another embodiment, the present invention provides a method of treating or preventing the disease, disorder, or condition of the subject determined by the method of the present invention, comprising the steps of: quantitatively associating the disease, disorder, or condition of the subject with the TCR or BCR repertoire; and selecting means for suitable treatment or prevention from the quantitative association.

In one embodiment, diseases, disorders or conditions of a subject targeted in the method of treating or preventing in the present invention include, but are not limited to, hematological tumor, colon cancer, immunological condition, rheumatoid arthritis, adult T-cell leukemia, T-cell large granular lymphocyte leukemia, idiopathic thrombocytopenic purpura, and the like.

In another aspect, the present invention provides a system (analysis system) for quantitatively analyzing a repertoire of variable regions of T cell receptors (TCR) or B cell receptors (BCR) of a subject by using a database. The system comprises (1) a kit for providing a nucleic acid sample comprising a nucleic acid sequence of the T cell receptor (TCR) or the B cell receptor (BCR) which is amplified from the subject in an unbiased manner; (2) an apparatus for determining the nucleic acid sequence comprised in the nucleic acid sample; and (3) an apparatus for calculating a frequency of appearance of each gene or a combination thereof based on the determined nucleic acid sequence to derive a TCR or BCR repertoire of the subject. Such a system and systems comprising one or more additional features explained herein are referred to as "repertoire analysis system of the present invention". The repertoire analysis system of the present invention materializes the "repertoire analysis method of the present invention".

In another embodiment, the nucleic acid sample comprises nucleic acid sequences of a plurality of types of T cell receptors (TCR) or B cell receptors (BCR), and the apparatus of (2) is configured to be able to determine the nucleic acid sequences by a single sequencing.

In another embodiment, the single sequencing is characterized in that at least one of the sequences used as a primer in amplification from the nucleic acid sample to a sample for sequencing has the same sequence as a C region. The system of the present invention can reduce or eliminate bias that can occur by determining a plurality of types of sequences by a single sequencing. Thus, the system of the present invention is useful especially in accurately detecting a TCR or BCR read that occurs at a low frequency.

In another embodiment, the single sequencing is characterized in that at least one of the sequences used as a primer in amplification from the nucleic acid sample to a sample for sequencing has the same sequence as a nucleic acid sequence encoding a C region or a complementary strand thereof. Such a primer may be furnished in the apparatus, comprised in a kit, or provided separately. Any TCR or BCR can be amplified in the same manner to achieve unbiasedness by using a primer having the same sequence as a nucleic acid sequence encoding a C region or a complementary strand thereof.

In one embodiment, a nucleic acid sequence comprised in a nucleic acid sample provided by the kit of the present invention is unbiasedly amplified, where the amplification is not V region specific amplification. Bias can be further reduced or eliminated compared to a case of performing unbiased amplification by devising a multiplex or the like using a V specific primer.

In one embodiment, the repertoire subjected to analysis of the system of the present invention is the repertoire of variable regions of a BCR, and the nucleic acid sequence is a BCR nucleic acid sequence. BCRs are considered to be prone to having a mutation, especially in a V region. Thus, accurate analysis of a BCR repertoire is difficult with an approach using V region specific amplification. Use of the system of the present invention also allows accurate analysis of a BCR repertoire.

In another aspect, the present invention provides a system (analysis system) for analyzing a disease, disorder, or condition of a subject, comprising the analysis system of the present invention and means for analyzing the disease, disorder, or condition of the subject based on a TCR or BCR repertoire derived based on the system. The means for analyzing a disease, disorder, or condition of a subject based on a TCR or BCR repertoire derived based on the system of the analysis system of the present invention starts from linking derived read data consisting of read types, number of reads, read frequency, V region, J region, C region CDR3 sequence or the like with clinical information such as disease, disorder, or condition to form a database by using a spreadsheet such as EXCEL. First, for a derived individual read sequence: 1. a TCR having a known function such as NKT or MAIT is searched; 2. existing public database is searched for collation with a TCR or BCR with a known function such as antigen specificity; and 3. the constructed database or an existing public database is searched to associate a common sample origin, property, or function with a disease, disorder, or condition. Next, for a read sequence in a sample: 1. it is clarified whether a specific read frequency increases (clonality increases); 2. examination is carried out to find out whether a specific V chain or J chain usage frequency increases or decreases depending on the condition of a disorder or onset of a disease; 3. examination is carried out to find out whether the length of a CDR3 sequence in a specific V chain increases or decreases depending on the condition of a disorder or onset of a disease; 4. the composition or sequence of a CDR3 region that changes depending on the

condition of a disorder or onset of a disease is examined. 5. a read that appears or disappears depending on the condition of a disorder or onset of a disease is searched; 6. a read that increases or decreases depending on the condition of a disorder or onset of a disease is searched; 7. a read that appears or increases/decreases depending on the condition of a disorder or onset of a disease is searched in another sample and associated with a disease, disorder, or condition; 8. a diversity index or similarity index is calculated with a statistical analysis software such as ESTIMATES or R (vegan) by using data such as number of samples, read type, or number of reads; and 9. a change in the diversity index or similarity index can be associated with the condition of a disorder or onset of a disease.

In one embodiment, the disease, disorder, or condition of the subject that can be analyzed by the analysis system of the present invention includes, but is not limited to, hematological tumor, colon cancer, immunological condition, rheumatoid arthritis, adult T-cell leukemia, T-cell large granular lymphocyte leukemia, idiopathic thrombocytopenic purpura, and the like.

In another aspect, the present invention provides a system (treatment system or prevention system) for treating or preventing a disease, disorder, or condition of a subject determined by the analysis system of the present invention, comprising: means for quantitatively associating the disease, disorder, or condition of the subject with the TCR or BCR repertoire; and means for selecting means for suitable treatment or prevention from the quantitative association.

The means for quantitatively associating the disease, disorder, or condition of the subject with the TCR or BCR repertoire in the system of the present invention can be materialized by the following configuration or the like. In other words, this can be materialized by reading out information of a repertoire derived out by the analysis system of the present invention and reading out information related to a disease, disorder, or condition of a subject and associating them. From the aggregated read data that is derived out, a V region, a J region, and a C region are assigned from collation with an existing reference sequence and a CDR3 sequence is determined. Matching reads are added up based on the V region, J region and CDR3 sequence. For each unique read (read with no other same sequence), the number of reads detected and the ratio with respect to the total number of reads (frequency) in a sample are calculated. The information (read sequence, number of reads, read frequency, V region, J region, C region, or CDR3 sequence) is linked to the clinical information of a subject (medical history, disease name, disease type, degree of progression, severity, HLA type, immune status or the like) to form a database by using a spreadsheet such as EXCEL or software having a database construction feature. Read sequences in a sample are sorted by the number of reads or frequency and ranked. Further, the number of reads is added up by each V region or J region to calculate usage frequency of a V region or usage frequency of a J region. Base on such information: 1. it is clarified whether a specific read frequency increases (clonality increases); 2. examination is carried out to find out whether a specific V chain or J chain usage frequency increases or decreases depending on the condition of a disorder or onset of a disease; 3. examination is carried out to find out whether the length of a CDR3 sequence in a specific V chain increases or decreases depending on the condition of a disorder or onset of a disease; 4. the composition or sequence of a CDR3 region that changes depending on the condition of a disorder or onset of a disease is examined. 5. a read that appears or disappears depending on the condition of a disorder or onset of a disease is searched; 6. a read that increases or decreases depending on the condition of a disorder or onset of a disease is searched; 7. a read that appears or increase/decrease depending on the condition of a disorder or onset of a disease is searched in another sample and associated with a disease, disorder, or condition; 8. a diversity index or similarly index is calculated with a statistical analysis software such as ESTIMATES or R (vegan) by using data such as number of samples, read type, or number of reads; and 9. a change in the diversity index or similarity index can be associated with the condition of a disorder or onset of a disease. The means for selecting means for suitable treatment or prevention from quantitative association can have the following configuration. Specifically, the selection of the selection means can be materialized by associating data indicating the quantitative properties with information on treatment, therapy, or prevention up to this point or currently available information and selecting those that can improve the subsequent prognosis.

In one embodiment, the disease, disorder, or condition of the subject includes, but is not limited to, hematological tumor, colon cancer, immunological condition, rheumatoid arthritis, adult T-cell leukemia, T-cell large granular lymphocyte leukemia, idiopathic thrombocytopenic purpura, and the like.

(Application) The present invention can be used to calculate a base sequence (read) of a TCR or BCR gene identified by a large-scale sequencing and a frequency of appearance thereof with software to draw a list, a distribution, or a graph. Based on such information, a change in a repertoire is detected by using the following various indicators. Association with a disease or disorder can be found based on such a change.

In one aspect, the present invention provides a method of detecting a usage frequency of a V gene by using the analysis method or the analysis system of the present invention. A V gene of each read can be identified to calculate the percentage of each V gene with the respect to the entire TCR or BCR gene. It is possible to find an increase or decrease in usage frequency of V associated with a disease or pathological condition.

In another aspect, the present invention provides a method of detecting a usage frequency of a J gene by using the analysis method or the analysis system of the present invention. A J gene of each read can be identified to calculate the percentage of each J gene with respect to the entire TCR or BCR gene. It is possible to find an increase or decrease

in usage frequency of J associated with a disease or pathological condition.

In another aspect, the present invention provides a method of detecting a usage frequency of subtype frequency analysis (BCR) by using the analysis method or the analysis system of the present invention. It is possible to calculate the frequency of presence of subtypes IgA1, IgA2, IgG1, IgG2, IgG3, and IgG4 based on sequencing of a C region. It is possible to find an increase or decrease in a specific subtype associated with a disease or pathological condition.

In another aspect, the present invention provides a method of analyzing a pattern of CDR3 sequence lengths by using the analysis method or the analysis system of the present invention. The CDR3 base sequence length of each read can be calculated to find the distribution thereof. A normal distribution-like peak pattern is exhibited from normal TCRs or BCRs. It is possible to find the association thereof with a disease or pathological condition by detecting a peak deviating away from a normal distribution.

In another aspect, the present invention provides a method of analyzing clonality of a TCR or a BCR by using the analysis method or the analysis system of the present invention. Reads having the same sequence are classified based on V sequence, J sequence, and CDR3 sequence of each read to calculate the number of copies thereof. It is possible to find a read that is present at a high frequency by calculating the percentage of the number of copies of each read relative to the number of all reads. The degree of clonality is assessed by sorting the reads in descending order by the frequency of appearance and comparing the percentage or number of reads that are present at a high frequency with a normal sample. A change in TCR or BCR clonality associated with a disease or pathological condition is examined therewith. The degree of clonality can be used particularly in detecting a leukemic cell or the like.

In another aspect, the present invention provides a method of extracting an overlapping read by using the analysis method or the analysis system of the present invention. A read of a sample classified by a specific disease, disease type, pathological condition, tissue, genotype (HLA or the like) is searched to extract overlapping TCR or BCR reads between samples. It is possible to find a TCR or BCR gene associated with a condition of a disease or disorder therewith. It is possible to identify a disease specific T cell involved in the pathology of an autoimmune disease, a B cell producing a disease associated antibody, a cancer specific T cell attacking a cancer cell, or the like.

In another aspect, the present invention provides a method of searching for a disease specific TCR or BCR clone by using the analysis method or the analysis system of the present invention. It is possible to predict the progression or amelioration in a pathological condition or the onset of a disease by searching for a TCR or BCR read associated with a specific condition of a disorder or disease in a test sample and finding the appearance or disappearance, or increase or decrease thereof.

In another aspect, the present invention provides a method of analyzing a subject with a diversity index by using the analysis method or the analysis system of the present invention. Alternatively, the present invention provides a method of assisting analysis on a subject with a diversity index by using the analysis method or the analysis system of the present invention. Read sequences identified based on a CDR3 sequence are counted, and the number of read types and number of individuals are calculated to form an index for diversity of a TCR or BCR repertoire. The Shannon-Wiener's diversity index (H'), Simpson's diversity index (λ, 1 - λ, or 1/λ), Pielou's evenness index (J'), Chaol index or the like is used to assess diversity by comparison with a normal sample. The index can be utilized as an indicator for measuring a degree of recovery of an immune system after bone marrow transplantation. Further, the index can be utilized as an indicator for detecting an abnormality in an immune system cell accompanied by a hematopoietic tumor.

In one embodiment, a method of analyzing a subject with a diversity index uses the diversity index as an indicator for measuring a degree of recovery of an immune system after bone marrow transplantation or as an indicator for detecting an abnormality in a cell of the immune system accompanied by a hematopoietic tumor. Such analysis using a diversity index was difficult with a conventional system.

Various diversity indices can be calculated by using an EXCEL spreadsheet or software such as ESTIMATES (Colwell, R. K. et al. Journal of Plant Ecology 5: 3-21.) or R package (vegan) from data for the number of samples, read types, or the number of reads. The Shannon-Wiener's diversity index (H'), Simpson's diversity index (λ, 1 - λ, or 1/λ), Pielou's evenness index (J'), and Chaol index are found by the mathematical equations shown below. N: total number of reads, $n_i$: number of reads in read i

Shannon-Weaver index H'

[Numeral 1]

$$H' = -\sum_{i=1}^{S} \frac{n_i}{N} \ln \frac{n_i}{N}$$

Simpson's index λ

[Numeral 2]

$$1-\lambda = 1-\sum_{i=1}^{S}\left(\frac{n_i(n_i-1)}{N(N-1)}\right)$$

Inverse Simpson's index

[Numeral 3]

$$\frac{1}{\lambda}$$

Pielou's J

[Numeral 4]

$$J=\frac{H'}{\log S}$$

$S_{Chaol}$ $S_{obs}$: total number of read types, $F_1$: singleton read, $F_2$: doubleton read

[Numeral 5]

$$S_{Chao1} = S_{obs}-\left(\frac{n-1}{n}\right)\frac{F_1(F_1-1)}{2(F_2+1)}$$

In another aspect, the present invention is a method of analyzing a subject with a similarity index by using the analysis method or the analysis system of the present invention. Alternatively, the present invention provides a method of assisting the analysis on a subject with a similarly index by using the analysis method or the analysis system of the present invention. The number of individuals and the number of types of read sequences identified based on a CDR3 sequence are calculated to find the degree of similarity of TCR or BCR repertoires between samples to be compared. The Morisita-Horn index, Kimoto's $C\pi$ index, or Pianka's $\alpha$ index is used to find a degree of similarity between samples. Such an index can be utilized in the assessment of a degree of similarity of repertoires between matching and mismatching HLA types, assessment of a degree of similarity of repertoires between a recipient and a donor after bone marrow transplantation.

In one embodiment, the similarity index is used as assessment of a degree of similarity of repertoires between matching and mismatching HLA types, or as assessment of a degree of similarity of repertoires between a recipient and a donor after bone marrow transplantation. Such analysis using a similarity index was difficult with a conventional system. Various similarity indices can be calculated with ESTIMATES (Colwell, R. K. et al. Journal of Plant Ecology 5: 3-21.) or R package (vegan) by using the following mathematical equations. The Morisita-Horn index, Kimoto's $C\pi$ index, and Pianka's $\alpha$ index are found by the mathematical equations shown below. Morisita-Horn index, $X_i$: number of times read i appear in all X reads from one of the samples, $y_i$: number of times read i appear in all Y reads from the other sample, S: number of unique reads.

[Numeral 6]

$$C_{MH} = \frac{2\sum_{i=1}^{S} x_i y_i}{\left(\frac{\sum_{i=1}^{S} x_i^2}{X^2}+\frac{\sum_{i=1}^{S} y_i^2}{Y^2}\right)XY}$$

Kimoto's π index

[Numeral 7]

$$C_\pi = \frac{2\sum_{i=1}^{S} x_i y_i}{\left(\sum_{i=1}^{S} p_{xi}^2 + \sum_{i=1}^{S} p_{yi}^2\right) XY}$$

[Numeral 8]

$$p_{xi} = \frac{x_i}{X}, \qquad p_{yi} = \frac{y_i}{Y}$$

Pianka's α index

[Numeral 9]

$$\alpha = \frac{\sum_{i=1}^{S} p_{xi} p_{yi}}{\sqrt{\sum_{i=1}^{S} p_{xi}^2 \sum_{i=1}^{S} p_{yi}^2}}$$

The present invention can use next generation sequencing technologies to prepare a sample for quantitative analysis of a repertoire of variable regions of T cell receptors (TCR) or B cell receptors (BCR). Such sequencing technologies can obtain a million or more reads from a sample at a reasonable cost. Even a genotype that exists at a low frequency of 1/1,000,000 or less can be detected by using these technologies in a specific and unbiased manner. An unbiased amplification method for amplifying all the different types of sequences of a specific portion of a gene or a transcript from a sample derived from a DNA of blood, bone marrow or the like is achieved.

<Cancer idiotype peptide sensitization immune cell therapy>

In one aspect, the present invention provides a method of preparing a composition for use in a cancer idiotype peptide sensitization immune cell therapy for a subject. The method comprises the steps of: (1) analyzing a T cell receptor (TCR) or B cell receptor (BCR) repertoire of the subject by using the repertoire analysis method of the present invention or the repertoire analysis system of the present invention; (2) determining a TCR or BCR derived from a cancer cell of the subject based on a result of the analysis, wherein the determining is performed by selecting a high ranking sequence in a frequency of presence ranking of a TCR or BCR gene derived from the cancer cell of the subject as the TCR or BCR derived from the cancer cell; (3) determining an amino acid sequence of a candidate HLA test peptide based on the determined TCR or BCR derived from cancer, wherein the determining is performed based on a score calculated by using an HLA binding peptide prediction algorithm; and (4) synthesizing the determined peptide. In this regard, a synthesized peptide can be used in a cancer idiotype peptide sensitization immune cell therapy. In some cases, this method is called a "cancer idiotype peptide sensitization immune cell therapy" herein.

A cancer idiotype peptide sensitization immune cell therapy can be performed in clinical practice by using the following specific procedures. In short, for example, (1) peripheral blood cells of a cancer patient suffering from a hematological tumor can be collected and lymphoid cells can be separated to subsequently perform the repertoire analysis method of the present invention, and a cancer idiotype peptide sensitization immune cell therapy can be performed with the use thereof.

In another embodiment, the repertoire analysis method of the present invention can be performed for a TCR in the case of T cell based tumors or for a BCR in the case of B cell based tumors. Subsequently, a high ranking sequence in a frequency of presence ranking of a TCR or BCR gene is selected as the TCR or BCR derived from the cancer cell. A peptide that binds to a human leukocyte antigen (HLA) of the cancer patient determined separately from a sequence comprising a CDR3 region of the TCR or BCR gene is predicted using an HLA binding peptide prediction program (any known program can be used as explained in other parts of the specification). In addition, an HLA binding peptide is synthesized by a peptide synthesizer and the following is subsequently performed. For a tailor-made peptide sensitization

CTL therapy, it is possible to collect peripheral blood mononuclear cells from a patient, culture a mixture of the mononuclear cells or antigen presenting cells from the patient and a $CD8^+$ T cell added with the peptide, and to apply a stimulation with an antigen peptide.

For a tailor-made peptide sensitization CTL therapy, a CTL therapy can be administered by introducing the peptide stimulated lymphoid cell into the patient.

Alternatively, another method of a tailor-made peptide sensitization DC vaccine therapy can be materialized by collecting peripheral blood mononuclear cells of a patient, separating a mononuclear cell, inducing differentiation into a dendritic cell (DC) in the presence of a differentiation inducing agent, adding the peptide and culturing the mixture, and introducing the peptide sensitized dendritic cell into the patient to administer dendritic cell therapy.

A cancer idiotype peptide sensitization immune cell therapy can be used in patients with hematologic cancer such as acute myeloid leukemia and related progenitor cell neoplasm, lymphoblastic leukemia/lymphoma, T lymphoblastic leukemia/lymphoma, chronic lymphocytic leukemia/small lymphocytic lymphoma, B-cell prolymphocytic leukemia, hairy cell leukemia, T-cell prolymphocytic leukemia, T-cell large granular lymphocyte leukemia, and adult T cell leukemia/lymphoma; diseases similar to leukemia such as multiple myeloma and myelodysplastic syndrome; autoimmune diseases such as rheumatoid arthritis, systemic lupus erythematosus, and type I diabetes; and various infections; as well as for patients with terminal cancer, refractory autoimmune disease, or severe infection. In particular, it is problematic for an antibody therapy targeting a tumor cell or the like when a target antigen is not expressed on a tumor cell or a target antigen is also expressed on a normal cell. In comparison thereto, a therapy with higher specificity and fewer side effects is expected because a sequence specific to a tumor cell is selected and utilized.

In one embodiment, the candidate HLA test peptide of step (3) in the present invention is determined using BIMAS, SYFPEITHI, RANKPEP or NetMHC.

In another embodiment, the present invention comprises, after step (4) in the present invention, the step of: mixing the peptide, an antigen presenting cell or a dendritic cell derived from the subject, and a $CD8^+$ T cell derived from the subject and culturing the mixture. This is also called an improved CTL method.

For example, unlike the existing broad T cell activation by an anti-CD3 antibody or IL-2, antigen specificity is imparted to a $CD8^+$ T cell utilizing an antigen peptide so that therapy with higher specificity and fewer side effects can be expected in the improved CTL method. Further, the method is characterized in that a higher therapeutic effect can be expected because an individualized peptide created based on the information obtained from a tumor cell of the patient is used.

An improved CTL method can be used in, for example, patients with hematologic cancer such as acute myeloid leukemia and related progenitor cell neoplasms, lymphoblastic leukemia/lymphoma, T lymphoblastic leukemia/lymphoma, chronic lymphocytic leukemia/small lymphocytic lymphoma, B-cell prolymphocytic leukemia, hairy cell leukemia, T-cell prolymphocytic leukemia, T-cell large granular lymphocyte leukemia, and adult T cell leukemia/lymphoma; diseases similar to leukemia such as multiple myeloma and myelodysplastic syndrome; autoimmune diseases such as rheumatoid arthritis, systemic lupus erythematosus, and type I diabetes; and various infections; as well as patients with terminal cancer, refractory autoimmune disease, or severe infection.

In another embodiment, the present invention comprises, after step (4) of the present invention, the step of: mixing the peptide with a dendritic cell derived from the subject and culturing the mixture. This is also called a DC vaccine therapy.

For example, since an individualized peptide is created based on the sequence information obtained from a tumor cell derived from the patient in DC vaccine therapy, such therapy does not act on a normal cell but acts more specifically to a tumor cell such that a high therapeutic effect can be expected. Since a peptide is used as an antigen, unlike proteins, there is an advantage in that chemical synthesis can be readily performed.

DC vaccine therapy can be used in, for example, patients with hematologic cancer such as acute myeloid leukemia and related progenitor cell neoplasms, lymphoblastic leukemia/lymphoma, T lymphoblastic leukemia/lymphoma, chronic lymphocytic leukemia/small lymphocytic lymphoma, B-cell prolymphocytic leukemia, hairy cell leukemia, T-cell prolymphocytic leukemia, T-cell large granular lymphocyte leukemia, and adult T cell leukemia/lymphoma; diseases similar to leukemia such as multiple myeloma and myelodysplastic syndrome; autoimmune diseases such as rheumatoid arthritis, systemic lupus erythematosus, and type I diabetes; and patients with various infections; as well as for patients with terminal cancer, a refractory autoimmune disease, or severe infection.

In another embodiment, the present invention comprises, after step (4) of the present invention, the steps of: mixing the peptide, an antigen presenting cell or the dendritic cell derived from the subject, and a $CD8^+$ T cell derived from the subject and culturing the mixture to produce a $CD8^+$ T cell-dendritic cell/antigen presenting cell-peptide mixture; and mixing the peptide with the dendritic cell derived from the subject and culturing the mixture to produce a dendritic cell-peptide mixture. This is also called a patient autoimmune cell therapy.

For example, a $CD8^+$ T cell is stimulated and activated with a peptide derived from the patient as in a CTL therapy and peptide sensitization of a dendritic cell is performed in a patient autoimmune cell therapy. Such a therapy is characterized in that a synergistic effect of a sustained effect due to the dendritic cell utilized as the antigen presenting

cell and an acute effect due to specificity imparted CTL can be expected by introducing both the dendritic cell and the CD8+ cell derived from the patient into the patient.

A patient autoimmune cell therapy can be used in, for example, patients with hematologic cancer (leukemia etc.) such as acute myeloid leukemia and related progenitor cell neoplasms, lymphoblastic leukemia/lymphoma, T lymphoblastic leukemia/lymphoma, chronic lymphocytic leukemia/small lymphocytic lymphoma, B-cell prolymphocytic leukemia, hairy cell leukemia, T-cell prolymphocytic leukemia, T-cell large granular lymphocyte leukemia, and adult T cell leukemia/lymphoma; diseases similar to leukemia such as multiple myeloma and myelodysplastic syndrome; autoimmune diseases such as rheumatoid arthritis, systemic lupus erythematosus, and type I diabetes; and various infections; as well as patients with terminal cancer, a refractory autoimmune disease, or severe infection.

In another aspect, the present invention provides a method of applying a cancer idiotype peptide sensitization immune cell therapy to a subject. The method comprises the steps of: (1) analyzing a T cell receptor (TCR) or B cell receptor (BCR) repertoire of the subject by using the repertoire analysis method of the present invention or the repertoire analysis system of the present invention; (2) determining a TCR or BCR derived from a cancer cell of the subject based on a result of the analysis, wherein the determining is performed by selecting a high ranking sequence in a frequency of presence ranking of a TCR or BCR gene derived from the cancer cell of the subject as the TCR or BCR derived from the cancer cell; (3) determining an amino acid sequence of a candidate HLA test peptide based on the determined TCR or BCR derived from cancer, wherein the determining is performed based on a score calculated by using an HLA binding peptide prediction algorithm; (4) synthesizing the determined peptide; and optionally (5) administering therapy by using the synthesized peptide. The method encompasses both a method of manufacturing a therapeutic agent and a method of therapy itself. When excluding a medical act, the method can be completed before step (5).

In a preferred embodiment, the candidate HLA test peptide of step (3) is determined by using BIMAS, SYFPEITHI, RANKPEP or NetMHC in the present invention.

BIMAS is a program for estimating HLA peptide bonds provided at www-bimas.cit.nih.gov/.

SYFPEITHI is a search engine and a database for MHC ligands and peptide motifs provided at www.syfpeithi.de/.

RANKPEP is a program for predicting a peptide bond to class I and class II MHC molecules, provided at http://imed.med.ucm.es/Tools/rankpep.html.

NetMHC is a program server for predicting binding of a peptide to numerous HLA alleles, provided at www.cbs.dtu.dk/services/NetMHC/.

In a preferred embodiment, the present invention comprises, after step (4), the steps of: mixing the peptide, an antigen presenting cell or a dendritic cell derived from the subject, and a $CD8^+$ T cell derived from the subject and culturing the mixture; and administering the mixture after culturing to a patient as an improved CTL method.

In a preferred embodiment, the present invention comprises, after step (4), the steps of: mixing the peptide with the dendritic cell derived from the subject and culturing the mixture; and administering the cultured mixture to a patient as a DC vaccine therapy.

In a preferred embodiment, the present invention comprises, after step (4), the steps of: mixing the peptide, an antigen presenting cell or the dendritic cell derived from the subject and a $CD8^+$ T cell derived from the subject and culturing the mixture to produce a $CD8^+$ T cell-dendritic cell/antigen presenting cell-peptide mixture; mixing the peptide with the dendritic cell derived from the subject and culturing the mixture to produce a dendritic cell-peptide mixture; and administering the $CD8^+$ T cell-dendritic cell/antigen presenting cell-peptide mixture and the dendritic cell-peptide mixture to a patient as a patient autoimmune cell therapy.

<Isolation of tailor-made cancer specific T cell receptor gene, isolation of cancer specific TCR gene by in vitro antigen stimulation>

In another aspect, the present invention provides a technique for isolating a tailor-made cancer specific T cell receptor gene or isolating a cancer specific TCR gene by *in vitro* antigen stimulation. Thus, the present invention provides a method of preparing an isolated cancer specific TCR gene by an *in vitro* antigen stimulation, comprising: (A) mixing the peptide determined in the "Cancer idiotype peptide sensitization immune cell therapy" of the present invention or a lymphocyte derived from the subject or an antigen peptide or antigen protein derived from a subject, an inactivated cancer cell derived from the subject, and a T lymphocyte derived from the subject and culturing the mixture to produce a tumor specific T cell; (B) analyzing a TCR of the tumor specific T cell by using the repertoire analysis method of the present invention or the repertoire analysis system of the present invention; and (C) isolating a desired tumor specific T cell based on a result of the analyzing. Such an isolated cancer specific TCR gene from an *in vitro* antigen stimulation can be prepared using any well-known approach in the art once the gene information is obtained. Such an isolated tailor-made cancer specific T cell receptor gene and cancer specific TCR gene can be used to treat or prevent a variety of cancers.

Such an isolated tailor-made cancer specific T cell receptor gene and cancer specific TCR gene can be implemented in clinical practice by using the following specific procedures.

In one embodiment, therapy using an isolated tailor-made cancer specific T cell receptor gene and cancer specific TCR gene can be materialized, for example, as follows: (1) tumor cells are extracted from a cancer patient; (2) after crushing the tumor cells from the patient, the cells are separated into single cells and inactivated by radiation irradiation or chemical treatment with mitomycin C or the like; (3) peripheral blood cells are separated from whole blood of the cancer patient; (4) an RNA is extracted from cells, with some of the peripheral blood cells as an untreated control sample; (5) the inactivated tumor cells and the peripheral blood cells are mixed and cultured to activate and proliferate the tumor specific T cells; (6) after activation, the peripheral blood cells are collected, and an RNA is extracted from the cells as a post-stimulation sample; (7) the repertoire analysis method of the present invention is performed from the RNA samples extracted in (4) and (6); (8) TCR genes that have greatly increased in the stimulated sample relative to a control sample are extracted and ranked, and then high ranking TCRα and TCRβ genes are selected; (9) the full-length TCRα and TCRβ genes are cloned and introduced into a retroviral vector for gene expression; (10) a gene-introducing virus is created from the TCRα and TCRβ gene expressing retroviral vector; (11) lymphocytes collected from the patient are infected independently and successively with TCRα and TCRβ for transfection, or a gene expressing retroviral vector comprising both TCRα and TCRβ genes is created to transform both genes at once; (12) expression of TCRα/TCRβ heterodimers on a cell surface is studied; and (13) a tumor specific patient lymphocyte expressing TCRα/TCRβ of interest is introduced into the patient.

Specifically, the TCR or BCR determined by the method described in "Cancer idiotype peptide sensitization immune cell therapy" can be used as an antigen or peptide, for example, for hematological tumor in the embodiments of the present invention. In this regard, any cancer antigen or inactivated cancer tissue from a patient can be utilized in the following typical method: a method of mixing any antigen protein or any antigen peptide, T lymphocyte, and antigen presenting cell; a method of mixing a lymphocyte from a subject and an inactivated cancer cell from the subject; and a method of mixing an antigen-resenting cell, T lymphocyte, and peptide derived from a TCR or BCR determined by the repertoire analysis provided in "Cancer idiotype peptide sensitization immune cell therapy".

Thus, in one embodiment, step (A) in the present invention comprises a step of mixing the antigen peptide or antigen protein derived from the subject, the inactivated cancer cell derived from the subject, and the T lymphocyte derived from the subject and culturing the mixture to produce a tumor specific T cell.

In a further embodiment, step (A) in the present invention is a step of mixing the lymphocyte derived from the subject, the inactivated cancer cell derived from the subject, and the T lymphocyte derived from the subject and culturing the mixture to produce a tumor specific T cell.

In a further embodiment, step (A) in the present invention is a step of mixing the peptide determined in the "Cancer idiotype peptide sensitization immune cell therapy", the inactivated cancer cell derived from the subject, and the T lymphocyte derived from the subject and culturing the mixture to produce a tumor specific T cell.

Such therapy using an isolated tailor-made cancer specific T cell receptor gene and a cancer specific TCR gene can be used in patients with a wide range of cancer, including, but not limited to, adrenocortical carcinoma, anal cancer, bile duct cancer, bladder cancer, breast cancer, cervical cancer, chronic lymphocytic leukemia, chronic myelogenous leukemia, colon cancer, endometrial cancer, esophageal cancer, Ewing tumor, gallbladder cancer, Hodgkin's disease, hypopharyngeal cancer, laryngeal cancer, lip and oral cavity cancer, liver cancer, non-small-cell lung cancer, non-Hodgkin's lymphoma, melanoma, mesothelioma, multiple myeloma, ovarian cancer, pancreatic cancer, prostate cancer, gastric cancer, testicular cancer, thyroid cancer and the like.

In a further aspect, the present invention provides isolation of a tailor-made cancer specific T cell receptor gene, and isolation of a cancer specific TCR gene by searching for a common sequence. Thus, the present invention provides a method of preparing an isolated cancer specific TCR gene by searching for a common sequence, comprising the steps of: (A) providing a lymphocyte or cancer tissue isolated from subjects having a common HLA; (B) analyzing a TCR of the tumor specific T cell by using the repertoire analysis method of the present invention or the repertoire analysis system of the present invention for the lymphocyte or cancer tissue; and (C) isolating a T cell having a sequence in common with the tumor specific T cell. Once genetic information is obtained, preparation of an isolated cancer specific TCR gene by searching for a common sequence can be performed by using any well-known approach in the art. A gene obtained by such isolation of tailor-made cancer specific T cell receptor gene or isolation of a cancer specific TCR gene by searching for a common sequence can be used in therapy and prevention of a variety of cancers. The method is also called "method of isolation of tailor-made cancer specific T cell receptor gene or isolation of cancer specific TCR gene by searching for a common sequence of the present invention".

A gene obtained by such isolation of a tailor-made cancer specific T cell receptor gene or isolation of a cancer specific TCR gene by searching for a common sequence can be implemented in clinical practice by using the following specific procedures. In one embodiment, it is possible to materialize therapy using a gene obtained by the isolation of a tailor-made cancer specific T cell receptor gene or the isolation of a cancer specific TCR gene by searching for a common sequence from the following: first (1) tumor cells are extracted or peripheral blood is separated from cancer patients with the same HLA; (2) repertoire analysis is performed by using a lymphoid cell or tumor tissue comprising a tumor cell infiltrated T cell; (3) a ranking is produced for each sample based on the frequency of presence thereof, and

a tumor specific T cell exhibiting a higher frequency of presence in the tumor cell relative to the peripheral blood cell is selected; (4) a common sequence in a plurality of HLA matching cancer patients is searched for the tumor specific T cells; (5) a tumor specific TCR gene shared by the most cancer patients is selected as a tumor specific TCR for therapy; (6) the full length TCRα and TCRβ genes are cloned and introduced into a retroviral vector for gene expression; (7) a gene-introducing virus is created from the TCRα and TCRβ gene expressing retroviral vector; (8) lymphocytes collected from the patients are infected independently and successively with TCRα and TCRβ for transfection, or a gene expressing retroviral vector comprising both TCRα and TCRβ genes is created to transform both genes at once; (9) expression of TCRα/TCRβ heterodimers on a cell surface is confirmed; and (10) a tumor specific patient lymphocyte expressing the TCRα/TCRβ of interest is introduced into the cells of the patient, which can materialize therapy using a gene obtained by isolation of a tailor-made cancer specific T cell receptor gene or isolation of a cancer specific TCR gene by searching for a common sequence.

Such therapy using a gene obtained by isolation of a tailor-made cancer specific T cell receptor gene or isolation of a cancer specific TCR gene by searching for a common sequence can be used in patients with a wide range of cancer including, but not limited to, adrenocortical carcinoma, anal cancer, bile duct cancer, bladder cancer, breast cancer, cervical cancer, chronic lymphocytic leukemia, chronic myelogenous leukemia, colon cancer, endometrial cancer, esophageal cancer, Ewing tumor, gallbladder cancer, Hodgkin’s disease, hypopharyngeal cancer, laryngeal cancer, lip and oral cavity cancer, liver cancer, non-small-cell lung cancer, non-Hodgkin’s lymphoma, melanoma, mesothelioma, multiple myeloma, ovarian cancer, pancreatic cancer, prostate cancer, gastric cancer, testicular cancer, thyroid cancer, and the like.

Thus, in another aspect, the present invention provides a method of isolating a cancer specific TCR gene by an *in vitro* antigen stimulation, comprising: (A) mixing an antigen peptide or antigen protein derived from a subject or a peptide determined in a cancer idiotype peptide sensitization immune cell therapy or a lymphocyte derived from the subject, an inactivated cancer cell derived from the subject, and a T lymphocyte derived from the subject and culturing the mixture to produce a tumor specific T cell; (B) analyzing a TCR of the tumor specific T cell by using the repertoire analysis method of the present invention or the repertoire analysis system of the present invention; and (C) isolating a desired tumor specific T cell based on a result of the analyzing. Once genetic information is obtained, such preparation of a cancer specific TCR gene isolated by an *in vitro* antigen stimulation can be performed using any well-known approach in the art. Such an isolated tailor-made cancer specific T cell receptor gene or cancer specific TCR gene can be used in therapy and prevention of a variety of cancers.

Thus, in one embodiment of the method of isolating a cancer specific TCR gene by an *in vitro* antigen stimulation, step (A) in the present invention comprises a step of mixing an antigen peptide or antigen protein derived from the subject, an inactivated cancer cell derived from a subject, and a T lymphocyte derived from the subject and culturing the mixture to produce a tumor specific T cell.

In a further embodiment, step (A) in the present invention is a step of mixing a lymphocyte derived from the subject, an inactivated cancer cell derived from the subject, and a T lymphocyte derived from the subject and culturing the mixture to produce a tumor specific T cell.

In a further embodiment, step (A) in the present invention is a step of mixing a peptide determined in "Cancer idiotype peptide sensitization immune cell therapy", an inactivated cancer cell derived from the subject, and a T lymphocyte derived from the subject and culturing the mixture to produce a tumor specific T cell.

In still another aspect, the present invention provides a technique of isolating a cancer specific TCR gene by searching for a common sequence or isolating a tailor-made cancer specific T cell receptor gene, comprising the steps of: (A) isolating a lymphocyte or cancer tissue from subjects having a common HLA; (B) analyzing a TCR of the tumor specific T cell by using the repertoire analysis method of the present invention for the lymphocyte or cancer tissue; and (C) isolating a T cell having a sequence in common with the tumor specific T cell. Such an isolated tailor-made cancer specific T cell receptor gene or cancer specific TCR gene can be used in therapy and prevention of a variety of cancers.

<Cell processing therapy>

In a further aspect, the present invention provides a cell processing therapy. Specifically, the present invention provides a method of preparing a T lymphocyte introduced with a tumor specific TCR gene for use in cell processing therapy, comprising the steps of: A) providing a T lymphocyte collected from a patient; B) analyzing TCRs based on the repertoire analysis method of the present invention or the repertoire analysis system of the present invention after applying an antigen stimulation to the T lymphocyte, wherein the antigen stimulation is applied with an antigen peptide or antigen protein derived from the subject, an inactivated cancer cell derived from the subject, or an idiotype peptide derived from a tumor; C) selecting an optimal TCR and an optimal antigen in the analyzed TCRs; and D) producing a tumor specific α and β TCR expression viral vector of a TCR gene of the optimal TCR. The cell processing therapy using the T lymphocyte introduced with a tumor specific TCR gene can be used for the therapy and prevention of a variety of cancers.

Such a cell processing therapy using a T lymphocyte introduced with a tumor specific TCR gene can be implemented in clinical practice by using the following specific procedures. For example, a lymphocyte introduced with a tumor specific TCR gene can be used by the method described in <Isolation of tailor-made cancer specific T cell receptor gene, isolation of cancer specific TCR gene by *in vitro* antigen stimulation> or <Isolation of tailor-made cancer specific T cell receptor gene, isolation of cancer specific TCR gene by searching for a common sequence>.

Thus, it is possible to manufacture or produce any cancer antigen or cancer peptide by synthesis as an antigen and to utilize an inactivated cancer cell collected from a patient or to utilize an idiotype peptide derived from tumor in the cell processing therapy of the present invention. As a selection method, it is possible to select an antigen highly expressed in cancer tissue or select a peptide that binds to the HLA type of a patient as an antigen.

In a preferred embodiment of the cell processing therapy of the present invention, examples of conceivable optimal antigens that can be selected include, but are not limited to, (1) an antigen highly expressed in the patient's cancer tissue, (2) an antigen that most strongly activates a T cell in an antigen specific lymphocyte stimulation test, and (3) an antigen that increases the frequency of a specific TCR the most from repertoire analysis before and after an antigen stimulation. It is also possible to conceive a method of selecting, as an optimal TCR, a TCR that has increased the most in examples of (3), where the frequency of a specific TCR increased the most from repertoire analysis before and after an antigen stimulation. It is also possible to select, as the optimal TCR, a candidate optimal TCR which is artificially transgenically introduced into a lymphocyte of a patient and exhibits the highest reactivity in actual cancer tissue of the patient as a typical example.

Such a cell processing therapy using a T lymphocyte introduced with a tumor specific TCR gene can be used in patients with a wide range of cancer Including, but not limited to, adrenocortical carcinoma, anal cancer, bile duct cancer, bladder cancer, breast cancer, cervical cancer, chronic lymphocytic leukemia, chronic myelogenous leukemia, colon cancer, endometrial cancer, esophageal cancer, Ewing tumor, gallbladder cancer, Hodgkin's disease, hypopharyngeal cancer, laryngeal cancer, lip and oral cavity cancer, liver cancer, non-small-cell lung cancer, non-Hodgkin's lymphoma, melanoma, mesothelioma, multiple myeloma, ovarian cancer, pancreatic cancer, prostate cancer, gastric cancer, testicular cancer, thyroid cancer, and the like.

Thus, in one embodiment, the antigen stimulation of the method of the present invention is applied with the antigen peptide or antigen protein derived from the subject.

In another embodiment, the antigen stimulation of the present invention is applied with the inactivated cancer cell derived from the subject.

In another embodiment, the antigen stimulation of the method of the present invention is applied with the idiotype peptide derived from tumor.

In another embodiment, step C) of the method of the present invention comprises selecting an antigen that is highly expressed in cancer tissue of the subject.

In another embodiment, step C) of the method of the present invention comprises selecting an antigen which most strongly activates a T cell in an antigen specific lymphocyte stimulation test.

In another embodiment, step C) of the method of the present invention comprises selecting an antigen that increases a frequency of a specific TCR the most from repertoire analysis conducted based on the repertoire analysis method of the present invention or the repertoire analysis system of the present invention before and after applying the antigen stimulation.

In one specific embodiment, the present invention provides a method of assessing efficacy and/or safety by using a cancer specific TCR gene isolated by <method of isolation of tailor-made cancer specific T cell receptor gene or isolation of cancer specific TCR gene by searching for a common sequence of the present invention> and performing a stimulation test *in vitro.*

Efficacy can be assessed, for example, by culturing an antigen protein or antigen peptide derived from a subject who received an antigen stimulation with an antigen protein or antigen peptide derived from the subject and a T cell introduced with a cancer specific TCR gene, an inactivated cancer cell derived from the subject who has received an antigen stimulation with an inactivated cancer cell derived from the subject, and an idiotype peptide derived from a tumor which has received an antigen stimulation with an idiotype peptide derived from a tumor, and then measuring the amount of cytokines (interferon $\gamma$ or the like) secreted out of a cell in response to T cell activation, measuring the amount of expression of a specific gene that is elevated in response to T cell activation, or measuring a cell surface molecule that is expressed or increases expression in response to T cell activation.

<Safety> When a T cell derived from the subject introduced with a cancer specific TCR gene is mixed with a normal cell derived from the subject, safety can be assessed, for example, by measuring secreted cytokines, gene expression, or expression of a cell surface molecule in response to activation of the T cell and verifying that the TCR gene introduced T cell is not activated by the normal cell.

In one embodiment, the specific steps of efficacy and/or safety assessment can be materialized as follows. For example: (1) a retroviral gene expression system is used to create a tumor specific TCR$\alpha$ and TCR$\beta$ gene introduced T lymphoid cell; (2) when assessing efficacy, a cancer cell derived from a patient is extracted, separated, and immortalized,

and then subjected to mixing and culturing with a T lymphocyte introduced with a tumor specific TCR gene; (3) reactivity to a tumor cell can be quantitatively assessed to select a TCR gene reacting more strongly to a tumor cell by performing a cell proliferation test (thymidine uptake test, MTT test, IL-2 production test, or the like) on the cultured cell; (4) when assessing safety, a control, which is an existing cell line, normal tissue free of patient's cancer cells (part of the normal tissue collected in the process of tumor extraction), or patient's peripheral blood cells in the case of solid tumor, is used and immortalized, and then subjected to mixing and culturing with a T lymphocyte introduced with a tumor specific TCR gene; and (5) reactivity to a tumor cell can be quantitatively assessed to select a TCR gene that exhibits no reactivity to a normal cell by using the cultured cell and performing a cell proliferation test (thymidine uptake test, MTT test, IL-2 production test, or the like).

Thus in another aspect, the present invention provides a cell processing therapy, comprising: A) collecting a T lymphocyte from a patient; B) analyzing TCRs based on the repertoire analysis method or the repertoire analysis system of the present invention after applying antigen stimulation to the T lymphocyte, wherein the antigen stimulation is applied by an antigen peptide or antigen protein derived from the subject, an inactivated cancer cell derived from the subject, or an idiotype peptide derived from tumor; C) selecting an optimal TCR and an optimal antigen in the analyzed TCRs; D) producing a tumor specific $\alpha$ and $\beta$ TCR expression viral vector of a TCR gene of the optimal TCR; and E) introducing the T lymphocyte introduced with a tumor specific TCR gene into the patient.

A method of performing the steps of introducing a resulting T lymphocyte introduced with a tumor specific TCR gene into the patient comprises the following steps: A) manufacturing a T lymphocyte introduced with a tumor specific TCR gene; B) confirming expression of tumor specific TCR$\alpha$ and TCR$\beta$; and C) intravenously introducing the T lymphocyte introduced with a tumor specific TCR gene by intravenous drip.

Thus, in one embodiment, the antigen stimulation in the cell processing therapy of the present invention is applied with the antigen peptide or antigen protein derived from the subject.

In another embodiment, the antigen stimulation in the cell processing therapy of the present invention is applied with the inactivated cancer cell derived from the subject.

In another embodiment, the antigen stimulation in the cell processing therapy of the present invention is applied with the idiotype peptide derived from tumor.

In another embodiment, step C) in the cell processing therapy of the present invention comprises selecting an antigen that is highly expressed in cancer tissue of the subject.

In another embodiment, step C) in the cell processing therapy of the present invention comprises selecting an antigen which most strongly activates a T cell in an antigen specific lymphocyte stimulation test.

In another embodiment, step C) in the cell processing therapy of the present invention comprises selecting an antigen that increases a frequency of a specific TCR the most from repertoire analysis conducted based on the repertoire analysis method of the present invention before and after applying the antigen stimulation.

<Isolation of human form antibody utilizing BCR repertoire analysis>

As one embodiment, the repertoire analysis method of the present invention can be used to perform BCR gene repertoire analysis to quickly obtain a human form antibody specific to a target antigen by the methods described below. (A) a method of immunizing a mouse with a target antigen protein or antigen peptide and separating a cell population (e.g., spleen, lymph node, or peripheral blood cells) comprising an antibody producing B cell from the mouse to analyze immunoglobulin heavy chain and light chain genes by BCR repertoire analysis using the repertoire analysis method of the present invention

(A1) the method of A, wherein the immunized mouse is a KM mouse capable of producing a complete human antibody while maintaining antibody diversity

(A2) the method of A, wherein the immunized mouse is a humanized mouse created by transplanting a human stem cell into an NOG (NOD/Shi-scid, IL-2R$\gamma$null) mouse exhibiting severe combined immunodeficiency made by mating an IL-2 receptor $\gamma$ chain knockout mouse with a NOD/scid mouse

(B) comparing immunoglobulin heavy chain and light chain genetic sequences obtained from samples derived from a control mouse and an immunized mouse or mice before and after antigen immunization, and frequencies thereof

(C) identifying immunoglobulin heavy chain and light chain genes that are strongly expressed or increased after immunization in an immunized mouse

(D) a method of selecting immunoglobulin heavy chain and light chain genes selected from step C and inserting the genes to match one type of antibody expression vector or inserting the genes separately into two types of antibody expression vectors

(E) introducing the immunoglobulin heavy chain and light chain gene expression vector made in step D into a eukaryotic cell such as CHO (Chinese Hamster Ovary) and culturing the cell

(F) separating/purifying an antibody molecule produced and secreted by a genetically modified cell to inspect the

specificity to a target antibody protein or peptide.

These are methods of directly and quickly obtaining an antigen specific human form antibody without alteration into a chimeric antibody or humanized antibody of a human antibody after obtaining an antibody gene derived from an animal by steps A to F. The methods can be used in the development and manufacture of an antibody medicine consisting of a human form antibody.

For KM mice used in this embodiment, Ishida I, Tomizuka K, Yoshida H, Tahara T, Takahashi N, Ohguma A, Tanaka S, Umehashi M, Maeda H, Nozaki C, Halk E, Lonberg N. Production of human monoclonal and polyclonal antibodies in TransChromo animals. Cloning Stem Cells. 2002; 4(1): 91-102. Review can be referred. For NOG mice, Ito M, Hiramatsu H, Kobayashi K, Suzue K, Kawahata M, Hioki K, Ueyama Y, Koyanagi Y, Sugamura K, Tsuji K, Heike T, Nakahata T. NOD/SCID/gamma(c)(null) mouse: an excellent recipient mouse model for engraftment of human cells. Blood. 2002 Nov 1; 100(9): 3175-82 can be referred. For CHO cells/antibody production, Jayapal KP, Wlaschin KF, Hu W-S, Yap MGS. Recombinant protein therapeutics from CHO cells-20 years and counting. Chem Eng Prog. 2007; 103: 40?47.; Chusainow J, Yang YS, Yeo JH, Toh PC, Asvadi P. Wong NS, Yap MG. A study of monoclonal antibody-producing CHO cell lines: what makes a stable high producer? Biotechnol Bioeng. 2009 Mar 1; 102(4): 1182-96 can be referred.

<Isolation of human form antibody utilizing BCR repertoire analysis>

As one embodiment, the BCR gene repertoire analysis method can be utilized to quickly obtain a human form antibody specific to a target antigen by the methods described below.

(A) a method of immunizing a mouse with a target antigen protein or an antigen peptide and separating a cell population (e.g., spleen, lymph node, or peripheral blood cells) comprising an antibody producing B cell from the mouse to analyze immunoglobulin heavy chain and light chain genes by a BCR repertoire analysis method
(A1) the method of A, wherein the immunized mouse is a KM mouse capable of producing a complete human antibody while maintaining antibody diversity
(A2) the method of A, wherein the immunized mouse is a humanized mouse created by transplanting a human stem cell into an NOG (NOD/Shi-scid, IL-2Rγnull) mouse exhibiting severe combined immunodeficiency made by mating an IL-2 receptor γ chain knockout mouse with an NOD/scid mouse
(B) comparing immunoglobulin heavy chain and light chain genetic sequences obtained from samples derived from a control mouse and an immunized mouse or mice before and after antigen immunization, and frequencies thereof
(C) identifying immunoglobulin heavy chain and light chain genes that are strongly expressed or increase after immunization in an immunized mouse
(D) a method of selecting immunoglobulin heavy chain and light chain genes selected from step C and inserting the genes to match one type of antibody expression vector or inserting the genes separately into two types of antibody expression vectors
(E) introducing the immunoglobulin heavy chain and light chain gene expression vector made in step D into a eukaryotic cell such as CHO (Chinese Hamster Ovary) and culturing the cell
(F) separating/purifying an antibody molecule produced and secreted by a genetically modified cell to inspect the specificity to a target antibody protein or peptide.

These are methods of directly and quickly obtaining an antigen specific human form antibody without alteration into a chimeric antibody or humanized antibody of a human antibody after obtaining an antibody gene derived from an animal by steps A to F. The methods can be used in the development and manufacture of an antibody medicine consisting of a human form antibody.

Embodiments of such methods include the following. As one example thereof,

1. A KM mouse is immunized with a Myelin Oligodendrocyte Glycoprotein (MOG35-55, MOG), which is an experimental autoimmune encephalomyelitis antigen peptide. The same quantity of 2mg/mL MOG peptide and complete Freund's adjuvant are mixed to create an emulsion. The mouse is subcutaneously immunized with 200 μg of MOG and simultaneously immunized in the peritoneal cavity with 400 ng of pertussis toxin. A control mouse is immunized with control PBS and complete Freund's adjuvant.
2. On day 2 after the first immunization, the mouse is immunized with 400 ng of pertussis toxin. After confirming an outbreak on day 10 after the immunization, the spleen is extracted from the mouse with an episode of encephalomyelitis.
3. The spleens of the challenged mouse and control mouse are used to carry out next generation BCR repertoire analysis. Frequencies of appearance of individual BCR sequences are counted and ranked for IgG immunoglobulin

heavy chains and immunoglobulin light chains.

4. BCR sequences with a large increase in the frequency of appearance in the challenged mouse relative to the control mouse are extracted and ranked. A combination of high ranking BCR sequences induced by the antibody administration is identified as a MOG specific antibody gene.
5. A full length human immunoglobulin sequence is cloned by PCR-cloning from a BCR gene amplicon amplified from the challenged mouse. Each of the IgG immunoglobulin heavy chain and the immunoglobulin light chain is cloned in an antibody expression vector. There is a method of inserting the genes to match one type of antibody expression vector or inserting the genes separately into two types of antibody expression vectors.
6. A CHO (Chinese Hamster Ovary) cell is transformed using Lipofectamine 3000 (Life Science) and IgG immunoglobulin heavy chain and immunoglobulin light chain are introduced with the constructed expression vector.
7. A CHO cell culture solution is collected. Secreted antibody proteins are collected by purification with a protein A affinity column and concentration with gel filtration.
8. Binding activity to MOG35-55 or MOG protein is measured by an ELISA assay using the collected antibody to investigate the specificity of the antibody.
9. When sufficient specificity is obtained, a cell line stably expressing an antibody is acquired and a human form anti-MOG antibody is manufactured with a large-scale culturing system.

Descriptions in all publications including reference literatures such as scientific literatures, patents, and patent applications cited herein are incorporated herein by reference to the same extent that the entirety of each document is specifically described.
The present invention has been described above with preferred embodiments to facilitate understanding. The present invention is described below based on Examples. The aforementioned description and the following Examples are not provided to limit the present invention, but for the sole purpose of exemplification. Thus, the scope of the present invention is not limited by the embodiments and Examples specifically described herein and is limited only by the scope of claims.
While the present invention is explained hereinafter in further detail with the following Examples, the present invention is not restricted in any way by the following Examples and the like.

[Examples]

The following reagents were used.

[Table 1]

| Regent | | Manufacturer |
|---|---|---|
| Kit | PrimeScript II High Fidelity One TaKaRa step RT-PCR kit | |
| Total RNA | Total RNA purified from mouse T cells (β immobilized) | |
| Inhibitor | RNasin Plus RNase Inhibitor (40 Promega U/μl) Ambion RNase Inhibitor (Cloned) (40 U/μl) Ambion SUPERaseIn RNase Inhibitor (20 U/μl) | |
| SS4 | SuperScript IV Reverse Invitrogen Transcriptase (200 U/μl) (=SS4) | |
| Primer | CleanAmp™ Precision Primers (Bolck TriLink Primer)<br>RT primer | |
| TS-Oligo | Template switch oligo (3 bases of 3' are RNA) | |

The sequences of oligonucleotides used are the following:

Block primer: GAGGGTAGCCTTTTGTTTGTTTGCAATCTC (SEQ ID NO: 1)

RT primer: AAGCACACGAGGGTAGCCTTTTGTTTGTTTGCAA (SEQ ID NO: 2)

Template switch Oligo (3 bases of 3' are RNA): AAGCAGTGGTATACCCGCAGAGTACATrGrGrG (SEQ ID NO: 3).

The block primer and RT primer are reverse primers that are specific to the constant region of a mouse TCRβ chain. The full length is designed to hybridize to a TCRβ mRNA. The RT primer is designed to be nested on the 3' side by 4 bases. The RT primer has the 5' side extended by 8 bases to enhance the affinity. The constant region of an mRNA encoding a TCRβ chain to the 5' terminus can be amplified by using such primers and performing reverse transcription template switching PCR. The amplified region includes an untranslated region or reconstituted VDJ comprising an antigen

recognition site and the like. Thus, a cDNA library of untranslated regions and antigen recognition sites of a TCRβ chain can be constructed by using the total RNA collected from a T cell population as a template and performing reverse transcription template switching PCR with such primers. If a single cell of T cell sorted by a cell sorter or the like is directly used as a template (RNA in the cells would be the template), the antigen recognition site of a TCRβ chain of an individual cell can be specifically amplified and sequenced.

In this test, reverse transcription template switching PCR was performed in one step. Typically, a reaction mixture with the composition in the following Table was used.

[Table 2]

| | | volume (μl) | final conc | |
|---|---|---|---|---|
| * | 2x one-step High Fidelity buffer | 5 | 1x | |
| * | PrimeScript II RT Enzyme mix (50x → x 1/1600) | 0.2 | 1/1600x | #1 |
| * | (12.5x) PrimeSTAR GXL for 1 step RT-PCR | 0.8 | 1x | #1 |
| | RT primer (0.5nM) | 0.4 | 0.02nM | |
| | CleanAmp™ Precision Primers (10μM) | 0.4 | 0.4 μM | |
| | Template switch oligo (10μM) | 0.4 | 0.4 μM | |
| | SS 4 (50U/μl) | 0.5 | 2.5 U/μl | |
| | Rnasein RNaseInhibitor | 0.1 | 0.4 U/μl | |
| | Rnase inhibitor (cloned) | 0.1 | 0.4 U/μl | |
| | SUPERase inhibitor | 0.1 | 0.2 U/μl | |
| | Total RNA | 0.5 | 33.75pg/μl | |
| | $H_2O$ | 1.5 | | |
| | Total | 10 | | |

* is included in the PrimerScript II HighFidelity Pnesep RT PCT Kit
#1: final amount in the manual is ×1.

Typically, the following thermal cycling conditions were used.

[Table 3]

| 45°C | 95°C | 98°C | 60°C | 60°C | 4°C |
|---|---|---|---|---|---|
| 0:30:00 | 0:05:00 | 0:00:10 | 0:00:06 | 0:05:00 | ∞ |
| 1 cycle | | 44 cycle | | 1 cycle | |
| **Ramp Rate 6°C/s | | | | | |

The composition of a reaction solution and thermal cycling conditions were partially changed when appropriate depending on the test.

[Test Example 1]

One-step reverse transcription template switching PCR was performed under the following total RNA concentration, block primer concentration, and RT primer concentration conditions.

[Table 4]

| | Total RNA conc. (pg/μl) | Block primer (μM) | RT primer (μM) |
|---|---|---|---|
| 1 | 34 | 0 | 0.4 |
| 2 | 34 | 0.4 | 0.04 |
| 3 | 34 | 0.4 | 0.01 |
| 4 | 34 | 0.4 | 0.0025 |
| 5 | 34 | 0.4 | 0.00063 |
| 6 | 34 | 0,4 | 0.00016 |
| 7 | 3.4 | 0.4 | 0.04 |
| 8 | 3,4 | 0.4 | 0.01 |
| 9 | 3.4 | 0.4 | 0.0025 |
| 10 | 3.4 | 0.4 | 0.00063 |
| 11 | 3.4 | 0.4 | 0.00016 |

The results are shown in Figure 1. When a block primer was not used, a large number of non-specific bands were detected (lane 1), but the non-specific bands disappeared by adding a block primer (lanes 2 to 11). Specific amplification of a TCRβ chain was observed at RT primer concentrations of 0.04 μM to 0.00016 μM. While minor non-specific bands were observed at relatively high RT primer concentrations (0.4 μM and the like), this was able to be suppressed by reducing the RT primer concentration.

[Test Example 2]

One-step reverse transcription template switching PCR was performed under the following cell count, block primer concentration, and RT primer concentration conditions. The number of PCR cycles was 48.

[Table 5]

| | Number of cells | Block primer (μM) | RT primer (μM) |
|---|---|---|---|
| 1 | 30 | 0.4 | 0.02 |
| 2 | 30 | 0.4 | 0.002 |
| 3 | 30 | 0.4 | 0 |

The results are shown in Figure 2. Specific amplification of a TCRβ chain was observed under any of the conditions. Specific amplification of a TCRβ chain was observed without adding an RT primer (lane 3). As a result of one-step reverse transcription template switching PCR under the same conditions as above by changing the cell count to 10 and the block primer to CleanAmp™ Turbo Primers (TriLink), specific amplification of a TCRβ chain was similarly observed.

[Test Example 3]

One-step reverse transcription template switching PCR was performed after directly adding a reaction solution to a single cell of mouse T cell in one step. The number of PCR cycles was 56.

The results are shown in Figure 3. Cells with only the full length of a TCRβ chain amplified, and cells with the full length and fragment of a TCRβ chain amplified were observed. Despite the high number of cycles at 56, non-specific amplification was not observed.

[Test Example 4]

The number of PCR amplification cycles was changed to various numbers (number of cycles: 38, 40, 42, and 44). The block primer concentration was 0.4 μm, and the RT primer concentration was 0.002 nM.

The results are shown in Figure 4 (lane 1: 38, lane 2: 40, lane 3: 42, and lane 4: 44). A specific band of a TCRβ

chain was observed at 44 cycles.

[Test Example 5]

One-step reverse transcription template switching PCR was performed under the following total RNA concentration, block primer concentration, and RT primer concentration conditions. The number of PCR cycles was 42.

[Table 6]

| | Total RNA conc. (pg/μl) | Block primer (μM) | RT primer |
|---|---|---|---|
| 1 | 3.4 | 0 | 0.4 μM |
| 2 | 3.4 | 0.2 | 0.2 μM |
| 3 | 3.4 | 0.4 | 0.02 μM |
| 4 | 3.4 | 0.4 | 2 nM |
| 5 | 3.4 | 0.4 | 0.2 nM |
| 6 | 3.4 | 0.4 | 0.02 nM |
| 7 | 3.4 | 0.4 | 2 pM |
| 8 | 3.4 | 0.4 | 0.2 pM |
| 9 | 3.4 | 0.4 | 0.02 pM |
| 10 | 3.4 | 0.4 | 0 |

The results are shown in Figure 5. When a block primer was not used, a large number of smeared non-specific bands were detected (lane 1), but the non-specific bands disappeared by adding a block primer (lanes 2 to 10). While minor non-specific bands remained at a relatively high RT primer concentration (0.2 μM), this was able to be suppressed by reducing the RT primer concentration. Specific amplification of a TCRβ chain was observed without adding an RT primer (lane 10).

[Test Example 6]

Repertoire analysis of lymphoid cells derived from genetically modified mouse (Pmel-1 mouse) overexpressing a specific TCRα chain and TCRβ chain was performed using a one-step reverse transcription template switching PCR repertoire analysis method.

List of reagents

- PrimeScript II HighFidelity Onestep RT-PCR kit (R026A, Takara Bio)
- 40U/uL RNasin Plus RNase Inhibitor (N2611, Promega)
- 40 U/uL Cloned RNase Inhibitor (Ambion, AM2682)
- 20 U/uL SUPERaseIn RNase Inhibitor (Ambion, AM2694)
- 200U/uL SuperScript IV Reverse Transcriptase (18090010, Invitrogen)
- 2xKAPA HiFi Hot Start Ready Mix (KK2602, Nippon Genetics)
- AmpureXP Agencourt (A63880, BECKMAN COULTER)
- Qubit dsDNA HS assay kit (Q32854, Thermo Fisher Scientific)
  Oligonucleotide sequences that were used
- RT primer (30 bases): ACACGAGGGTAGCCTTTTGTTTGTTTGCAA (SEQ ID NO: 4)
- Block primer (30 bases): GAGGGTAGCCTTTTGTTTGTTTGCAATCTC (SEQ ID NO: 5)
- TS-Oligo (30 bases): AAGCAGTGGTATCAACGCAGAGTACAT[G][G](G)* (SEQ ID NO: 6)
  *2nd and 3rd bases from the right end in [ ] are RNA, and the base on the right end in () is an LNA
- Forward Tag primer v1 (63 bases): <u>GTCTCGTGGGCTCGGAGATGTGTATAAGAGACAG</u>AAGCAGTGGTATCAACGCAGAGTAC ATGGG (underlined portion is the tag sequence site) (SEQ ID NO: 7)
- Reverse Tag primer v1 (63 bases): <u>TCGTCGGCAGCGTCAGATGTGTATAAGAGACAG</u>GAGGGTAGCCTTTT-

GTTTGTTTGCAA TCTC (underlined portion is the tag sequence site) (SEQ ID NO: 8)

- Index primer: selected from 12 N7 series (SEQ ID NO: 9 to 20) and 8 S5 series (SEQ ID NO: 21 to 28) of Nextera XT Index Kit v2 Set A (Illumina)

The index sequences in the N7 series primers are the following.
TCGCCTTA (N701), CTAGTACG (N702), TTCTGCCT (N703), GCTCAGGA (N704), AGGAGTCC (N705), CATGCCTA (N706), GTAGAGAG (N707), CAGCCTCG (N710), TGCCTCTT (N711), TCCTCTAC (N712), TCATGAGC (N714), CCTGAGAT (N715)

The index sequences in the S5 series primers are the following.
CTCTCTAT (S502), TATCCTCT (S503), GTAAGGAG (S505), ACTGCATA (S506), AAGGAGTA (S507), CTAAGCCT (S508), CGTCTAAT (S510), TCTCTCCG (S511)

This test used a combination of indices of N715-S505 and N715-S506.

(1) RNA Preparation

This test extracted a total RNA from lymphoid cells derived from a genetically modified mouse (Pmel-1 mouse) overexpressing a specific TCRα chain and TCRβ chain.

(2) One-step reverse transcription template switching PCR

A heating reaction-PCR cycle was continuously performed using extracted RNA with a reaction solution having the following composition. First, a reverse transcription reaction for 30 minutes at 45°C, subsequently a block primer activation reaction for 5 minutes at 95° C, next a two-step PCR reaction for 10 seconds at 98° C and for 6 second at 60° C (44 cycles), and lastly a final extension reaction for 5 minutes at 60° C were performed to complete a one-step reverse transcription template switching PCR reaction.

[Table 7]

Table 7 Composition of one-step reverse transcription template switching PCR reaction solution

| Reagent | Volume (μL) |
|---|---|
| 2x one-step high fidelity buffer | 5.0 |
| 12.5x PrimeSTAR GXL for 1 step RT-PCR | 0.8 |
| RT primer (5 nM) | 0.4 |
| Block Primer (lOuM) | 0.4 |
| TS-Oligo (10uM) | 0.4 |
| SuperScript IV (50U/ul) | 0.5 |
| RNasin Plus RNase Inhibitor (40U/ul) | 0.1 |
| RNase Inhibitor (Cloned) (40 U/ul) | 0.1 |
| SUPERaseIn RNase Inhibitor (20 U/ul) | 0.1 |
| Total RNA (0.5 ng/ul) | 1.0 |
| DW | 1.2 |
| total | 10.0 |
| *Heating reaction-PCR cycle | |

30 minutes at 45°C (reverse transcription reaction)
↓

5 minutes at 95°C (block primer activation reaction) ↓

10 seconds at 98° C

6 second at 60°C (44 cycles) (PCR reaction)
↓
5 minutes at 60° C (final extension reaction)
↓
4° C stock

(3) Tag PCR

A tag added TCR cDNA amplicon was prepared by a three step PCR reaction (20 cycles, with final extension reaction for 2 minutes at 72° C after the final cycle) for 3 minutes at 95° C, 20 seconds at 98° C, 30 seconds at 65° C, and 2 minutes at 72° C using a DNA primer pair to which tag sequences of 34 bases and 33 bases were added to each of the 5' terminuses of a reverse primer and a forward primer (a pair of Forward Tag primer v1 and Reverse Tag primer v1) for adding a tag sequence for a Miseq sequencing run with a reaction solution having the following composition. A tag sequence is a sequence that is required for sequencing by Miseq. Sequencing is initiated from a tag sequence. A tag sequence also functions as a priming site of index PCR for adding an index sequence.

[Table 8]

Table 8 Composition of tag PCR reaction solution

| Reagent | Volume (uL) |
|---|---|
| One step RT-TS-PCR product | 3.0 |
| 2×KAPA HiFi Hot Start Ready Mix | 10.0 |
| Tag primer (Forward) 10uM | 0.4 |
| Tag primer (Reverse) 10uM | 0.4 |
| DW | 6.2 |
| Total | 20.0 |
| *PCR cycle | |

3 minutes at 95° C
↓
20 seconds at 98° C
30 second at 65°C (20 cycles)
2 minutes at 72° C
↓
2 minutes at 72° C
↓
12° C stock

(4) Index PCR

A PCR reaction for adding an index sequence for sample classification after a Miseq sequencing run and a sequence for immobilization to an analysis substrate, flow cell, at the most 5' terminus side was performed with a reaction solution having the following composition. A DNA sample for a sequencing run was prepared by a reaction for 3 minutes at 95° C, and then a three step PCR reaction (13 cycles, with final extension reaction for 5 minutes at 72°C after the final cycle) for 30 seconds at 95°C, 30 seconds at 55° C, and 30 seconds at 72° C.

[Table 9]

Table 9

| Reagent | Volume(uL) |
|---|---|
| Tag PCR product | 2.0 |
| 2×KAPA HiFi Hot Start Ready Mix | 10.0 |
| N primer(Nextera XT Index Kit v2) | 2.0 |
| S primer(Nextera XT Index Kit v2) | 2.0 |
| DW | 4.0 |
| Total | 20.0 |
| *PCR cycle | |

3 minutes at 95° C
↓
20 seconds at 98° C
30 second at 65° C (13 cycles)
2 minutes at 72° C
↓
2 minutes at 72° C
↓
4° C stock

(5) Confirmation by electrophoresis of index PCR reaction solution

Electrophoresis was performed for 30 minutes on 3 μl of an index PCR reaction solution in 1.5% agarose gel to confirm that the TCR DNA amplicons were amplified in the experimental steps up to this point (Figure **6**).

(6) Purification, concentration measurement, and concentration measurement of DNA fragments

10 μl of a reaction solution was dispensed, 8 μl of AmpureXP Agencourt beads solution was added, a reaction to bind DNA to the beads was performed in accordance with the protocol of the manufacturer, the beads were washed, and then purified DNA was collected in 25 μl of pure water. The concentration of purified DNA was measured with a Qubit dsDNA HS assay kit.

(7) Conducting a Miseq run

After diluting a purified DNA sample to 4 nM, a sequencing run was conducted in accordance with the standard protocol of Illumina's Miseq sequencer.

(8) Repertoire data analysis

Sequencing data in a Fastq format was analyzed with the Repertoire Genesis software. Usage frequency graphs for V genes and J genes in a sample (Figure **7**) and V-J usage frequency graph (Figure **8**) were obtained. Tabulation of the unique read ranking including CDR3 sequences (SEQ ID NOs: 29 to 33) is shown in Table 10.

[Table 10]

Table 10 (Tabulation of unique read ranking including CDR3 sequences)

| Rank | TRBV | TRBJ | CDR3 | Reads | %Reads |
|---|---|---|---|---|---|
| 1 | TRBV14 | TRBJ1-6 | CAS SFHRDYNS PLYF | 248.347 | 92.53 |
| 2 | TRBV14 | TRBJ1-6 | CAS SFHRDYNS PLYL | 560 | 0.21 |
| 3 | TRBV14 | TRBJ1-6 | WAS SFHRDYNS PLYF | 511 | 0.19 |
| 4 | TRBV14 | TRBJ1-6 | CASSFHRDYNAPLYF | 470 | 0.18 |
| 5 | TRBV14 | TRBJ1-6 | CAS SVHRDYNS PLYF | 409 | 0.15 |

[Test Example 7]

One-step RT-TS-PCR repertoire analysis was conducted using a C57BL/6 mouse spleen tissue specimen.
Repertoire analysis was conducted on the spleens using a one-step reverse transcription template switching PCR repertoire analysis method. As the specimens subjected to the experiment, a C57BL/6 mouse spleen tissue specimen and lymphoid cells derived from the Pmel-1 mouse that were collected 48 hours after the stimulation with an anti-CD3 antibody and an anti-CD28 antibody were used.

List of reagents

- PrimeScript II HighFidelity Onestep RT-PCR kit (R026A, Takara Bio)
- 40U/uL RNasin Plus RNase Inhibitor (N2611, Promega)
- 40 U/uL Cloned RNase Inhibitor (Ambion, AM2682)
- 20 U/uL SUPERaseIn RNase Inhibitor (Ambion, AM2694)
- 200U/uL SuperScript IV Reverse Transcriptase (18090010, Invitrogen)
- 2×KAPA HiFi Hot Start Ready Mix (KK2602, Nippon Genetics)
- AmpureXP Agencourt (A63880, BECKMAN COULTER)
- Qubit dsDNA HS assay kit (Q32854, Thermo Fisher Scientific)
  Oligonucleotide sequences that were used
- RT primer (30 bases): ACACGAGGGTAGCCTTTTGTTTGTTTGCAA (SEQ ID NO: 4)
- Block primer (30 bases): GAGGGTAGCCTTTTGTTTGTTTGCAATCTC (SEQ ID NO: 5)
- TS-Oligo (30 bases): AAGCAGTGGTATCAACGCAGAGTACAT[G][G](G)*(SEQ ID NO: 6)
  *2nd and 3rd bases from the right end in [ ] are RNA, and the base on the right end in () is an LNA
- Forward Tag primer v1 (63 bases): <u>GTCTCGTGGGCTCGGAGATGTGTATAAGAGACAG</u>AAGCAGTGGTATCAACGCAGAGTAC ATGGG (underlined portion is the tag sequence site) (SEQ ID NO: 7)
- Reverse Tag primer v1 (63 bases): <u>TCGTCGGCAGCGTCAGATGTGTATAAGAGACAG</u>GAGGGTAGCCTTTTGTTTGTTTGCAA TCTC (underlined portion is the tag sequence site) (SEQ ID NO: 8)
- Index primer: selected from 12 N7 series (SEQ ID NO: 9 to 20) and 8 S5 series (SEQ ID NO: 21 to 28) of Nextera XT Index Kit v2 Set A (Illumina)

The index sequences in the N7 series primers are the following.
TCGCCTTA (N701), CTAGTACG (N702), TTCTGCCT (N703), GCTCAGGA (N704), AGGAGTCC (N705), CATGCCTA (N706), GTAGAGAG (N707), CAGCCTCG (N710), TGCCTCTT (N711), TCCTCTAC (N712), TCATGAGC (N714), CCTGAGAT (N715)
The index sequences in the S5 series primers are the following.
CTCTCTAT (S502), TATCCTCT (S503), GTAAGGAG (S505), ACTGCATA (S506), AAGGAGTA (S507), CTAAGCCT (S508), CGTCTAAT (S510), TCTCTCCG (S511).
This test used a combination of indices of N715-S502, S503, S505, S506, S507, S508, S510, or S511.

Experimental procedure

(1) RNA preparation

RNA was extracted from spleen tissue and lymphoid cells for TCRβ repertoire analysis in this test.

(2) One-step reverse transcription template switching PCR

A heating reaction-PCR cycle was continuously performed using extracted RNA with a reaction solution having the following composition. First, a reverse transcription reaction for 30 minutes at 45° C, subsequently a block primer activation reaction for 5 minutes at 95° C, next a two-step PCR reaction for 10 seconds at 98° C and for 6 second at 60° C (44 cycles), and lastly a final extension reaction for 5 minutes at 60° C were performed to complete a one-step reverse transcription template switching PCR reaction.

[Table 11]

Table 11 Composition of one-step reverse transcription template switching PCR reaction solution

| Reagent | Volume(uL) |
|---|---|
| 2x one-step high fidelity buffer | 5.0 |
| 12.5x PrimeSTAR GXL for 1 step RT-PCR | 0.8 |
| RT primer (5 nM) | 0.4 |
| Block Primer (10uM) | 0.4 |
| TS-Oligo (10uM) | 0.4 |
| SuperScript IV (50U/ul) | 0.5 |
| RNasin Plus RNase Inhibitor (40U/ul) | 0.1 |
| RNase Inhibitor (Cloned) (40 U/ul) | 0.1 |
| SUPERaseIn RNase Inhibitor (20 U/ul) | 0.1 |
| Total RNA (0.5 ng/ul) | 1.0 |
| DW | 1.2 |
| total | 10.0 |
| *Heating reaction-PCR cycle | |

30 minutes at 45° C (reverse transcription reaction)
↓

5 minutes at 95° C (block primer activation)
↓

10 seconds at 98° C

6 second at 60° C (44 cycles) (PCR reaction)
↓

5 minutes at 60°C (final extension reaction)
↓

4° C stock

(3) Tag PCR

A tag added TCR cDNA amplicon was prepared by a three step PCR reaction (20 cycles, with final extension reaction for 2 minutes at 72° C after the final cycle) for 3 minutes at 95° C, 20 seconds at 98° C, 30 seconds at 65° C, and 2 minutes at 72° C using a DNA primer pair to which tag sequences of 34 bases and 33 bases were added to each of the 5' terminuses of a reverse primer and a forward primer (a pair of Forward Tag primer v1 and Reverse Tag primer v1) for adding a tag sequence for a Miseq sequencing run with a reaction solution having the following composition. A

tag sequence is a sequence that is required for sequencing by Miseq. Sequencing is initiated from a tag sequence. A tag sequence also functions as a priming site of index PCR for adding an index sequence.

[Table 12]

Table 12 Composition of tag PCR reaction solution

| Reagent | Volume (uL) |
|---|---|
| One step RT-TS-PCR product | 3.0 |
| 2×KAPA HiFi Hot Start Ready Mix | 10.0 |
| Tag primer (Forward) 10uM | 0.4 |
| Tag primer (Reverse) 10uM | 0.4 |
| DW | 6.2 |
| Total | 20.0 |
| *PCR cycle | |

3 minutes at 95°C
↓
20 seconds at 98° C
30 second at 65° C (20 cycles)
2 minutes at 72° C
1
2 minutes at 72° C
↓
12° C stock

(4) Index PCR

A PCR reaction for adding an index sequence for sample classification after a Miseq sequencing run and a sequence for immobilization to an analysis substrate, flow cell, at the most 5' terminus side (sequence for bridge PCR) was performed with a reaction solution having the following composition. A DNA sample for a sequencing run was prepared by a reaction for 3 minutes at 95°C, and then a three step PCR reaction (13 cycles, with final extension reaction for 5 minutes at 72° C after the final cycle) for 30 seconds at 95°C, 30 seconds at 55° C, and 30 seconds at 72°C.

[Table 13]

Table 13

| Reagent | Volume (uL) |
|---|---|
| Tag PCR product | 2.0 |
| 2×KAPA HiFi Hot Start Ready Mix | 10.0 |
| N primer (Nextera XT Index Kit v2) | 2.0 |
| S primer (Nextera XT Index Kit v2) | 2.0 |
| DW | 4.0 |
| Total | 20.0 |
| *PCR cycle | |

3 minutes at 95° C

↓
20 seconds at 98° C
30 second at 65°C (13 cycles)
2 minutes at 72°C
↓
2 minutes at 72° C
↓
4° C stock

(5) Confirmation by electrophoresis of index PCR reaction solution

Electrophoresis was performed for 30 minutes on 3 μl of an index PCR reaction solution in 1.5% agarose gel to confirm that the TCR DNA amplicons were amplified in the experimental steps up to this point (Figure **9**). The lanes in Figure **9** indicate, from the left in order, 1: marker DNA, 2 to 6: mouse spleen tissue (1000 ng, 200 ng, 40 ng, 8 ng, and 1.6 ng), 7 to 8: Pmel-1 derived lymphocytes (8 ng and 1.6 ng), and 9: blank.

(6) Purification, concentration measurement, and concentration measurement of DNA fragments

10 μl of a reaction solution was dispensed, 8 μl of AmpureXP Agencourt beads solution was added, a reaction to bind DNA to the beads was performed in accordance with the protocol of the manufacturer, the beads were washed, and then purified DNA was collected in 25 μl of pure water. The concentration of purified DNA was measured with a Qubit dsDNA HS assay kit.

(7) Conducting a Miseq run

After diluting a purified DNA sample to 4 nM, a sequencing run was conducted in accordance with the standard protocol of Illumina's Miseq sequencer.

(8) Repertoire data analysis

Sequencing data in a Fastq format obtained in spleen tissue specimens was analyzed with the Repertoire Genesis software. Usage frequency graphs for V genes and J genes in a sample (Figure **10**) and V-J usage frequency graph (Figure 11) were obtained. Tabulation of the unique read ranking including CDR3 sequences (SEQ ID NOs: 34 to 38) is shown in Table 14.

[Table 14]

Table 14 (Tabulation of unique read ranking including CDR3 sequences)

| Rank | TRBV | TRBJ | CDR3 | Reads | %Reads |
|---|---|---|---|---|---|
| 1 | TRBV13-1 | TRBJ2-5 | CASSDVQDTQYF | 833 | 4.72 |
| 2 | TRBV13-1 | TRBJ2-4 | CASSEDWGVQNTLYF | 241 | 1.37 |
| 3 | TRBV20 | TRBJ1-5 | CGALRDRNQAPLF | 147 | 0.83 |
| 4 | TRBV16 | TRBJ1-1 | CASSLPGGDTEVFF | 142 | 0.81 |
| 5 | TRBV13-2 | TRBJ1-3 | CASGDAPDRLGNTLYF | 138 | 0.78 |

While the present invention has been explained while emphasizing the preferred embodiments, it is evident to those skilled in the art that the preferred embodiment can be modified. The present invention is intended to be practicable by a method other than those described in detail herein. Therefore, the present invention includes all modifications that are encompassed within the spirit and scope of the appended "Claims".

The content described in all of the publications including the patents and patent applications discussed herein are incorporated herein by reference to the same extent that the entirety thereof is explicitly described herein. The present invention claims priority to Japanese Patent Application No. 2016-125007 filed on June 23, 2016, which is

incorporated herein to the same extent that the entirety thereof is explicitly described herein.

[Industrial Applicability]

The present invention is expected to perform reverse transcription template switching PCR with high specificity in one step. In particular, even if the number of copies of a template RNA is low and the number of PCR cycles is high, a specific PCR product can be expected to be amplified while suppressing side reactions. Specifically amplified nucleic acid samples are provided by utilizing such a reverse transcription template switching PCR technology, which is especially useful in clinical application settings that particularly require quantitative analysis.

## Claims

1. A method of analyzing a repertoire of variable regions of T cell receptors (TCR) or B cell receptors (BCR) of a subject, comprising the steps of:

   (1) providing a nucleic acid sample comprising nucleic acid sequences of a plurality of types of T cell receptors

(TCR) or B cell receptors (BCR) amplified from an RNA obtained from the subject;
(2) determining the nucleic acid sequences contained in the nucleic acid sample; and
(3) calculating a frequency of appearance of each gene or a combination thereof based on the determined nucleic acid sequences to derive a TCR or BCR repertoire of the subject;

wherein step (1) comprises the steps of:

a) mixing an RNA obtained from the subject, a reagent required for reverse transcription, a reagent required for template switching, and a reagent required for a polymerase chain reaction and subjecting the mixture to a condition under which reverse transcription occurs to provide a cDNA comprising nucleic acid sequences of a plurality of types of T cell receptors (TCR) or B cell receptors (BCR); and
b) subjecting the cDNA obtained in step a) to a condition under which a polymerase chain reaction occurs to provide the nucleic acid sample comprising nucleic acid sequences of a plurality of types of T cell receptors (TCR) or B cell receptors (BCR);

wherein the reagent required for template switching comprises a template switching oligonucleotide, and
wherein the regent required for a polymerase chain reaction comprises a primer specific to a C region of the TCR or the BCR, wherein the primer specific to a C region is a modified oligonucleotide primer designed to have a primer function that is partially or completely blocked in step a) and designed to have blocking of the primer function cleared in step b).

2. A method of producing a nucleic acid sample for analyzing a repertoire of variable regions of T cell receptors (TCR) or B cell receptors (BCR) of a subject, the method comprising the step of (1) providing a nucleic acid sample comprising a nucleic acid sequence of a plurality of types of T cell receptors (TCR) or B cell receptors (BCR) amplified from an RNA obtained from the subject, step (1) comprising the steps of:

a) mixing an RNA obtained from the subject, a reagent required for reverse transcription, a reagent required for template switching, and a reagent required for a polymerase chain reaction and subjecting the mixture to a condition under which reverse transcription occurs to provide a cDNA comprising nucleic acid sequences of a plurality of types of T cell receptors (TCR) or B cell receptors (BCR); and
b) subjecting the cDNA obtained in step a) to a condition under which a polymerase chain reaction occurs to provide the nucleic acid sample comprising a nucleic acid sequence of a plurality of types of T cell receptors (TCR) or B cell receptors (BCR);

wherein the reagent required for template switching comprises a template switching oligonucleotide, and
wherein the regent required for a polymerase chain reaction comprises a primer specific to a C region of the TCR or the BCR, wherein the primer specific to a C region is a modified oligonucleotide primer designed to have a primer function that is partially or completely blocked in step a) and designed to have blocking of the primer function cleared in step b).

3. The method of claim 1 or 2, wherein the nucleic acid sample is a nucleic acid sample that has been amplified in an unbiased manner.

4. The method of any one of claims 1 to 3, wherein the reagent required for a polymerase chain reaction optionally further comprises a 5' anchor oligonucleotide primer comprising at least a part of an anchor sequence comprised in the template switching oligonucleotide.

5. The method of claim 4, wherein the reagent required for a polymerase chain reaction does not comprise the 5' anchor oligonucleotide primer, and the template switching oligonucleotide functions as a 5' anchor oligonucleotide primer.

6. The method of any one of claims 1 to 5, wherein the reagent required for reverse transcription comprises an oligonucleotide primer that initiates reverse transcription, and the oligonucleotide primer that initiates reverse transcription is comprised in the mixture at a final concentration of about 40 nM or less, or at a mole ratio of about 1:10 or less relative to the modified oligonucleotide primer.

7. The method of any one of claims 1 to 6, wherein the modified oligonucleotide primer has one or more complementary regions on a sequence of the same modified oligonucleotide primer, and has a turn structure by the complementary

regions or comprises a thermolabile modifying group before initial thermal denaturation of PCR.

**8.** The method of any one of claims 1 to 7, wherein a part of the modified oligonucleotide, whose primer function has not been blocked, functions as an oligonucleotide primer that initiates reverse transcription by hybridizing to a template RNA.

**9.** A kit for amplifying a variable region of a T cell receptor (TCR) or a B cell receptor (BCR), the kit comprising:

i) a reagent required for reverse transcription;
ii) a reagent required for template switching;
iii) a reagent required for a polymerase chain reaction using a modified oligonucleotide primer; and
iv) optionally a user manual;

**characterized in that** the reagents of i) to iii) and the modified oligonucleotide primer are all mixed in a reaction system as of the initiation of a reaction,
wherein the reagent of ii) comprises a template switching oligonucleotide, and
wherein the modified oligonucleotide primer is a primer specific to a C region of the TCR or the BCR which is designed to have a primer function that is partially or completely blocked under a condition where reverse transcription occurs and designed to have blocking of the primer function cleared under a condition where a polymerase chain reaction occurs.

**10.** The kit of claim 9, wherein the reagent required for a polymerase chain reaction comprises a 5' anchor oligonucleotide primer comprising at least a part of an anchor sequence comprised in the template switching oligonucleotide.

**11.** The kit of claim 10, wherein the reagent required for a polymerase chain reaction does not comprise the 5' anchor oligonucleotide primer.

**12.** The kit of any one of claims 9 to 11, **characterized in that** the reagent required for reverse transcription comprises an oligonucleotide primer that initiates reverse transcription, and the oligonucleotide primer that initiates reverse transcription is used at a final concentration of about 40 nM or less, or at a mole ratio of about 1:10 or less relative to the modified oligonucleotide primer.

**13.** The kit of any one of claims 9 to 12, wherein the modified oligonucleotide primer has one or more complementary regions on a sequence of the same modified oligonucleotide primer, and has a turn structure by the complementary regions or comprises a thermolabile modifying group before initial thermal denaturation of PCR.

**14.** The kit of claim 13, wherein a part of the modified oligonucleotide whose primer function has not been blocked functions as an oligonucleotide primer that initiates reverse transcription by hybridizing to a partial sequence of a C region of a template RNA of the TCR or the BCR.

**15.** The kit of any one of claims 9 to 14 for providing a nucleic acid sample comprising nucleic acid sequences of a plurality of types of T cell receptors (TCR) or B cell receptors (BCR) amplified from an RNA obtained from the subject in an unbiased manner.

**16.** The method of claim 1, or claims 3 to 8 when dependent from claim 1, wherein step (1) further comprises a step of providing a nucleic acid sample to which a sequence that is suitable for sequence analysis is added.

**17.** A method of claim 16, wherein the sequence that is suitable for sequence analysis is a sequence that is suitable for sequence analysis used in bridge PCR or emulsion PCR.

**18.** The method of claim 16, wherein step (1) further comprises the following steps:

c) subjecting a mixture comprising a PCR amplicon of step b), a second 5' anchor oligonucleotide primer to which a first tag sequence is added, and a second primer specific to a C region of TCR or BCR to which a second tag sequence is added to a condition under which a polymerase chain reaction occurs to provide a nucleic acid sample comprising a nucleic acid sequence of a plurality of types of T cell receptors (TCR) or B cell receptors (BCR) to which a tag sequence is added; and
d) subjecting a mixture comprising a PCR amplicon of step c), a third 5' anchor oligonucleotide primer, and a

third primer specific to a C region of TCR or BCR to a condition under which a polymerization chain reaction occurs to provide the nucleic acid sample comprising a nucleic acid sequence of a plurality of types of T cell receptors (TCR) or B cell receptors (BCR) to which an index sequence is added, wherein an index sequence and a sequences for immobilization to a substrate of sequence analysis are added to the third 5' anchor oligonucleotide primer and the third primer specific to a C region of TCR or BCR.

**19.** The method of claim 18, wherein step (3) comprises the following steps:

(3-1) providing a reference database for each gene region comprising at least one of a V region, a D region, a J region, and optionally a C region;
(3-2) providing an input sequence set which is optionally trimmed and optionally extracted to have a suitable length;
(3-3) searching for homology of the input sequence set with the reference database for the each gene region and recording an alignment with an approximate reference allele and/or a sequence of the reference allele;
(3-4) assigning the V region and the J region for the input sequence set and extracting a nucleic acid sequence of the D region based on a result of assigning;
(3-5) translating the nucleic acid sequence of the D region into an amino acid sequence and classifying the D region by utilizing the amino acid sequence; and
(3-6) calculating a frequency of appearance for each of the V region, the D region, the J region and optionally the C region or a frequency of appearance of a combination thereof based on the classifying in step (3-5) to derive the TCR or BCR repertoire.

**20.** A system for quantitatively analyzing a repertoire of variable regions of T cell receptors (TCR) or B cell receptors (BCR) of a subject by using a database, the system comprising:

(1) the kit of claim 15;
(2) an apparatus for determining the nucleic acid sequence comprised in the nucleic acid sample; and
(3) an apparatus for calculating a frequency of appearance of each gene or a combination thereof based on the determined nucleic acid sequence to derive a TCR or BCR repertoire of the subject.

**21.** The system of claim 20, wherein (1) the kit further comprises:

c) means for subjecting a mixture comprising a PCR amplicon of step b), a second 5' anchor oligonucleotide primer to which a first tag sequence is added, and a second primer specific to a C region of TCR or BCR to which a second tag sequence is added to a condition under which a polymerase chain reaction occurs to provide a nucleic acid sample comprising a nucleic acid sequence of a plurality of types of T cell receptors (TCR) or B cell receptors (BCR) to which a tag sequence is added; and
d) means for subjecting a mixture comprising a PCR amplicon of step c), a third 5' anchor oligonucleotide primer, and a third primer specific to a C region of TCR or BCR to a condition under which a polymerization chain reaction occurs to provide the nucleic acid sample comprising a nucleic acid sequence of a plurality of types of T cell receptors (TCR) or B cell receptors (BCR) to which an index sequence is added, wherein an index sequence and a sequences for immobilization to a substrate of sequence analysis are added to the third 5' anchor oligonucleotide primer and the third primer specific to a C region of TCR or BCR.

**22.** The system of claims 20 or 21, wherein (3) the apparatus for deriving a TCR or BCR repertoire comprises:

(3-1) means for providing a reference database for each gene region comprising at least one of a V region, a D region, a J region, and optionally a C region;
(3-2) means for providing an input sequence set which is optionally trimmed and optionally extracted to have a suitable length;
(3-3) means for searching for homology of the input sequence set with the reference database for the each gene region and recording an alignment with an approximate reference allele and/or a sequence of the reference allele;
(3-4) means for assigning the V region and the J region for the input sequence set and extracting a nucleic acid sequence of the D region based on a result of assigning;
(3-5) means for translating the nucleic acid sequence of the D region into an amino acid sequence and classifying the D region by utilizing the amino acid sequence; and
(3-6) means for calculating a frequency of appearance for each of the V region, the D region, the J region, and

optionally the C region or a frequency of appearance of a combination thereof based on the classifying in step (3-5) to derive the TCR or BCR repertoire.

**23.** A system for analyzing a disease, disorder, or condition of a subject, comprising the system of any one of claims 20 to 22 and means for analyzing the disease, disorder, or condition of the subject based on a TCR or BCR repertoire derived based on the system.

**24.** A system for treating or preventing a disease, disorder, or condition of a subject, comprising: means for quantitatively associating the disease, disorder, or condition of the subject determined by the system of claim 23 with the TCR or BCR repertoire; and means for selecting means for suitable treatment or prevention from the quantitative association.

FIG.1

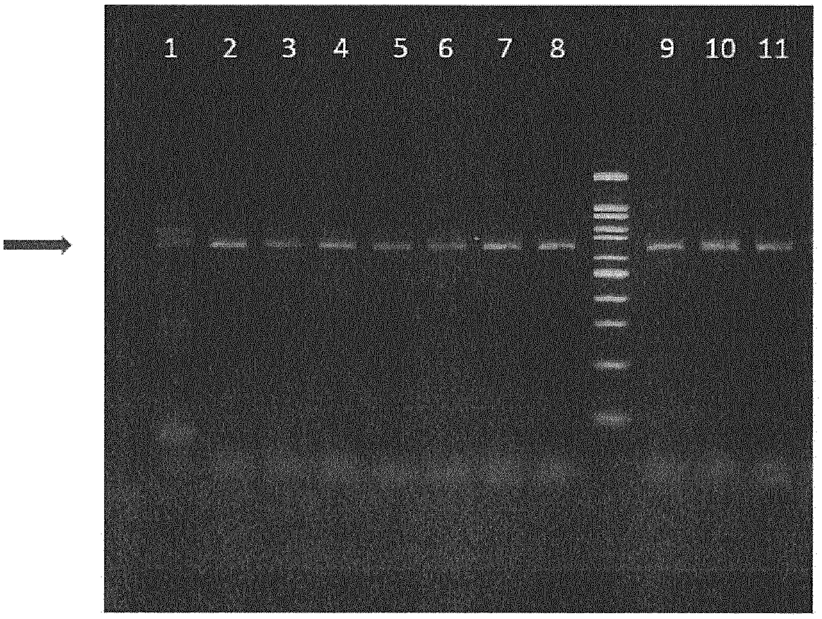


FIG.2

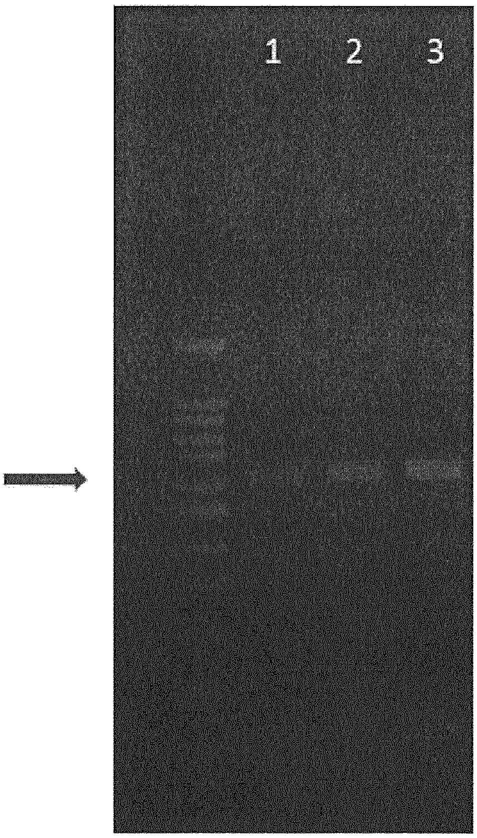

FIG.3

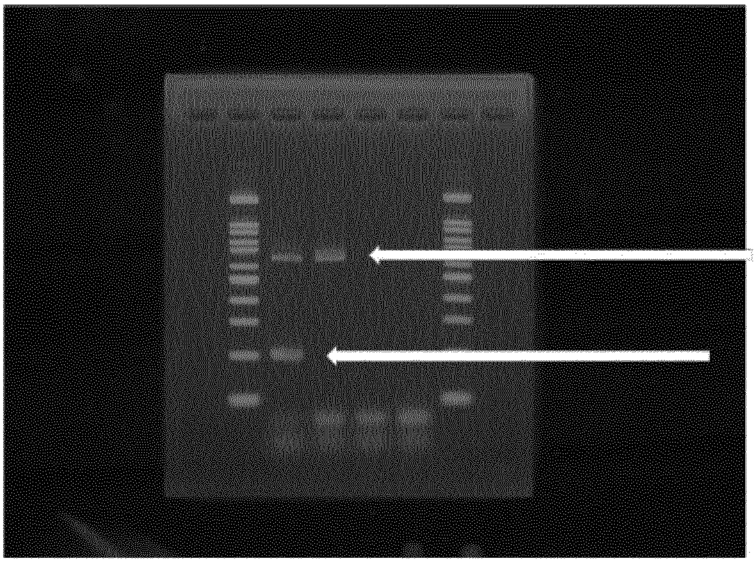

FIG.4

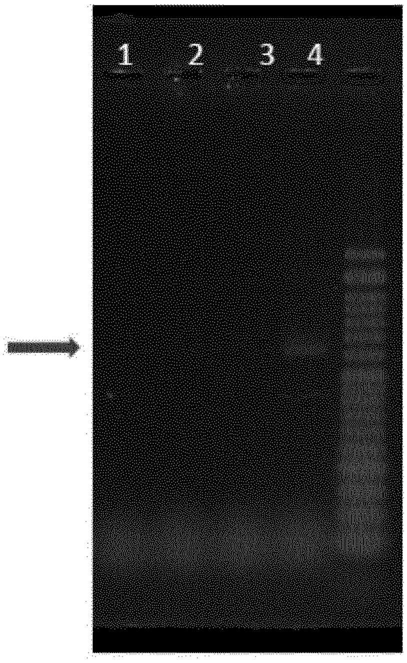

FIG.5

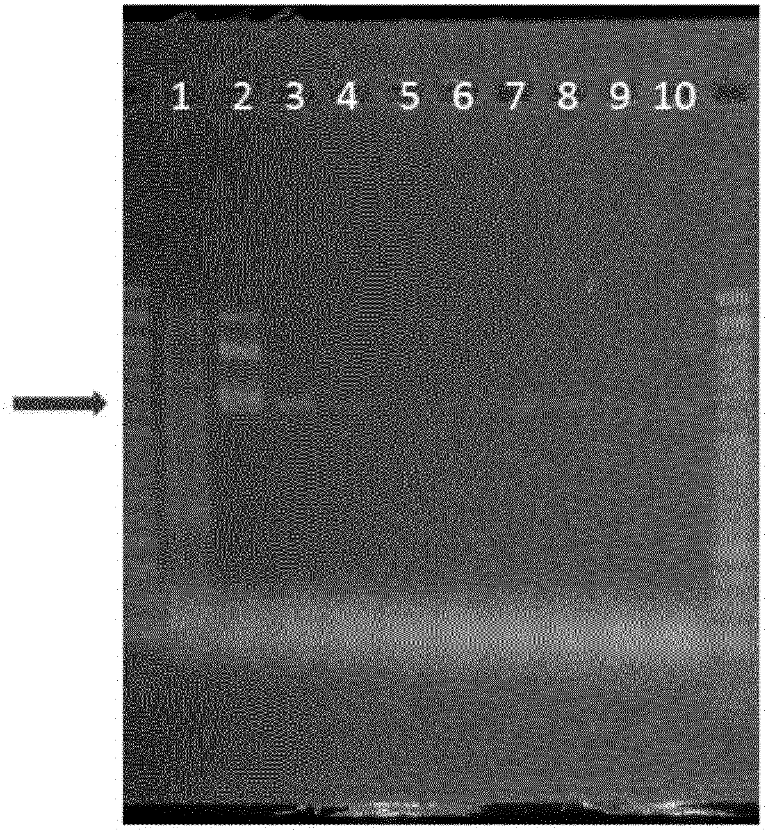


FIG.6

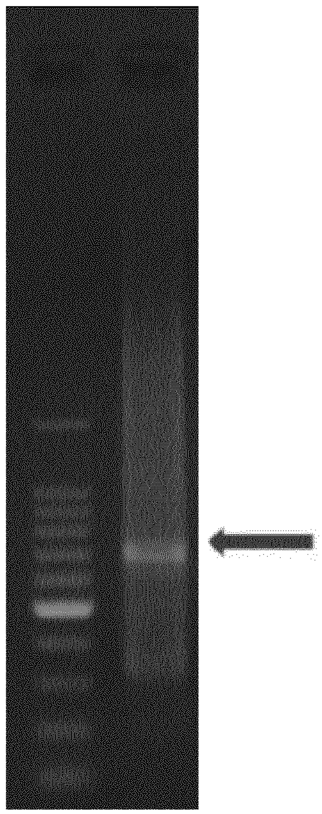

# Fig. 7

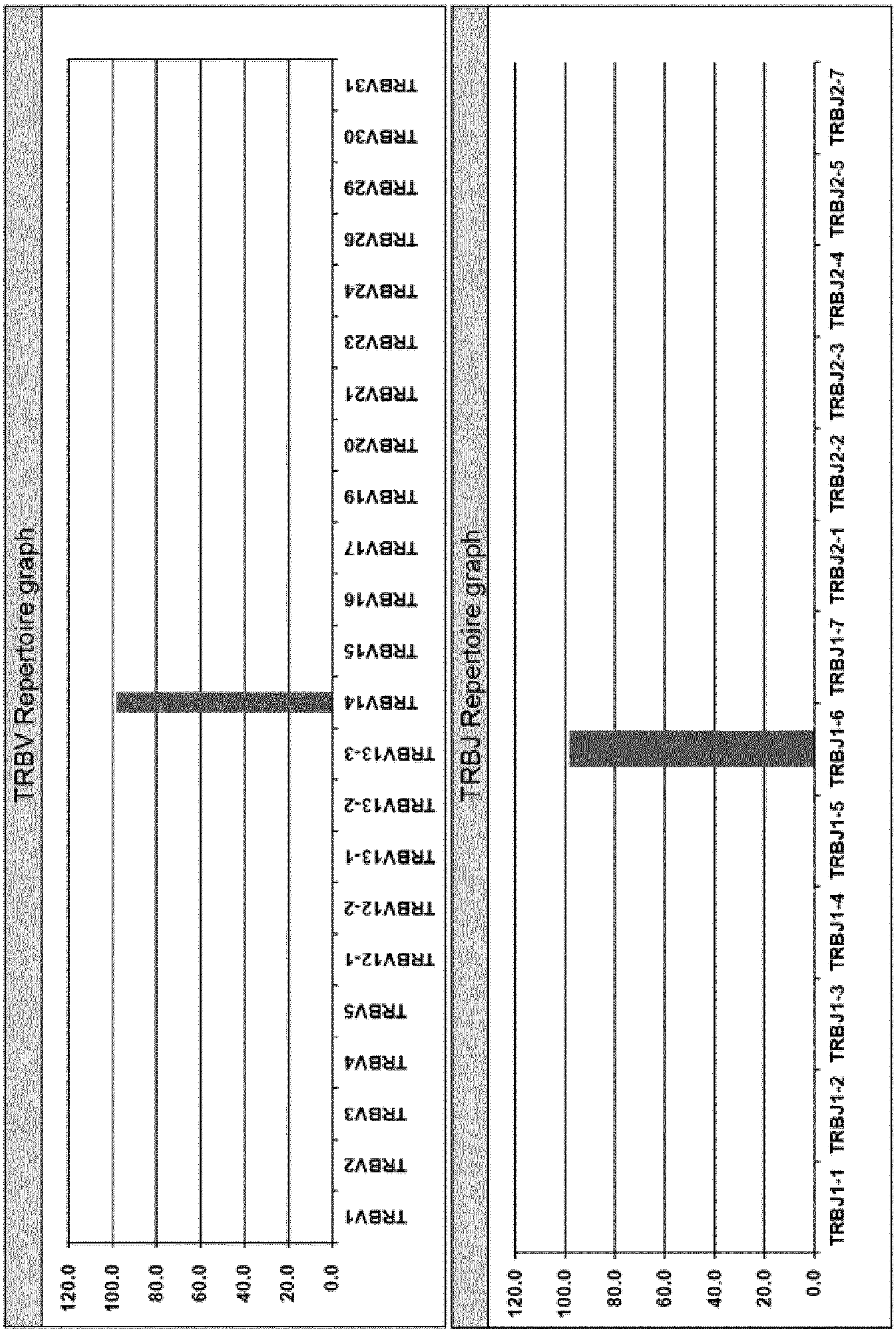
TRBV Repertoire graph
120.0
100.0
80.0
60.0
40.0
20.0
0.0
TRBV1
TRBV2
TRBV3
TRBV4
TRBV5
TRBV12-1
TRBV12-2
TRBV13-1
TRBV13-2
TRBV13-3
TRBV14
TRBV15
TRBV16
TRBV17
TRBV19
TRBV20
TRBV21
TRBV23
TRBV24
TRBV26
TRBV29
TRBV30
TRBV31
TRBJ Repertoire graph
120.0
100.0
80.0
60.0
40.0
20.0
0.0
TRBJ1-1
TRBJ1-2
TRBJ1-3
TRBJ1-4
TRBJ1-5
TRBJ1-6
TRBJ1-7
TRBJ2-1
TRBJ2-2
TRBJ2-3
TRBJ2-4
TRBJ2-5
TRBJ2-7

Fig. 8

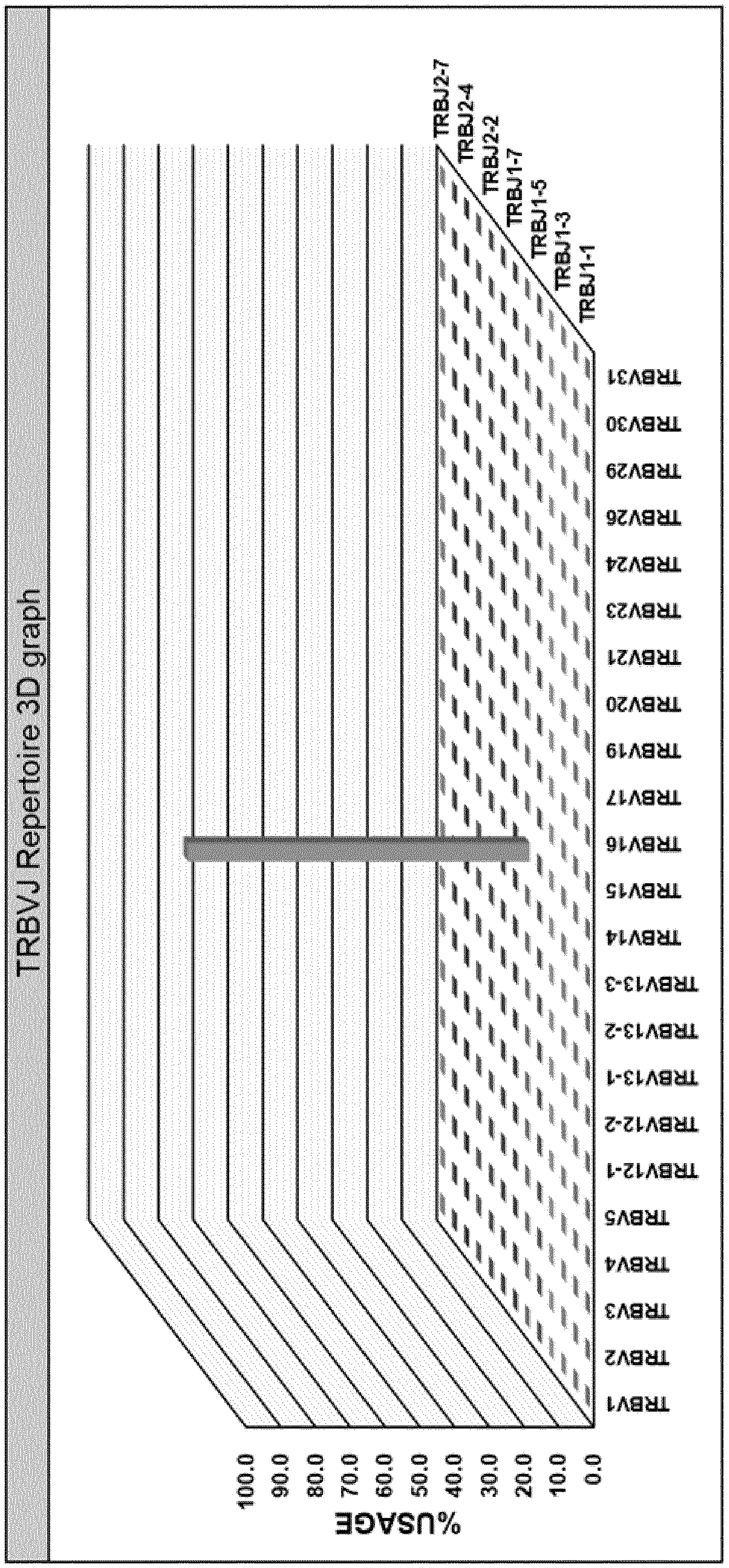
TRBVJ Repertoire 3D graph
%USAGE
100.0
90.0
80.0
70.0
60.0
50.0
40.0
30.0
20.0
10.0
0.0
TRBV1
TRBV2
TRBV3
TRBV4
TRBV5
TRBV12-1
TRBV12-2
TRBV13-1
TRBV13-2
TRBV13-3
TRBV14
TRBV15
TRBV16
TRBV17
TRBV19
TRBV20
TRBV21
TRBV23
TRBV24
TRBV26
TRBV29
TRBV30
TRBV31
TRBJ1-1
TRBJ1-3
TRBJ1-5
TRBJ1-7
TRBJ2-2
TRBJ2-4
TRBJ2-7

FIG.9

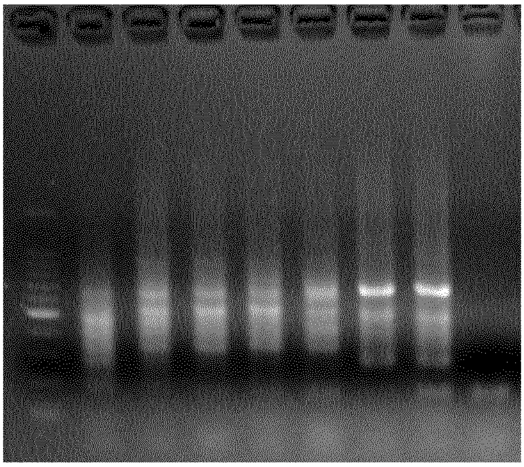

Fig. 10

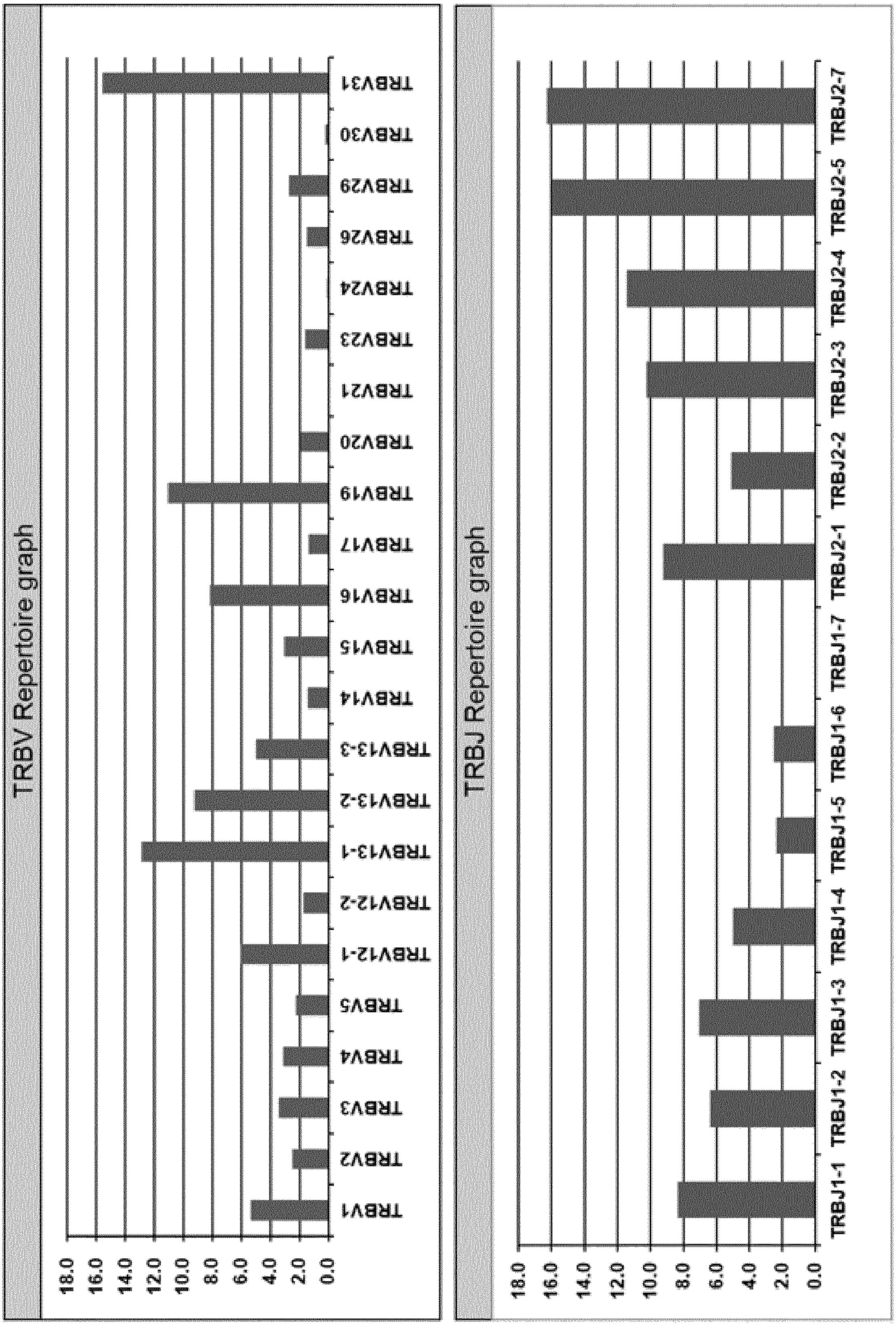
TRBV Repertoire graph
18.0
16.0
14.0
12.0
10.0
8.0
6.0
4.0
2.0
0.0
TRBV1
TRBV2
TRBV3
TRBV4
TRBV5
TRBV12-1
TRBV12-2
TRBV13-1
TRBV13-2
TRBV13-3
TRBV14
TRBV15
TRBV16
TRBV17
TRBV19
TRBV20
TRBV21
TRBV23
TRBV24
TRBV26
TRBV29
TRBV30
TRBV31
TRBJ Repertoire graph
18.0
16.0
14.0
12.0
10.0
8.0
6.0
4.0
2.0
0.0
TRBJ1-1
TRBJ1-2
TRBJ1-3
TRBJ1-4
TRBJ1-5
TRBJ1-6
TRBJ1-7
TRBJ2-1
TRBJ2-2
TRBJ2-3
TRBJ2-4
TRBJ2-5
TRBJ2-7

Fig. 11

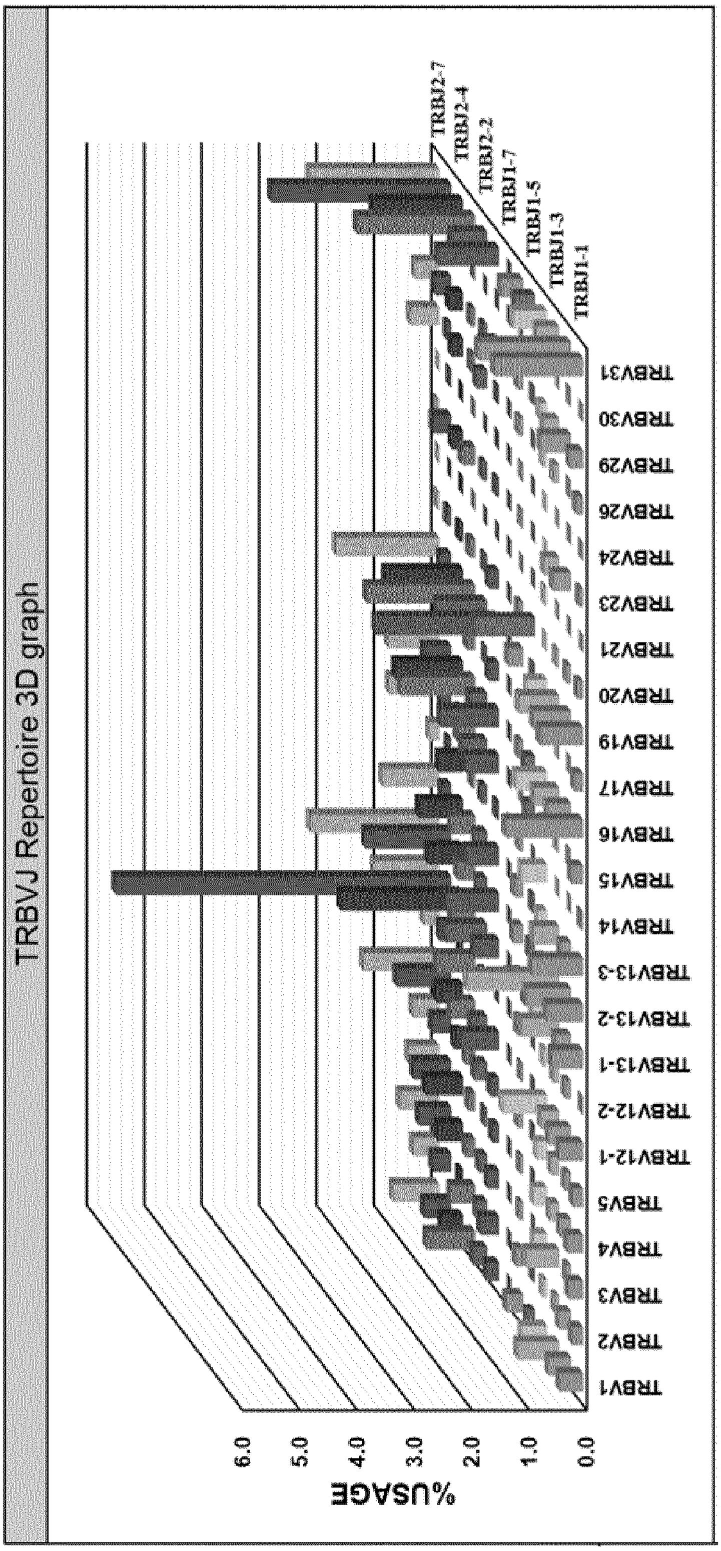
TRBVJ Repertoire 3D graph
%USAGE
6.0
5.0
4.0
3.0
2.0
1.0
0.0
TRBV1
TRBV2
TRBV3
TRBV4
TRBV5
TRBV12-1
TRBV12-2
TRBV13-1
TRBV13-2
TRBV13-3
TRBV14
TRBV15
TRBV16
TRBV17
TRBV19
TRBV20
TRBV21
TRBV23
TRBV24
TRBV26
TRBV29
TRBV30
TRBV31
TRBJ1-1
TRBJ1-3
TRBJ1-5
TRBJ1-7
TRBJ2-2
TRBJ2-4
TRBJ2-7

FIG.12

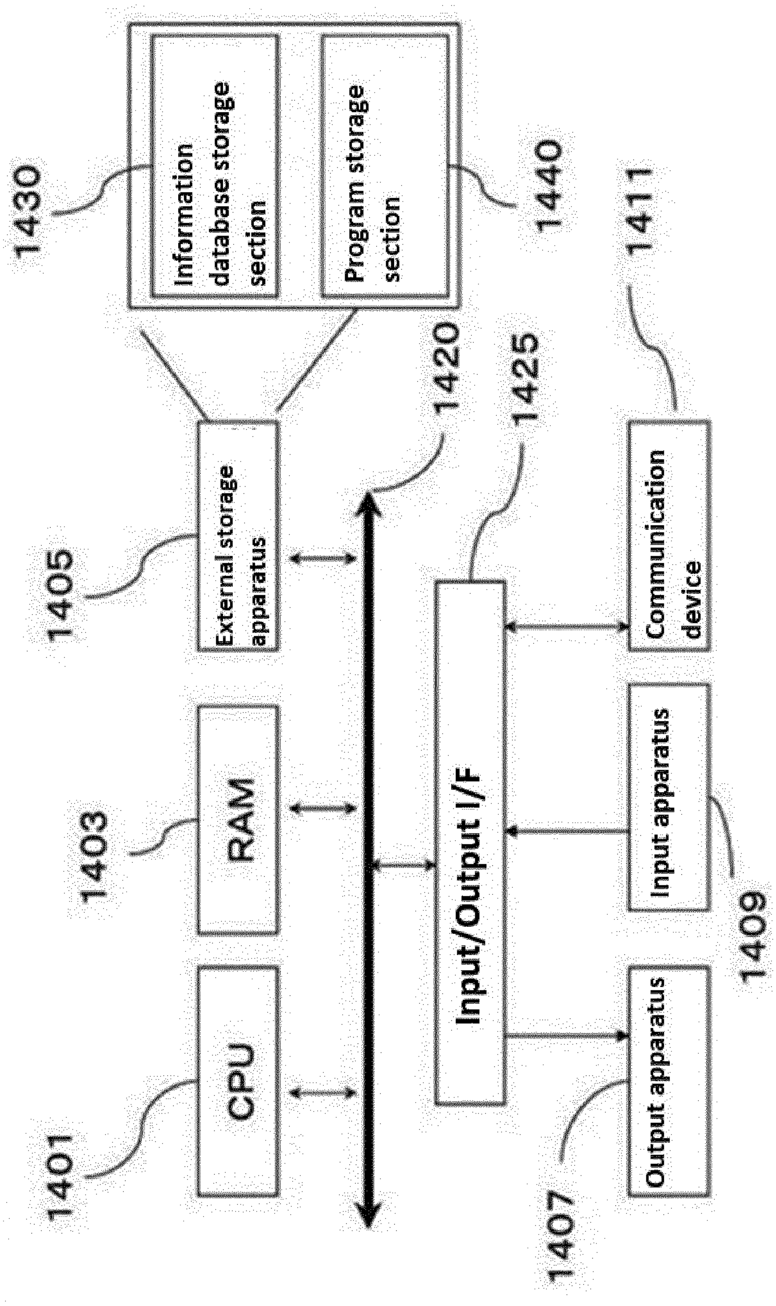
1430
Information database storage section
Program storage section
1440
1411
1420
1425
1405
External storage apparatus
Communication device
1403
RAM
Input apparatus
Input/Output I/F
1409
1401
CPU
Output apparatus
1407

**INTERNATIONAL SEARCH REPORT**

International application No. PCT/JP2017/023253

A. CLASSIFICATION OF SUBJECT MATTER
*C12N15/09*(2006.01)i, *C12Q1/68*(2006.01)i

According to International Patent Classification (IPC) or to both national classification and IPC

B. FIELDS SEARCHED

Minimum documentation searched (classification system followed by classification symbols)
C12N15/09, C12Q1/68

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2009-538621 A (Trilink Biotechnologies), 12 November 2009 (12.11.2009), claims 31 to 39; paragraphs [0035], [0176] & WO 2007/139723 A1 paragraphs [0034], [0178] & US 2007/0281308 A1 & EP 2032714 A1 | 1-24 |
| Y | JP 2000-502905 A (Clontech Laboratories, Inc.), 14 March 2000 (14.03.2000), claims; page 30, 2nd paragraph & WO 1997/024455 A2 claims; page 17, 3rd paragraph & US 5962271 A & US 5962272 A & EP 871780 A1 | 1-24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 August 2017 (10.08.17) | 29 August 2017 (29.08.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/023253

C (Continuation). DOCUMENTS CONSIDERED TO BE RELEVANT

| Category* | Citation of document, with indication, where appropriate, of the relevant passages | Relevant to claim No. |
|---|---|---|
| Y | Jeremy KAPTEYN, et al., Incorporation of non-natural nucleotides into template-switching oligonulceotides reduces background and improves cDNA synthesis from very small RNA samples, BMC Genomics, 2010, Vol.11:413, pp.1-9 entire text | 1-6,8-12, 14-24 |
| Y | WO 2015/075939 A1 (Repertoire Genesis Inc.), 28 May 2015 (28.05.2015), claims & US 2016/0289760 A1 claims & EP 3091074 A1 & CN 106103711 A | 1-24 |
| A | JP 2011-521646 A (Trilink Biotechnologies), 28 July 2011 (28.07.2011), & US 2010/0003724 A1 & WO 2009/151921 A1 & EP 2294076 A1 & CN 102105481 A | 1-24 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8133669 B
- US 8361753 B
- WO 2015075939 A
- JP 2016125007 A


### Non-patent literature cited in the description


- Curr Protoc Nucleic Acid Chem. September 2009
- *Nucleic Acids Res.,* November 2008, vol. 36 (20), e131
- *Proc. Natl. Acad. Sci. USA,* 1997, vol. 94, 105061
- *Nat. Struct. Biol.,* 1998, vol. 5, 950
- *Nat. Struct. Biol.,* 1998, vol. 5, 954
- *J. Am. Chem. Soc.,* 1999, vol. 121, 2323
- *J. Am. Chem. Soc.,* 1989, vol. 111, 8322
- *Nucleic Acids Res.,* 2005, vol. 33, 5640
- *Nucleic Acids Res.,* 2007, vol. 35, 4238
- *J. Am. Chem. Soc.,* 1999, vol. 121, 11585
- *J. Am. Chem. Soc.,* 2009, vol. 131, 14620
- *Proc. Natl. Acad. Sci. USA,* 2001, vol. 98, 4922
- *J. Am. Chem. Soc.,* 2005, vol. 127, 17286
- *Nucleic Acids Res.,* 2005, vol. 33, e129
- *Biotechniques,* 2006, vol. 40, 711
- *J. Am. Chem. Soc.,* 2004, vol. 126, 13298
- *Nat. Methods,* 2006, vol. 3, 729
- *J. Am. Chem. Soc.,* 2007, vol. 129, 15549
- *Nucleic Acids Res.,* 2009, vol. 37, e14
- *J. Am. Chem. Soc.,* 2004, vol. 126, 11826
- *J. Am. Chem. Soc.,* 2009, vol. 131, 1644
- *Angew. Chem. Int. Ed.,* 2005, vol. 44, 596
- **ARONESTY E.** *The Open BioinformaticsJournal,* 2013, vol. 7, 1-8
- **ALTSCHUL et al.** *J.Mol.Biol.,* 1990, vol. 215, 403-410
- **PEARSON ; LIPMAN.** *Proc.Natl.Acad.Sci.,* 1988, vol. 85, 2444-2448
- **SMITH ; WATERMAN.** *J.Mol.Biol.,* 1981, vol. 147, 195-197
- **NEEDLEMAN ; WUNSCH.** *J.Mol.Biol.,* 1970, vol. 48, 443-453
- **HARRIS. T.D.** *Science,* 2008, 320-160, 109
- **LITOSH VA et al.** *Nucleic Acids Res.,* March 2011, vol. 39 (6), e39
- **HUTTER D et al.** *Nucleosides Nucleotides Nucleic Acid,* 2010, vol. 29 (11), 879-95
- **COLWELL, R. K. et al.** *Journal of Plant Ecology,* vol. 5, 3-21
- **ISHIDA I ; TOMIZUKA K ; YOSHIDA H ; TAHARA T ; TAKAHASHI N ; OHGUMA A ; TANAKA S ; UMEHASHI M ; MAEDA H ; NOZAKI C.** Production of human monoclonal and polyclonal antibodies in TransChromo animals. *Cloning Stem Cells,* 2002, vol. 4 (1), 91-102
- **ITO M ; HIRAMATSU H ; KOBAYASHI K ; SUZUE K ; KAWAHATA M ; HIOKI K ; UEYAMA Y ; KOYANAGI Y ; SUGAMURA K ; TSUJI K.** NOD/SCID/gamma(c)(null) mouse: an excellent recipient mouse model for engraftment of human cells. *Blood,* 01 November 2002, vol. 100 (9), 3175-82
- **JAYAPAL KP ; WLASCHIN KF ; HU W-S ; YAP MGS.** Recombinant protein therapeutics from CHO cells-20 years and counting. *Chem Eng Prog.,* 2007, vol. 103, 40-47
- **CHUSAINOW J ; YANG YS ; YEO JH ; TOH PC ; ASVADI P ; WONG NS ; YAP MG.** A study of monoclonal antibody-producing CHO cell lines: what makes a stable high producer?. *Biotechnol Bioeng.,* 01 March 2009, vol. 102 (4), 1182-96

SEQUENCE LISTING

<110> RIKEN

<120> T CELL RECEPTOR AND B CELL RECEPTOR REPERTOIRE ANALYSIS SYSTEM USING ONE-STEP REVERSE TRANSCRIPTION TEMPLATE SWITCH PCR

<130> EC003PCT

<150> JP 2016-125007
<151> 2016-06-23

<160> 38

<170> PatentIn version 3.5

<210> 1
<211> 30
<212> DNA
<213> Artificial Sequence

<220>
<223> Block Primer in Example 1-5

<400> 1
gagggtagcc ttttgtttgt ttgcaatctc 30

<210> 2
<211> 34
<212> DNA
<213> Artificial Sequence

<220>
<223> RT Primer in Example 1-5

<400> 2
aagcacacga gggtagcctt ttgtttgttt gcaa 34

<210> 3
<211> 30
<212> DNA
<213> Artificial Sequence

<220>
<223> Template switch Oligo in Example 1-5

<400> 3
aagcagtggt atacccgcag agtacatggg 30

<210> 4
<211> 30
<212> DNA
<213> artificial sequence

<220>
<223> RT Primer in Example 6 and 7

<400> 4
acacgagggt agccttttgt ttgtttgcaa 30

<210> 5
<211> 30
<212> DNA
<213> artificial sequence

<220>
<223> Block Primer in Example 6 and 7

<400> 5
gagggtagcc ttttgtttgt ttgcaatctc 30

<210> 6
<211> 30
<212> DNA
<213> artificial sequence

<220>
<223> Template switch Oligo in Example 6 and 7

<400> 6
aagcagtggt atcaacgcag agtacatggg 30

<210> 7
<211> 64
<212> DNA
<213> artificial sequence

<220>
<223> Forward Tag primer v1

<400> 7
gtctcgtggg ctcggagatg tgtataagag acagaagcag tggtatcaac gcagagtaca 60

tggg 64

<210> 8
<211> 63
<212> DNA
<213> artificial sequence

<220>
<223> Reverse Tag primer v1

<400> 8
tcgtcggcag cgtcagatgt gtataagaga caggagggta gccttttgtt tgtttgcaat 60

ctc 63

<210> 9
<211> 47
<212> DNA
<213> artificial sequence

<220>
<223> Index Primer N701

<400> 9
caagcagaag acggcatacg agattcgcct tagtctcgtg ggctcgg 47

<210> 10
<211> 47
<212> DNA
<213> artificial sequence

<220>
<223> Index Primer N702

<400> 10
caagcagaag acggcatacg agatctagta cggtctcgtg ggctcgg 47

<210> 11
<211> 47
<212> DNA
<213> artificial sequence

<220>
<223> Index Primer N703

<400> 11
caagcagaag acggcatacg agatttctgc ctgtctcgtg ggctcgg 47

<210> 12
<211> 47
<212> DNA
<213> artificial sequence

<220>
<223> Index Primer N 704

<400> 12
caagcagaag acggcatacg agatgctcag gagtctcgtg ggctcgg 47

<210> 13
<211> 47
<212> DNA
<213> artificial sequence

<220>
<223> Index Primer N705

<400> 13
caagcagaag acggcatacg agataggagt ccgtctcgtg ggctcgg 47

<210> 14
<211> 47
<212> DNA
<213> artificial sequence

<220>
<223> Index Primer N706

<400> 14
caagcagaag acggcatacg agatcatgcc tagtctcgtg ggctcgg 47

<210> 15

<211> 47
<212> DNA
<213> artificial sequence

<220>
<223> Index Primer N707

<400> 15
caagcagaag acggcatacg agatgtagag aggtctcgtg ggctcgg 47

<210> 16
<211> 47
<212> DNA
<213> artificial sequence

<220>
<223> Index Primer N710

<400> 16
caagcagaag acggcatacg agatcagcct cggtctcgtg ggctcgg 47

<210> 17
<211> 47
<212> DNA
<213> artificial sequence

<220>
<223> Index Primer N711

<400> 17
caagcagaag acggcatacg agattgcctc ttgtctcgtg ggctcgg 47

<210> 18
<211> 47
<212> DNA
<213> artificial sequence

<220>
<223> Index Primer N712

<400> 18
caagcagaag acggcatacg agattcctct acgtctcgtg ggctcgg 47

<210> 19
<211> 47
<212> DNA
<213> artificial sequence

<220>
<223> Index Primer N714

<400> 19
caagcagaag acggcatacg agattcatga gcgtctcgtg ggctcgg 47

<210> 20
<211> 47
<212> DNA
<213> artificial sequence

<220>
<223> Index Primer N715

<400> 20
caagcagaag acggcatacg agatcctgag atgtctcgtg ggctcgg 47

<210> 21
<211> 51
<212> DNA
<213> artificial sequence

<220>
<223> Index Primer S502

<400> 21
aatgatacgg cgaccaccga gatctacacc tctctattcg tcggcagcgt c 51

<210> 22
<211> 51
<212> DNA
<213> artificial sequence

<220>
<223> Index Primer S503

<400> 22
aatgatacgg cgaccaccga gatctacact atcctcttcg tcggcagcgt c 51

<210> 23
<211> 51
<212> DNA
<213> artificial sequence

<220>
<223> Index Primer S505

<400> 23
aatgatacgg cgaccaccga gatctacacg taaggagtcg tcggcagcgt c 51

<210> 24
<211> 51
<212> DNA
<213> artificial sequence

<220>
<223> Index Primer S506

<400> 24
aatgatacgg cgaccaccga gatctacaca ctgcatatcg tcggcagcgt c 51

<210> 25
<211> 51
<212> DNA
<213> artificial sequence

<220>
<223> Index Primer S507

<400>  25
aatgatacgg cgaccaccga gatctacaca aggagtatcg tcggcagcgt c                51

<210>  26
<211>  51
<212>  DNA
<213>  artificial sequence

<220>
<223>  Index Primer S508

<400>  26
aatgatacgg cgaccaccga gatctacacc taagccttcg tcggcagcgt c                51

<210>  27
<211>  51
<212>  DNA
<213>  artificial sequence

<220>
<223>  Index Primer S510

<400>  27
aatgatacgg cgaccaccga gatctacacc gtctaattcg tcggcagcgt c                51

<210>  28
<211>  51
<212>  DNA
<213>  artificial sequence

<220>
<223>  Index Primer S511

<400>  28
aatgatacgg cgaccaccga gatctacact ctctccgtcg tcggcagcgt c                51

<210>  29
<211>  15
<212>  PRT
<213>  artificial sequence

<220>
<223>  CDR3 sequences 1 in Table 10

<400>  29

Cys Ala Ser Ser Phe His Arg Asp Tyr Asn Ser Pro Leu Tyr Phe
1               5                   10                  15

<210>  30
<211>  15
<212>  PRT
<213>  artificial sequence

<220>
<223>  CDR3 sequences 2 in Table 10

<400> 30

Cys Ala Ser Ser Phe His Arg Asp Tyr Asn Ser Pro Leu Tyr Leu
1 5 10 15

<210> 31
<211> 15
<212> PRT
<213> artificial sequence

<220>
<223> CDR3 sequences 3 in Table 10

<400> 31

Trp Ala Ser Ser Phe His Arg Asp Tyr Asn Ser Pro Leu Tyr Phe
1 5 10 15

<210> 32
<211> 15
<212> PRT
<213> artificial sequence

<220>
<223> CDR3 sequences 4 in Table 10

<400> 32

Cys Ala Ser Ser Phe His Arg Asp Tyr Asn Ala Pro Leu Tyr Phe
1 5 10 15

<210> 33
<211> 15
<212> PRT
<213> artificial sequence

<220>
<223> CDR3 sequences 5 in Table 10

<400> 33

Cys Ala Ser Ser Val His Arg Asp Tyr Asn Ser Pro Leu Tyr Phe
1 5 10 15

<210> 34
<211> 12
<212> PRT
<213> artificial sequence

<220>
<223> CDR3 sequences 1 in Table 14

<400> 34

Cys Ala Ser Ser Asp Val Gln Asp Thr Gln Tyr Phe
1 5 10

<210> 35

<211> 15
<212> PRT
<213> artificial sequence

<220>
<223> CDR3 sequences 2 in Table 14

<400> 35

```
Cys Ala Ser Ser Glu Asp Trp Gly Val Gln Asn Thr Leu Tyr Phe
1               5                   10                  15
```

<210> 36
<211> 13
<212> PRT
<213> artificial sequence

<220>
<223> CDR3 sequences 3 in Table 14

<400> 36

```
Cys Gly Ala Leu Arg Asp Arg Asn Gln Ala Pro Leu Phe
1               5                   10
```

<210> 37
<211> 14
<212> PRT
<213> artificial sequence

<220>
<223> CDR3 sequences 4 in Table 14

<400> 37

```
Cys Ala Ser Ser Leu Pro Gly Gly Asp Thr Glu Val Phe Phe
1               5                   10
```

<210> 38
<211> 16
<212> PRT
<213> artificial sequence

<220>
<223> CDR3 sequences 5 in Table 14

<400> 38

```
Cys Ala Ser Gly Asp Ala Pro Asp Arg Leu Gly Asn Thr Leu Tyr Phe
1               5                   10                  15
```